(19) Europäisches Patentamt / European Patent Office / Office européen des brevets

(11) EP 1 857 467 A1

(12) EUROPEAN PATENT APPLICATION
published in accordance with Art. 158(3) EPC

(43) Date of publication:
21.11.2007 Bulletin 2007/47

(21) Application number: 06715400.5

(22) Date of filing: 08.03.2006

(51) Int Cl.:
*C08B 37/08* (2006.01) *A61K 47/48* (2006.01)
*C07K 14/605* (2006.01)

(86) International application number:
PCT/JP2006/304480

(87) International publication number:
WO 2006/095775 (14.09.2006 Gazette 2006/37)

(84) Designated Contracting States:
AT BE BG CH CY CZ DE DK EE ES FI FR GB GR HU IE IS IT LI LT LU LV MC NL PL PT RO SE SI SK TR

(30) Priority: 08.03.2005 JP 2005064122
13.02.2006 JP 2006035645

(71) Applicant: CHUGAI SEIYAKU KABUSHIKI KAISHA
Tokyo, 115-8543 (JP)

(72) Inventors:
- NAKAMURA, Teruo
1-chome, Gotenba-shi, Shizuoka, 4128513 (JP)
- KATO, Tatsuya
1-chome, Gotenba-shi, Shizuoka, 4128513 (JP)
- TOGAWA, Hideyuki
1-chome, Gotenba-shi, Shizuoka, 4128513 (JP)
- YASUGI, Kenji
1-chome, Gotenba-shi, Shizuoka, 4128513 (JP)
- KONISHI, Hiroko
1-chome, Gotenba-shi, Shizuoka, 4128513 (JP)
- SEKIMORI, Yasuo
1-chome, Gotenba-shi, Shizuoka, 4128513 (JP)
- SHIMOBOJI, Tsuyoshi
1-chome, Gotenba-shi, Shizuoka, 4128513 (JP)

(74) Representative: Vossius & Partner
Siebertstrasse 4
81675 München (DE)

# (54) CONJUGATE OF WATER-SOLUBLE MODIFIED HYALURONIC ACID AND GLP-1 ANALOGUE

(57) To provide a GLP-1 analogue long-acting prophylactic or therapeutic agent for diabetes, diabetic complications and/or obesity due to diabetes which provides an extended half-life of a GLP-1 analogue in the blood to prevent frequent administration, and is biodegradable and safe.

The present invention provides a conjugate obtained by binding, to a GLP-1 analogue into which a mercapto group is incorporated, water soluble hyaluronic acid modification product obtained by incorporating a substituent via an amide bond to the carboxyl group of glucuronic acid portion of hyaluronic acid as a derivative thereof, using a specific condensing agent in an aprotic polar solvent; and a prophylacitc or therapeutic agent having a durable blood glucose lowering effect for diabetes, diabetic complications or obesity.

[Figure 9]

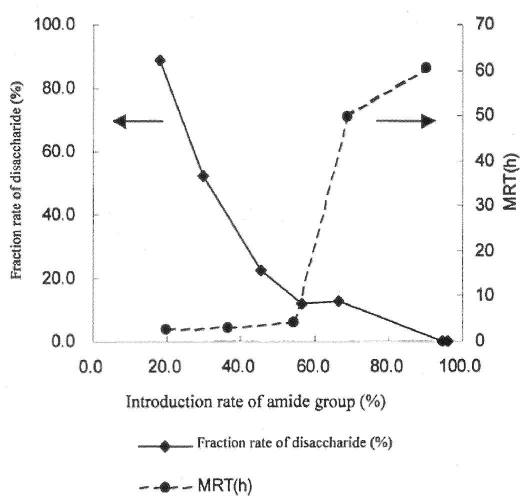


EP 1 857 467 A1

**Description**

(TECHNICAL FIELD)

The present invention relates to a conjugate of a water-soluble hyaluronic acid modification product with GLP-1 analogue, which is useful as a drug for preventing or treating diabetes, diabetic complication attributed to hyperglycemia, and obesity, and relates to a long-acting drug for preventing or treating diabetes, diabetic complication attributed to hyperglycemia, and obesity, which comprises the conjugate.

(BACKGROUND ART)

Glucagon-like peptide-1 (GLP-1) is a peptide comprising 31 amino acids, which is secreted from L-cell of small intestine in response to dietary intake. It is known that it acts on β-cell of pancreas, promotes insulin secretion and decreases blood glucose concentration (refer to the non patent document 1). Since the action is not observed at low blood glucose level (~4.5 mM), it is known that the risk of hypoglycemia is low. Furthermore, it is known that GLP-1 stimulates P-cell, which produces insulin, proliferation, enhances differentiation of β-cell from precursor cells, inhibits glucagon secretion, reduces gastric emptying, and/or suppress food intake and/or acts the like (refer to the non patent document 1). Use as a drug for preventing or treating diabetes, diabetic complication attributed to hyperglycemia, and obesity is keenly desired. However, the serum half-life of GLP-1 is a several minutes caused by inactivation by dipeptidylpeptidase IV (DPP IV) digestion and elimination from the kidney; therefore frequent administration is necessary for being used as a drug for prevention and treatment of diabetes, diabetic complication attributed to hyperglycemia, and obesity Various GLP-1 analogues and derivatives, which keep biological activities and are resistant to DPPIV, have been reported (referred to the patent documents 1-8), but since renal excretion cannot be evaded, the elongation of residence time in blood is not adequate.

Further, in general, formation of conjugate (conjugation) of a drug and a water soluble polymer has been tried for aiming the improvement of residence property in blood, the improvement of stability, the improvement of solubility, reduction of antigenicity of low molecular weight medicine, peptide medicine, protein medicine and the like. In particular, polyethylene glycol (hereinafter, also referred to as "PEG") is widely used because it an inert property and an effect for preventing the adsorption of medicine by protein in the body, and a PEG conjugated proteins have already been practically used as pharmaceuticals. However, it is not clear on some problems such as safety when PEG is accumulated in the body by long term administration, because PEG is not a biodegradable polymer. Furthermore, phenomenon for PEG conjugated liposome to clear rapidly from blood at the second administration thereof (Accelerated Blood Clearance phenomenon) has been recently reported (refer to the non-patent documents 2 and 3). It is hardly said that the safety and effectiveness of medicine conjugated with PEG were entirely established.

Hyaluronic acid (hereinafter, also referred to as "HA") is a polysaccharide isolated from the vitreous body of bovine eye by K. Meyer in 1934 and has been known as the main component of extracellular matrix for a long time.

HA is a kind of glucosamideglycans comprising disaccharide units in which D-glucuronic acid and N-acetylglucosamine are coupled via β (1→3) glycosidic bond. There is no species difference in the chemical and physical structure of HA and humans also have a metabolic system for HA. Further, in terms of immunity and toxicity, HA is considered as a very safe biomaterial. Recently, microbial mass production of high-molecular weight HA became possible allowing developing commercial use of HA in the fields of therapeutic agents for osteoarthritic joints, cosmetics, etc. It has been reported that conjugation of a drug with hyaluronic acid enables to achieve targeting of the drag to cancer tissues (refer to the patent document 9) or to liver (refer to the patent document 10), reduction of antigenicity (refer to the patent document 11), elongation of residence time in blood (refer to the patent documents 12, 13 and 14) and the like.

In comparison with PEG generally used, the advantages of using hyaluronic acid as a conjugate carrier of a drug are that it is biodegradable, it is available in giant size, further, a plural number of drugs (a plural number of the same drugs or two or more of different drugs) can be equipped in a molecule thereof because it has many reaction points in the molecule. The use of hyaluronic acid, which has such advantages, as a conjugate carrier of a drug provide us a opportunity for designing and developing a conjugate having advanced pharmacokinetics controlling functions such as targeting, controlled release and the like. Further, since hyaluronic acid is biodegradable and has no species difference in its chemical structure, it can be said that hyaluronic acid is also a more superior carrier than PEG from the viewpoint of safety.

However, the residence time of hyaluronic acid in blood itself is short and it has been reported that half-life is 2 minutes after intravenous administration (hereinafter, referred to as "iv") (refer to the non patent document 4). The study of the present inventors has shown that conventional conjugation of hyaluronic acid with a drug does not provide elongation of the residence time of the drug in blood or improvement of sustainability of the effects of drug. The main sites of hyaluronic acid metabolism are liver and lymph gland, and the metabolism is caused mainly by intracellular incorporation via cell membrane localized receptors such as CD44, RHAMM, HARE and the like, which specifically bind

to hyaluronic acid, followed by degradation by hyaluronidase. It has been reported that each of these receptor molecules recognizes the continuous free carboxyl groups (six saccharides) of hyaluronic acid as the main recognition site (refer to the non patent document 5).

Therefore, there is a trial for utilizing hyaluronic acid modification products, which are produced by introducing substituents in hyaluronic acid, as a drug carrier in order to solve the problem that the residence time of hyaluronic acid in blood is short (refer to the patent documents 4, 5 and 6). In general, it is considered that introducing substituents into hyaluronic acid elongates the residence time thereof in blood, and the extent of the elongation correlates with the introduction rate of the substituent. The hyaluronic acid modification products in which substituents were introduced in various sites of hyaluronic acid have been reported. Among them, it is considered that the introduction of a substituent in the carboxyl groups of glucuronic acid moiety in hyaluronic acid via amide bond, which is resistant to hydrolysis, is effective for inhibiting bind the hyaluronic acid modification product to a hyaluronic acid receptor, and such a hyaluronic acid modification product is also superior in the residence time in blood.

Also, it has also been reported that a hyaluronic acid modification product obtained by converting hyaluronic acid to a tetrabutyl ammonium salt and amidating the carboxyl group of hyaluronic acid by reacting it with a substituent in dimethylsulfoxide (refer to the patent document 7). However, the objective of the invention is preparation of a crosslinked product, and the invention is not intended for the improvement of the residential property in blood. Furthermore, 1,1-carbonyldiimidazole (hereinafter, also referred to as "CDI") is used as a condensing agent in invention. Our study has shown that even if CDI is used as a condensing agent, a hyaluronic acid derivative which have adequate resistance to degradation by hyaluronidase, showing that residence property in blood is improved sufficiently, cannot be obtained, and that the molecular weight of hyaluronic acid is lowered greatly during reaction.

In addition to these, an example of introduction of a substituent into the carboxyl group of hyaluronic acid in a mixed solvent of water and a polar organic solvent has also been reported (refer to the patent document 8). However, there is no description as to whether the elongated residence time in blood is confirmed in the resultant hyaluronic acid modification product.

As stated above, there has been not known a water-soluble hyaluronic acid modification product suitable for a practical use as a drug carrier, in particular, a water-soluble hyaluronic acid modification product in which the residence time in blood is elongated to a practical level.

As a polymer conjugate of GLP-1 analogue, those bound to PEG at the C-terminal has been reported (refer to the patent documents 17 and 18), but there is not any report relating to the conjugate of GLP-1 analogue with a hyaluronic acid modification product having improved residential property in blood.


(Patent document 1) International Publication WO91/11457 pamphlet,
(Patent document 2) JP-A-11-310597,
(Patent document 3) International Publication WO99/43705 pamphlet,
(Patent document 4) International Publication WO00/069911 pamphlet,
(Patent document 5) International Publication WO95/31214 pamphlet,
(Patent document 6) International Publication WO00/07617 pamphlet,
(Patent document 7) International Publication WO03/103572 pamphlet,
(Patent document 8) International Publication WO97/29180 pamphlet,
(Patent document 9) International Publication WO92/06714 pamphlet,
(Patent document 10) JP-A-2001-81103,
(Patent document 11) JP-A-2-273176,
(Patent document 12) JP-A-5-85942,
(Patent document 13) International Publication WO01/05434 pamphlet,
(Patent document 14) International Publication WO01/60412 pamphlet,
(Patent document 15) Japanese Patent Application Domestic Announcement No.2002-519481,
(Patent document 16) International Publication WO94/19376 pamphlet,
(Patent document 17) International Publication WO04/022004 pamphlet,
(Patent document 18) International Publication WO03/040309 pamphlet,
(Non-patent document 1) Trends Pharacol. Sci. Vol.24, Page 377-383, 2003 ,
(Non-patent document 2) Int. J. Pharm., Vol.255, pages 167-174, 2003,
(Non-patent document 3) J. Control. Rel., Vol.88, pages 35-42, 2003,
(Non-patent document 4) J. Inter. Med., Vol.242, pages 27-33, 1997,
(Non-patent document 5) Exp. Cell Res., Vol.228, pages 216-228, 1996

(DISCLOSURE OF THE INVENTION)

(PROBLEMS TO BE SOLVED BY THE INVENTION)

The objective of the invention is to provide a GLP-1 analogue conjugate useful for prevention or treatment of chronic disease such as diabetes, hyperglycemia, diabetic complication and/or obesity, which has practically sufficient residence time in blood and is biodegradable and safe. Further, the objective is to provide a process for producing the GLP-1 analogue conjugate, GLP-1 analogue which can be used for production of the conjugate, a pharmaceutical composition containing the conjugate and a treatment method.

(MEASURES FOR SOLVING PROBLEM)

The present inventors promoted extensively study for solving such problems, and have found that the conjugate of GLP-1 analogue with water-soluble hyaluronic acid modification product which was obtained by introducing a substituent to the carboxyl groups of glucuronic acid of hyaluronic acid or its derivative via an amide bond has practically sufficient residence time in blood, and have found that the conjugate has prolonged blood glucose lowering effect to complete the present invention.

According to one aspect of the present invention, there is provided a hyaluronic acid-peptide conjugate or a salt thereof wherein one or more glucagon-like peptide-1 (GLP-1) analogues are bound with water-soluble hyaluronic acid modification product. The GLP-1 analogues are conjugated with the hyaluronic acid modification product through a polyvalent or divalent linker. The above-mentioned salt of the hyaluronic acid-peptide conjugate is not limited, but includes, for example, a pharmaceutically acceptable salt (for example, an acid addition salt (for example, a salt of hydrochloric acid, a salt of sulfuric acid, a salt of hydrobromic acid, a salt of acetic acid, a salt of phosphoric acid and the like), a base addition salt (for example, a sodium salt, a potassium salt, a magnesium salt, a calcium salt, an aluminum salt, an ammonium salt, a tetrabutylammonium salt and the like).

The water-soluble hyaluronic acid modification product is not limited, but for example, a compound in which a carboxyl group contained in the molecule of hyaluronic acid converted to a substituted amide group is used. In the water-soluble hyaluronic acid modification product used for the present invention, preferably 70% or more of carboxylic groups contained in the glucuronic acid portion of hyaluronic acid are converted to N-substituted amide groups, in which the substituents of respective N-substituted amide groups in the hyaluronic acid modification product may be the same or different and at least one of the substituents may be a divalent linker which is linked with the GLP-1 analogues.

According to another aspect of the present invention, there is provided a hyaluronic acid-peptide conjugate or a salt thereof defined hereinbefore, wherein the GLP-1 analogue is a peptide with an amino acid sequence of GLP-1 (1-36) or GLP-1 (7-36) added with an amino acid sequence represented by -Xa-Cys at the C-terminal thereof, or a peptide with an amino acid sequence of the peptide in which 1 to 5 amino acids are deleted, substituted and/or added wherein the amino acid sequence may be substituted and/or added with a natural amino acid and/or a non-natural amino acid, in which Xa is a direct bond or a sequence comprising 1 to 9 amino acids independently selected from proline, glycine, serine and glutamic acid, and wherein the carboxyl group of cysteine of the C-terminal of the peptide may be optionally converted to an amide group.

According to one embodiment in the aspect of the present invention, the GLP-1 analogue is, for example, a peptide with an amino acid sequence of GLP-1 (1-36) or GLP-1 (7-36) added with an amino acid sequence represented by -Xa-Cys at the C-terminal thereof in which the 8-position of alanine ($Ala^8$) of the sequence is substituted with a natural amino acid or a non-natural amino acid, and wherein the carboxyl group of cysteine of the C-terminal of the peptide may be optionally converted to an amide group. The natural amino acid or the non-natural amino acid with which the alanine may be substitute includes, for example, glycine, serine, valine, leucine, isoleucine, threonine, methionine, phenylalanine, asparagic acid, glutamine, arginine, aminobutyric acid and the like. The fore-mentioned alanine is preferably substituted with an amino acid selected from glycine, serine, valine, leucine, isoleucine and threonine, and more preferably substituted with an amino acid selected from glycine and serine.

According to another embodiment in the aspect of the present invention, the GLP-1 analogue used for the present invention is a peptide represented by His-Gly-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-Xa-Cys (Sequence Number 1) or a peptide in which the carboxyl group of cysteine of the C-terminal of the peptide may be optionally converted to an amide group. Xa is as defined already, and includes, for example, a direct bond or -Gly-Pro-Pro-Pro-.

According to another aspect of the present invention, there is provided the hyaluronic acid-peptide conjugate or a salt thereof described already, wherein the GLP-1 analogue is a peptide with an amino acid sequence of GLP-1 (1-36), GLP-1 (1-37), GLP-1 (7-36) or GLP-1 (7-37) added with a thiol compound represented by -Qa-SH at the C-terminal thereof, or a peptide with the amino acid sequence of the peptide in which 1 to 5 amino acids are deleted, substituted and/or added in the amino acid sequence, wherein the amino acid sequence may be optionally substituted

and/or added with a natural amino acid and/or a non-natural amino acid, in which Qa is selected from -NH-$X^5$-, -CO-$X^5$- and -CONH-$X^5$- and is linked with a carboxyl group, an amine group or a hydroxyl group which is contained in the C-terminal amino acid of the peptide, to form an amide bond, a urea bond or an ester bond, and

$X^5$ is a $C_{1-50}$ alkylene group in which an oxygen atom may be inserted between two carbon atoms contained in the alkylene group at one or more sites of the alkylene group and a carbon atom of the alkylene group may be independently substituted with one or more substituents selected from a hydroxyl group and a $C_{1-6}$ alkyl group.

In the further aspect of the present invention, there is provided the hyaluronic acid-peptide conjugate or a salt thereof described already, wherein one end of a divalent linker is bound with a GLP-1 analogue via a mercapto group introduced into a GLP-1 analogue.

In another embodiment of the aspect of the present invention, the divalent linker may be represented by the formula (I):

[Formula 1]

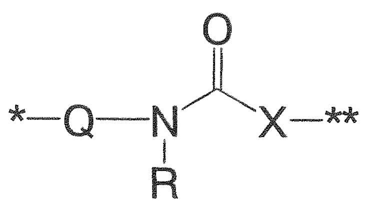

(I)

wherein Q is a $C_{1-400}$ alkylene group (including for example, $C_{1-10}$ alkylene group), in which an oxygen atom may be inserted between two carbon contained in the alkylene group at one or more sites of the alkylene group, further, -CO-, -NHCO- or -CONH- may be optionally inserted between carbon atoms or at the end of the alkylene group at one or more sites of the alkylene group and a carbon atom of the alkylene group may be optionally substituted with one or more substituents selected from a hydroxyl group and a $C_{1-6}$ alkyl group independently;

X is $-CH_2-CH_2-$**, $-CH_2-CH_2-CH_2-$** or $-CH(-CH_3)-CH_2-$** and R is a hydrogen atom or a $C_{1-6}$ alkyl group; or

X and R together with a carbon atom and a nitrogen atom to which they are attached may form a group represented by formula (II);

[Formula 2]

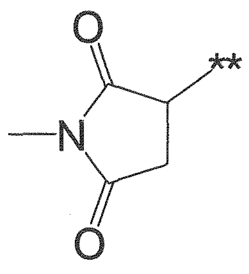

(II)

wherein

* represents the position linked to a nitrogen atom of an amide group in a hyaluronic acid modification product and
** represents the position linked to a sulfur atom of a mercapto group of the GLP-1 analogue.

The above-mentioned divalent linker includes for example, a divalent linker represented by the formula (Ia):

[Formula 3]

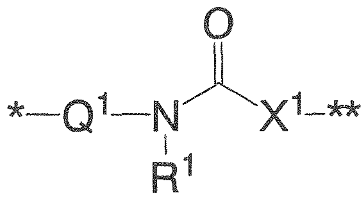

(Ia)

wherein $Q^1$ is a $C_{1-10}$ alkylene group, in which an oxygen atom may be inserted between two carbon atoms contained in the alkylene group at one or more sites of the alkylene group, and a carbon atom of the alkylene group may be optionally substituted with one or more substituents selected from a hydroxyl group and a $C_{1-6}$ alkyl group independently;

$X^1$ is $-CH_2-CH_2-**$, $-CH_2-CH_2-CH_2-**$ or $-CH(-CH_3)-CH_2-**$ and $R^1$ is a hydrogen atom or a $C_{1-6}$ alkyl group; or

$X^1$ and $R^1$ together with a carbon atom and a nitrogen atom to which they are attached may form a group represented by the formula (IIa);

[Formula 4]

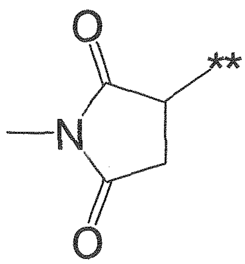

(IIa);

or

$X^1$ is a group represented by the formula (Ib):

[Formula 5]

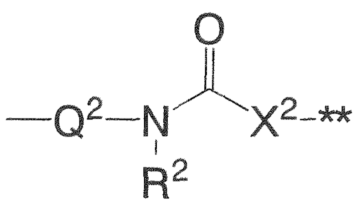

(Ib)

wherein $Q^2$ is a $C_{1-10}$ alkylene group, in which an oxygen atom may be inserted between two carbon atoms contained in the alkylene group at one or more sites of the alkylene group, and a carbon atom of the alkylene group may be optionally substituted with one or more substituents selected from a hydroxyl group and a $C_{1-6}$ alkyl group independently;

$X^2$ is $-CH_2-CH_2-**$, $-CH_2-CH_2-CH_2-**$ or $-CH(-CH_3)-CH_2-**$ and $R^2$ is a hydrogen atom or a $C_{1-6}$ alkyl group; or

$X^2$ and $R^2$ together with a carbon atom and a nitrogen atom to which they are attached may form a group represented by the formula (IIb);

[Formula 6]

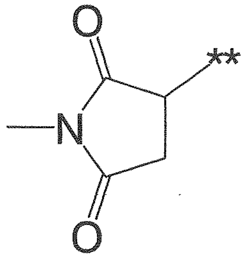

(IIb);

or

$X^2$ is a group represented by the formula (Ic):

[Formula 7]

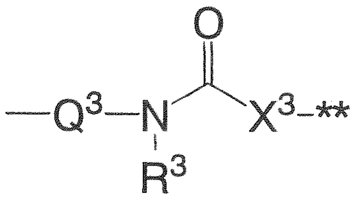

(Ic)

wherein $Q^3$ is a $C_{1-10}$ alkylene group, in which an oxygen atom may be inserted between two carbon atoms contained in the alkylene group at one or more sites of the alkylene group, and a carbon atom of the alkylene group may be optionally substituted with one or more substituents selected from a hydroxyl group and a $C_{1-6}$ alkyl group independently;

$X^3$ is $-CH_2-CH_2-$**, $-CH_2-CH_2-CH_2-$** or $-CH(-CH_3)-CH_2-$** and $R^3$ is a hydrogen atom or a $C_{1-6}$ alkyl group; or

$X^3$ and $R^3$ together with a carbon atom and a nitrogen atom they are attached may form a group represented by the formula (IIc);

[Formula 8]

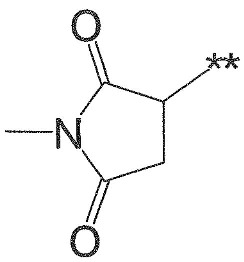

(IIc);

**[0038]** * represents the position linked to a nitrogen atom of an amide group in the hyaluronic acid modification product and ** represents the position linked to a sulfur atom of a mercapto group of the GLP-1 analogue.

In the above-mentioned formula (Ia), for example, $Q^1$ may be a group represented by the formula: $-(CH_2)_m-$or

$-(CH_2)_m-(O-CH_2-CH_2)_n-$

(wherein m is an integer selected from 1 to 20, preferably 1 to 15 and more preferably 2 to 10 and n is an integer selected from 1 to 200, preferably 1 to 25 and more preferably 1 to 4).

In the above-mentioned formula (Ia), for example, $X^1$ is a group represented by the formula: $-(CH_2)_m-(O-CH_2-CH_2)_p-NHCO-(CH_2)_q-Y^1-**$ or $-(CH_2)_r-Y^1-**$

wherein m is an integer respectively selected from 1 to 20, preferably 1 to 15 and more preferably 2 to 10, p is an integer selected from 1 to 200, preferably 1 to 25 and more preferably 1 to 4 and q and r is an integer selected from 1 to 20, preferably 1 to 15 and more preferably 1 to 10, $Y^1$ is a group represented by the formula (IIc):

[Formula 9]

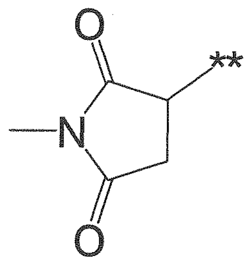

(IIc)

** represents the position linked to a sulfur atom of a mercapto group of the GLP-1 analogue.

In another embodiment of the aspect of the present invention, there is provided the hyaluronic acid-peptide conjugate or a salt thereof described already, comprising a repeating unit which is represented by the formula (VIa):

[Formula 10]

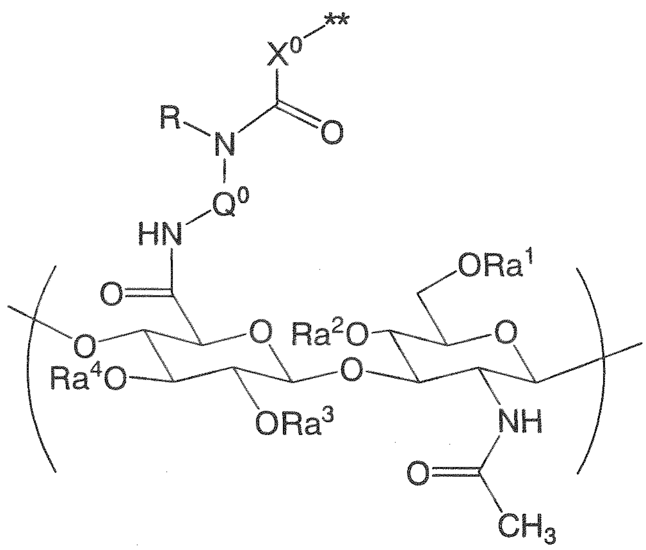

(VIa)

wherein each of $Ra^1$, $Ra^2$, $Ra^3$ and $Ra^4$ is independently selected from a hydrogen atom, a $C_{1-6}$ alkyl group or a $C_{1-6}$ alkylcarbonyl group,

$X^0$ represents X or $X^1$ defined already,

$Q^0$ represents Q or $Q^1$ defined already, and

R and ** are as defined already);

a repeating unit which is represented by the formula (VIb):

[Formula 11]

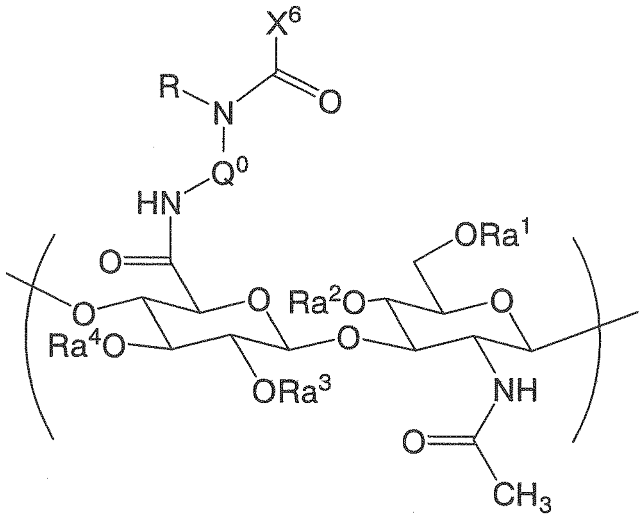

(VIb)

(wherein $Ra^1$, $Ra^2$, $Ra^3$, $Ra^4$, $X^0$, $Q^0$, R and ** are as defined already),

$X^6$ is $-CH_2=CH_2$, $-CH_2-CH=CH_2$, $-C(CH)_3=CH_2$, $-(CH_2)_m-Y^2$

or $-(CH_2)_m-(O-CH_2-CH_2)_n-Y^2$, and $Y^2$ is a group represented by the formula (VII):

[Formula 12]

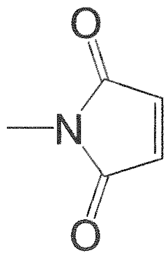

(VII)

m is, for example, 1 to 20 and preferably an integer selected from 1 to 10, and n is, for example, 1 to 200 and preferably an integer selected from 1 to 25; and a repeating unit which is represented by the formula (VIc):

[Formula 13]

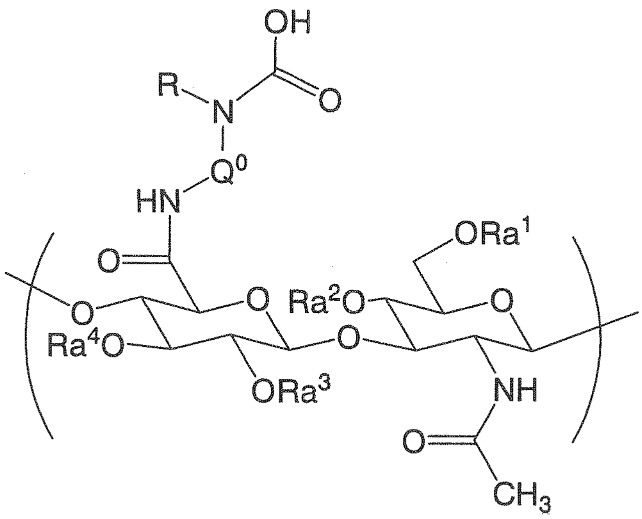

(VIc)

wherein $Ra^1$, $Ra^2$, $Ra^3$, $Ra^4$, $Q^0$ and R are as defined already.

In further aspect of the present invention, there is provided the hyaluronic acid-peptide conjugate or a salt thereof described already, wherein one or more of substituents introduced on a nitrogen atom of the amide group is represented by the formula (IV):

[Formula 14]

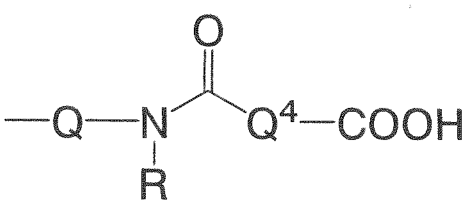

(IV)

wherein Q and R are as defined already and $Q^4$ is a $C_{2-6}$ alkylene group.

In further aspect of the present invention, there is also provided the hyaluronic acid-peptide conjugate or a salt thereof according to any one of Claims 1 to 16, wherein the modification rate of a carboxyl group contained in hyaluronic acid to an N-substituted amide group is 70% by mole or more.

In one embodiment of the present invention, the introduction rate of an amide group substituted with a linker bound with GLP-1 analogues, to a carboxylic group contained in hyaluronic acid is, for example, 0.1% by mole or more and 15% by mole or less in average, preferably 0.1% by mole to 10% by mole and further preferably 0.1% by mole to 5% by mole. Further, the average molecular weight of the hyaluronic acid-peptide conjugate related to the present invention is 5000 dalton to one million dalton when it is measured as viscosity average molecular weight, preferably 10000 dalton to 300000 dalton and further preferably 80000 dalton to 300000 dalton.

In the further aspect of the present invention, there is also provided a pharmaceutical composition containing the hyaluronic acid-peptide conjugate or a salt thereof described already. The administration method of the pharmaceutical composition is not limited but it can be administrated by a method known to those skilled in the art. It is also administrated by means selected from intravenous administration, intramuscular administration, subcutaneous administration, intraperitoneal administration, intranasal administration and pulmonary administration.

According to the further aspect of the present invention, there is provided a pharmaceutical used for prevention or treatment of a disease selected from diabetes, hyperglycemia, diabetic complication and obesity, containing the hyaluronic acid-peptide conjugate or a salt thereof described already.

According to the another aspect of the present invention, there is provided a method for prevention or treatment of a disease selected from diabetes, hyperglycemia, diabetic complication and obesity, including the administration of the effective quantity for treatment of the hyaluronic acid-peptide conjugate or a salt thereof described already.

According to the another aspect of the present invention, there is provided a process for producing the hyaluronic acid-peptide conjugate or a salt thereof described already, comprising a step of obtaining a water-soluble hyaluronic acid modification product by converting a carboxyl group contained in the glucuronic acid portion of hyaluronic acid in an aprotic polar solvent, using a condensing agent represented by the formula (V):

[Formula 15]

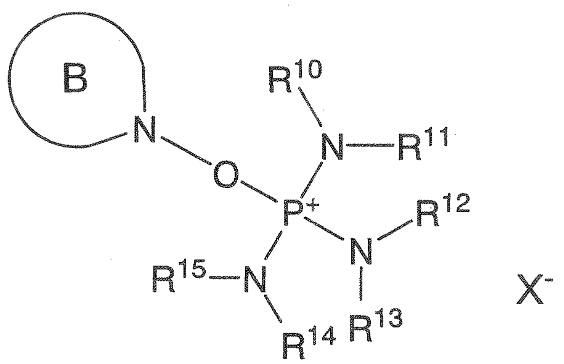

(V)

wherein each of $R^{10}$, $R^{11}$, $R^{12}$, $R^{13}$, $R^{14}$ and $R^{15}$ is independently selected from a $C_{1-6}$ alkyl group, or each of $R^{10}$ and $R^{11}$, $R^{12}$ and $R^{13}$, and $R^{14}$ and $R^{15}$ together with the nitrogen atom to which they are attached may independently form a nitrogen-containing heterocyclic ring, a ring B is a monocyclic or condensed nitrogen-containing heterocyclic ring which may be optionally substituted, and $X^-$ represents an anion. As the fore-mentioned aprotic polar solvent, for example, dimethylformamide, dimethylacetoamide, dimethylsulfoxide, 1,3-dimethyl-2-imidazolidinone, sulfolane, N-methylpyrrolidone or a mix solvent of 2 or more thereof may be used. As the fore-mentioned aprotic polar solvent, dimethylsulfoxide may be preferably used.

In the above-mentioned formula (V), the ring B is preferably benzotriazole-1-yl, and examples of the above-mentioned condensing agent represented by the above-mentioned formula (V) include a BOP-type condensing agent selected from benzotriazol-1-yloxy-tris(dimethylamino)phosphonium hexafluorophosphate, benzotriazol-1-yloxy-tris (pyrrolidino)phosphonium hexafluorophosphate and a mixture thereof.

According to the further aspect of the present invention, there is provided the hyaluronic acid-peptide conjugate or a salt thereof described already, which can be produced by the above-mentioned production process.

According to the further aspect of the present invention, there is provided a GLP-1 analogue which is a peptide with an amino acid sequence of GLP-1 (1-36) or GLP-1 (7-36) added with an amino acid sequence represented by -Xa-Cys at the C-terminal thereof, or a peptide with the amino acid sequence of the peptide in which 1 to 5 of amino acids are deleted, substituted and/or added in the amino acid sequence wherein the amino acid sequence may be optionally substituted and/or added with a natural amino acid or a non-natural amino acid, in which Xa is a direct bond or a sequence comprising 1 to 9 amino acids independently selected from proline, glycine, serine and glutamic acid, wherein the carboxyl group of cysteine of the C-terminal of the peptide may be optionally converted to an amide group.

According to one embodiment of the aspect of the present invention, the GLP-1 analogue is a peptide with an amino acid sequence of GLP-1 (1-36) or GLP-1 (7-36) added with an amino acid sequence represented by -Xa-Cys at the C-terminal thereof in which the alanine in the 8-position ($Ala^8$) of the amino acid sequence is substituted with a natural amino acid or a non-natural amino acid, wherein the carboxyl group of cysteine of the C-terminal of the peptide may be optionally converted to an group and Xa is as defined already. Examples of the natural amino acid or non-natural amino acid with which the fore-mentioned alanine is substituted include glycine, serine, valine, leucine, isoleucine, threonine, methionine, phenylalanine, asparagic acid, glutamine, arginine, aminobutyric acid and the like. The alanine is preferably substituted with an amino acid selected from glycine, serine, valine, leucine, isoleucine and threonine and further preferably with an amino acid selected from glycine and serine.

Examples of the GLP-1 analogues include a peptide represented by Sequence Number 1 or a peptide in which the carboxyl group of cysteine of the C-terminal of the peptide is converted to an amide group. Xa is preferably a direct bond or -Gly-Pro-Pro-Pro-.

According to another aspect of the present invention, the water-soluble hyaluronic acid modification product is

not specifically limited, but is preferably those in which the water-soluble hyaluronic acid modification product is decomposed by hyaluronidase which can decompose hyaluronic acid to disaccharide being its compositional unit and prepare unsaturated disaccharide degradation product having a Δ-4,5-glucuronic acid at non-reducing end, and when the absorption at 232 nm of the degraded product obtained is measured, a proportion of absorption derived from disaccharide to the total absorption derived from the degraded product is 30% or less.

[Effect of the Invention]

The elongation of residence time in blood is achieved by using the hyaluronic acid modification product-GLP-1 analogue of the present invention and it is possible to provide a preparation for prolonged action of the practical and safe GLP-1 analogue which could not be achieved by conventional technology and prevention or treatment containing the GLP-1 analogue for diabetes, hyperglycemia, diabetic complication or obesity.

[BRIEF DESCRIPTION OF DRAWINGS]

Figure 1 is an example of GPC chromatograms after hyaluronidase treatment on water soluble hyaluronic acid modification products that may be used in the present invention;
Figure 2 is an example of NMR spectra of the water soluble hyaluronic acid modification products that may be used in the present invention;
Figure 3 is an example of NMR spectra of water soluble hyaluronic acid modification products in which an amino group of the water soluble hyaluronic acid modification product that may be used in the present invention is converted to carboxylic acid (Example 2-2);
Figure 4 is an example of GPC chromatograms after hyaluronidase treatment on water soluble hyaluronic acid modification products in which the amino group of the water soluble hyaluronic acid modification products that may be used in the present invention is converted to carboxylic acid;
Figure 5 is an example of NMR spectra of water soluble hyaluronic acid modification products that may be used in the present invention (Example 3-1);
Figure 6 shows change in plasma concentration of fluorescently labeled HA modification products having different molecular weights;
Figure 7 is an example of NMR spectra of water soluble hyaluronic acid modification products that may be used in the present invention (Example 4-1);
Figure 8 shows change in plasma concentration of fluorescently labeled HA modification products, which are synthesized from HA of the molecular weight of 200 kDa and have different modification rate of amino group;
Figure 9 shows the relationship between amino group modification rate of HA modification product and mean residence time(MRT), and the relationship between amino group modification rate and fraction rate of disaccharide that is a degraded product with hyaluronidase;
Figure 10 shows change in plasma concentration after HA-GLP-1 analogue 1 conjugate is intravenously administered to rats;
Figure 11 is an example of GPC elution patterns of HA-GLP-1 analogue 2 conjugate and a control compound;
Figure 12 shows change in plasma concentration after HA-GLP-1 analogue 2 conjugate is intravenously administered to rats; and
Figure 13 shows change in plasma concentration after HA-GLP-1 analogue 1 conjugate is intravenously administered to rats.

[EMBODIMENTS FOR CARRYING OUT THE INVENTION]

The present invention is further specifically illustrated.

The water-solubility of the hyaluronic acid modification product used in the present invention is not specifically limited in so far as the hyaluronic acid-peptide conjugate obtained exhibits desired effect, but the hyaluronic acid modification product having a solubility of, for example, 0.1 to 1000 mg/mL and preferably 1 to 100 mg/mL for water can be used for the present invention.

The water-soluble hyaluronic acid modification product used in the present invention can be obtained by converting the carboxylic group of the glucuronic acid of hyaluronic acid or its derivative to an N-substituted amide group in an aprotic solvent such as an aprotic polar solvent, using a condensing agent.
The modification rate of the water-soluble HA modification product is calculated as the introduction rate of an amide group by the formula below, which corresponds to the proportion of a carboxyl group modified, namely, the proportion

of a carboxyl group converted to an N-substituted amide group:

[Formula 16]

$$\text{Introduction rate of amide group} = \frac{\text{Total number of amide group introduced into respective molecules}}{\text{Total number of glucuronic acid in respective molecules}} \times 100$$

The lower limit of the introduction rate of amide group of the water-soluble HA modification product used in the present invention is preferably 70% or more and more preferably 85% or more. Further, the upper limit may be 100% by mol or less.

The above-mentioned conversion reaction to an N-substituted group can be carried out using hyaluronic acid and its derivative, various amines and an appropriate condensing agent. The amines used are not specifically limited, but examples include amines represented by the formulae below:

$H_2N-(CHR^5)_m-NH_2$;
$H_2N-CH_2CH_2-(OCH_2CH_2)_n-NH_2$;
$H_2N-(CHR^5)_m-OH$;
$H_2N-CH_2CH_2-(OCH_2CH_2)_nOH$;
$H_2N-(CHR^5)_m-SR^6$;
$H_2N-CH_2CH_2-(OCH_2CH_2)_n-SR^6$;
$H_2N-(CHR^5)_m-O-CO-C(R^7)=CH_2$;
$H_2N-(CHR^5)_m-CO-O-(CHR^5)_p-C(R^7)=CH_2$;
$H_2N-(CHR^5)_m-NHCO-C(R^7)=CH_2$;
$H_2N-(CHR^5)_m-NHCO-CH_2C(R^7)=CH_2$;
$H2_N-(CHR^5)_m-CONH-(CHR^5)_p-C(R^7)=CH_2$;
$H_2N-CH_2CH_2-(OCH_2CH_2)_n-NHCO-C(R^7)=CH_2$;
$H_2N-CH_2CH_2-(OCH_2CH_2)_n-NHCO-CH_2C(R^7)=CH_2$;
$H_2N-CH_2CH_2-(OCH_2CH_2)_n-CONH-(CHR^5)_p-C(R^7)=CH_2$;
$H_2N-CH_2CH_2-(OCH_2CH_2)_n-O-CO-C(R^7)=CH_2$;
$H_2N-CH_2CH_2-(OCH_2CH_2)_n-CO-O-(CHR^5)_p-C(R^7)=CH_2$;
$H_2N-CH_2CH_2-(OCH_2CH_2)_n-NHCO-(CH_2)_m-Y^2$;
$H_2N-CH_2CH_2-(OCH_2CH_2)_n-CONH-(CH_2)_m-Y^2$;
$H_2N-CH_2CH_2-(OCH_2CH_2)_n-NHCO-CH_2CH_2-(OCH_2CH_2)_n-Y^2$;
$H_2N-CH_2CH_2-(OCH_2CH_2)_n-CONH-CH_2CH_2-(OCH_2CH_2)_n-Y^2$;
$H_2N-(CHR^5)_m-NHCO-(CH_2)_m-Y^2$;
$H_2N-(CHR^5)_m-CONH-(CH_2)_m-Y^2$;
$H_2N-(CHR^5)_m-NHCO-CH_2CH_2-(OCH_2CH_2)_n-Y^2$;
$H_2N-(CHR^5)_m-CONH-CH_2CH_2-(OCH_2CH_2)_n-Y^2$;
$H_2N-(CHR^5)_m-Y^2$; or $H_2N-CH_2-CH_2-(O-CH_2-CH_2)_n-Y^2$

(wherein each occurrence of m is independently an integer of, for example, 1 to 20 and preferably 1 to 10, each occurrence of n is independently an integer of, for example, 1 to 200 and preferably 1 to 25, each occurrence of p is independently an integer of, for example, 0 to 20 and preferably 0 to 10, each occurrence of $R^5$ existing is independently selected from a hydrogen atom, a $C_{1-6}$ alkyl group and a hydroxyl group, each occurrence of $R^6$ is independently selected from a hydrogen atom, a $C_{1-6}$ alkyl group and the protective group of a mercapto group (for example, a pyridylsulfide group, an acetyl group, a trityl group and an ethoxycarbonylethyl group, and the like),
each occurrence of $R^7$ is independently selected from a hydrogen atom and a $C_{1-6}$ alkyl group, and $Y^2$ is a group represented by the formula (VII):

[Formula 17]

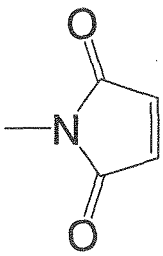

(VII)

Accordingly, among the hyaluronic acid modification products in which a carboxyl group contained in the glucuronic acid portion of hyaluronic acid is converted to an N-substituted amide group, the hyaluronic acid modification products in which a substituent is groups below:

$-(CHR^5)_m-NH_2$;
$-CH_2CH_2-(OCH_2CH_2)_n-NH_2$;
$-(CHR^5)_m-OH$;
$-CH_2CH_2-(OCH_2CH_2)_n-OH$;
$-(CHR^5)_mSR^6$;
$-CH_2CH_2-(OCH_2CH_2)_n-SR^6$;
$-(CHR^5)_m-O-CO-C(R^7)=CH_2$;
$-(CHR^5)_m-CO-O-(CHR^5)_p-C(R^7)=CH_2$;
$-(CHR^5)_m-NHCO-C(R^7)=CH_2$;
$-(CHR^5)_m-NHCO-CH_2C(R^7)=CH_2$;
$-(CHR^5)_m-CONH-(CHR^5)_p-C(R^7)=CH_2$;
$-CH_2CH_2-(OCH_2CH_2)_n-NHCO-C(R^7)=CH_2$;
$-CH_2CH_2-(OCH_2CH_2)_n-NHCO-CH_2C(R^7)=CH_2$;
$-CH_2CH_2-(OCH_2CH_2)_n-CONH-(CHR^5)_p-C(R^7)=CH_2$:
$-CH_2CH_2-(OCH_2CH_2)_n-O-CO-C(R^7)=CH_2$;
$-CH_2CH_2-(OCH_2CH_2)_n-CO-O-C(R^7)=CH_2$;
$-CH_2CH_2-(OCH_2CH_2)_n-NHCO-(CH_2)_m-Y^2$;
$-CH_2CH_2-(OCH_2CH_2)_n-CONH-(CH_2)_m-Y^2$;
$-CH_2CH_2-(OCH_2CH_2)_n-NHCO-CH_2CH_2-(OCH_2CH_2)_n-Y^2$;
$-CH_2CH_2-(OCH_2CH_2)_n-CONH-CH_2CH_2-(OCH_2CH_2)_nY^2$;
$-(CHR^5)_m-NHCO-(CH_2)_m-Y^2$;
$-(CHR^5)_m-CONH-(CH_2)_m-Y^2$;
$-(CHR^5)_m-NHCO-CH_2CH_2-(OCH_2CH_2)_n-Y^2$;
$-(CHR^5)_m-CONH-CH_2CH_2-(OCH_2CH_2)_n-Y^2$;
$-(CHR^5)_m-Y^2$; or $-CH_2-CH_2-(O-CH_2-CH_2)_n-Y^2$ can be prepared from respectively corresponding amines.

Amines preferable for synthesis of a hyaluronic acid-peptide conjugate of the present invention are diamines ($H_2N-(CHR^5)_m-NH_2$ and $H_2N-CH_2-CH_2-(O-CH_2-CH_2)_n-NH_2$). The above-mentioned diamines are commercially available from, for example, Sigma-Aldrich Chemicals and can be arbitrarily bought to be used. Further, they may be synthesized according to methods described in literatures or referring to methods described in literatures.

As the above-mentioned solvent, a polar organic solvent is preferable and an aprotic polar solvent is preferable in particular. When a mix solvent of an aprotic polar solvent with water or water is used as a solvent, it is difficult to obtain an introduction rate necessary for imparting residential property enough for practical use even if any condensing agent is used. The aprotic polar solvent that may be used includes dimethylformamide (hereinafter, also referred to as "DMF"), dimethylacetamide (hereinafter, also referred to as "DMAc"), dimethylsulfoxide (hereinafter, also referred to as "DMSO"), 1,3-dimethyl-2-imidazolidinone (hereinafter, also referred to as "DMI"), sulfolane (hereinafter, also referred to as "SF"), N-methylpyrrolidone (hereinafter, also referred to as "NMP") or a mix solvent of 2 or more of these, etc. Dimethylformamide, dimethylacetoamide, dimethylsulfoxide, N-methylpyrrolidone or a mix solvent of 2 or more of these is preferable and dimethylsulfoxide is preferable in particular.

The concentration of reaction substrates in the above-mentioned reaction solvent is not specifically limited, but

the molar concentration of the repeating unit of N-acetylglucosamine-glucuronic acid in hyaluronic acid being the reaction substrate can be selected from a range of, for example, 0.025 to 250 mmol/L and preferably 0.25 to 50 mmol/L.

Reaction temperature is not specifically limited, but can be selected from a range of, for example, 4 to 80°C, preferably 4 to 40°C and more preferably 20 to 40°C.

As the condensing agent, a condensing agent indicated by the formula (V):

[Formula 18]

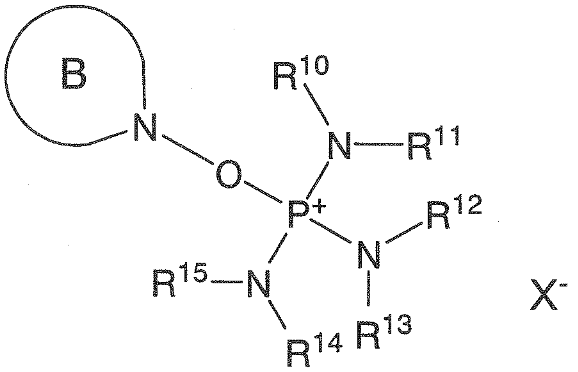

(V)

wherein $R^{10}$, $R^{11}$, $R^{12}$, $R^{13}$, $R^{14}$, $R^{15}$, a ring B and X-are as defined already can be used. The ring B is not specifically limited in so far as it is a nitrogen-containing hetero ring group not having acidic proton and may be optionally substituted with a substituent such as a $C_{1-6}$ alkyl group or a halogen atom. Examples of the ring B include pyrrolidin-2,5-dion-l-yl, 3,4-dihydro-3-hydroxy-4-oxo-1,2,3-benzotriazine, benzotriazol-1-yl and the like. Examples of $X^-$ include halide ion such as fluoride ion, chloride ion, bromide ion and iodide ion, $CF_3SO_3$-, $BF_4^-$, $PF_6^-$ and the like.

The nitrogen-containing hetero ring which $R^{10}$ and $R^{11}$, $R^{12}$ and $R^{13}$ and $R^{14}$ and $R^{15}$ form together with a nitrogen atom with which those are bound is preferably a 5 to 7-membered saturated nitrogen-containing hetero ring and includes specifically pyrrolidine, piperidine, homopiperidine and the like.

The condensing agent is preferably a BOP-type condensing agent in which the ring B is benzotriazol-1-yl. The preferable BOP-type condensing agent includes benzotriazol-1-yloxy-tris(dimethylamino)phosphonium hexafluorophosphate (hereinafter, also referred to as "BOP"), benzotriazol-1-yloxy-tris(pyrrolidino)phosphonium hexafluorophosphate (hereinafter, also referred to as "PyBOP") and a mixture thereof. The BOP-type condensing agent can be utilized alone or arbitrarily utilized in combination.

When hyaluronic acid is reacted with diamines, equivalent or more than equivalent amount of diamines against a carboxylic acid contained in hyaluronic acid, namely, the repeating unit (one unit) of N-acetylglucosamine-glucuronic acid in hyaluronic acid is preferably used to be reacted, and a molar ratio of (diamines)/(HA unit) is selected from a range of, for example, 1 to 1000, preferably 20 to 750 and more preferably 50 to 500. Further, the molar ratio (condensing agent/HA unit) of the condensing agent used in case of introducing the diamines is arbitrary adjusted depending on the reactivity of an amino group of the diamines, but is selected form a range of, for example, 1 to 10, preferably 1 to 5 and more preferably 1 to 3.

Hyaluronic acid (HA) available for the above-mentioned production step is not specifically limited in so far as it has an HA skeleton and includes an HA derivative in which the portion of HA is derivatized, HA whose molecular weight is lowered by enzyme, thermal decomposition and the like, HA and a salt of an HA derivative (a sodium salt, a potassium salt, a magnesium salt, a calcium salt, an aluminum salt and the like). HA used in the present invention may be any HA by whatever method it is obtained, and its origin such as HA extracted from animal tissue, HA obtained by fermentation method, HA obtained by chemical synthesis and the like is not limited. Further, those which are ion-exchanged with highly hydrophobic counter ion such as tetrabutylammonium salt may be used in order to solubilize HA into organic solvents. As the hyaluronic acid derivative capable of being used in the above-mentioned production step, for example, those which are partially alkylated, alkyl esterified or deacetylated are included.

The introduction rate of the N-substituted amide group may be quantified by proton NMR. Specifically, it can be determined by comparison of a peak attributed to an amino compound in the aminated HA with a peak derived from HA which is obtained by proton NMR. For example, there is measured the ratio of a peak (2.9 to 3.1 ppm: measurement solvent is $D_2O$) derived from an amine compound which is obtained by the proton NMR of the hyaluronic acid modification product (hereinafter, also referred to as "HA-AM") which introduced an amino group with ethylenediamine (hereinafter, also referred to as "EDA") to a peak derived from HA (1.8 to 1.9 ppm: measurement solvent is $D_2O$).

The hyaluronic acid modification product which introduced an amino group (hereinafter, also referred to as "HA-AM") can be used as the precursor of the hyaluronic acid-peptide conjugate of the present invention by introducing an appropriate substituent to an amino group. The hyaluronic acid modification product which is obtained by reacting hyaluronic acid or a hyaluronic acid derivative with diamines by the above-mentioned method is a hyaluronic acid modification product in which an amino group is introduced, containing a repeating unit represented by, for example, the formula (VId):

[Formula 19]

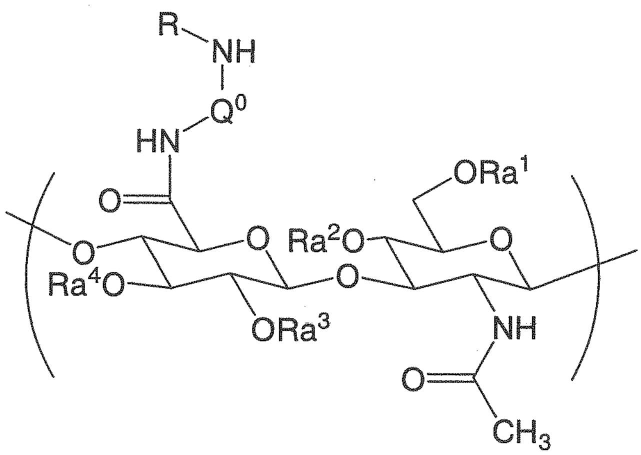

(VId)

wherein $Ra^1$, $Ra^2$, $Ra^3$, $Ra^4$, $Q^0$ and R are as defined already, and may be used as the precursor of the hyaluronic acid-peptide conjugate of the present invention.

The preparation method of the hyaluronic acid-peptide conjugate related to the present invention can use methods already known which are used in the conjugation of the medicine with a polymer. For example, there can be utilized the dehydration condensation reaction of the carboxyl group of the GLP-1 analogue with the amino group of the HA modification product; the dehydration condensation reaction of the carboxyl group of the water-soluble HA modification product with the amino group of the GLP-1 analogue; the reaction of the amino group of the water-soluble HA modification product with the reactive group of GLP-1 analogue in which a reactive group was introduced as isothiocyanate, isocyanate, acylazide and N-hydroxysuccinimide (hereinafter, also referred to as "NHS") ester and epoxide; the reaction of the amino group of the GLP-1 analogue with the reactive group of water-soluble HA modification product in which a reactive group was introduced as isothiocyanate, isocyanate, acylazide and NHS ester and epoxide and the like; the formation of Shiff's base and reductive amination of the amino group of the water-soluble HA modification product with the reactive group of GLP-1 analogue in which a reactive group was introduced as aldehyde and ketone and the like by carbonylation; the formation of Shiff's base and reductive amination of the amino group of the GLP-1 analogue with the reactive group of water-soluble HA modification product in which a reactive group introduced as aldehyde and ketone an the like by carbonylation; the reaction of a mercapto group introduced in the water-soluble HA modification product with the reactive group of GLP-1 analogue in which a reactive group was introduced as maleimide, acryl ester, acryl amide, methacryl ester, methacryl amide, allyl, vinyl sulfone and mercapto and the like; the reaction of a mercapto group introduced in the GLP-1 analogue with the reactive group of water-soluble HA modification product in which a reactive group was introduced as maleimide, acryl ester, acryl amide, methacryl ester, methacryl amide, allyl, vinyl sulfone and a mercapto and the like; the conjugation of avidin introduced in the water-soluble HA modification product with biotin introduced into the GLP-1 analogue; the conjugation of avidin introduced in the GLP-1 analogue with biotin introduced into the water-soluble HA modification product; and the like. A compound having in a molecule 2 or more and preferably 2 groups which are arbitrarily selected from these reactive groups and a carboxyl group (the carboxyl group may be optionally an active ester such as the NHS ester) is used for introducing these modification groups (including avidin and biotin), and an intramolecular structure other than these groups is not specifically limited in the compound in so far as disadvantageous reaction does not proceed until a conjugate is prepared. The compound may be commercially available as a reagent or may be synthesized referring to methods known in literatures.

Specifically, HA modified product which is incorporated N-substituted amide group with amino group (HA-AM) is synthesized, and the portion of the amino group is reacted with N-succinimidyl 3-[2-pyridylthio]propionate (SPDP) to prepare HA introduced mercapto groups (hereinafter, also referred to as "HA-SH"). At this time, it is preferable that

residual amino groups are treated with, for example, succinic anhydride and the like to be converted to carboxyl groups and total charge is anionic. On the other hand, maleimide, vinyl sulfone, acryloyl, methacryloyl, allyl and the like are introduced to the GLP-1 analogue as a reactive group specifically reacts with a mercapto group. This may be reacted with HA-SH to prepare a conjugate.

Further, inversely, the conjugate of the present invention may be prepared by introducing a double bond by modifying the amino group of the water-soluble HA modification product in which the substituent of a N-substituted amide group contains an amino group and by being reacted with the GLP-1 analogue containing a mercapto group. The water-soluble HA modification product in which a reactive group containing a double bond is introduced can be prepared, for example, by reacting a hyaluronic acid modification product containing a repeating unit represented by the formula (VId):

[Formula 20]

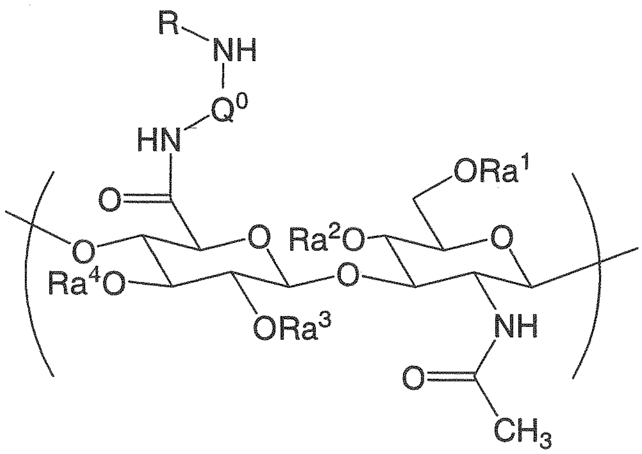

(VId)

wherein $Ra^1$, $Ra^2$, $Ra^3$, $Ra^4$, $Q^0$ and R are as defined already, in the molecule with an appropriate reagent and by converting it to the formula (VIb):

[Formula 21]

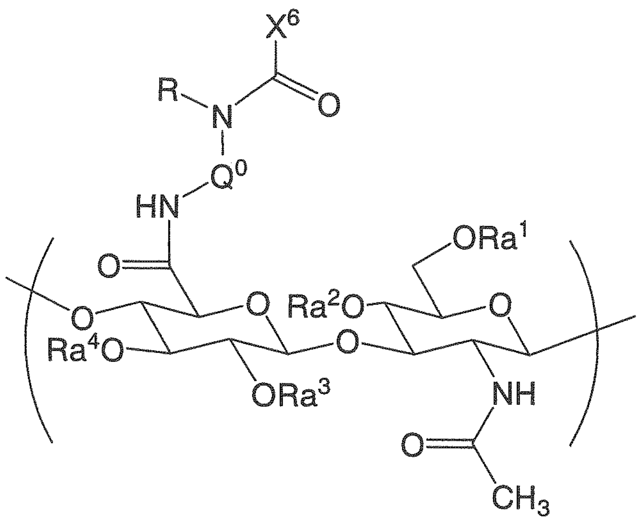

(VIb)

wherein $Ra^1$, $Ra^2$, $Ra^3$, $Ra^4$, Q°, R and $X^6$ are as defined already. Examples of the reagents capable of being used in the above-mentioned reaction include acrylic chloride (when $X^6$ is $-CH=CH_2$), methacrylic chloride (when $X^6$ is $-C(CH_3)=CH_2$), N-(γ-maleimidobutyryloxy)sulfosuccinimide ester, N-(κ-maleimidoundecanoyloxy)sulfosuccinimide ester (both are when $X^6$ is $-(CH_2)_m-Y^2$), and N-hydroxysuccinimidyl-15-(3-maleimidopropionyl)-amido-4,7,10,13-tetraoxapentadecanoate (hereinafter, also referred to as "NHS-$(EO)_4$-MI") and N-hydroxysuccinimidyl-75-(3-maleimidopropionyl)-amido-4,7,10,13,16,19,22,25,28,31,34,37,40,43,46,49,52,55,58,61,64,6 7,70,73-tetracosaoxapentaheptacontanoate (both when $X^6$ is $-CH_2-CH_2-(O-CH_2-CH_2)_n-Y^2$). The residual amino groups may be treated with, for example, succinic anhydride and the like.

On the other hand, cysteine may be introduced into the GLP-1 analogue or a linker having a mercapto group may be reacted and this may be reacted with HA-maleimide to prepare a conjugate. Among these, the reaction of a maleimide group with a mercapto group is preferable in particular and, for example, conjugation by reaction of the mercapto group of cysteine introduced into the GLP-1 analogue with a maleimide group introduced with NHS-$(EO)_4$-MI at the portion of HA-AM is preferable. The introduction rate of the GLP-1 analogue is preferably 0.1% by mol or more and 15% by mol or less per one molecule of HA in average. When the introduction rate is low, HA weight fraction occupying a dose is relatively increased and when a high dose of the GLP-1 analogue is administrated, the concentration of HA is raised at an actually acceptable administration volume and viscosity is high to be difficult in administration. On the other hand, when the introduction rate is too high, a conjugate is easily insolubilized.

Further, the amino group remaining in HA-AM after introduction of a functional group for a conjugate such as a maleimide group used in the present invention is treated with dicarboxylic anhydride such as, for example, succinic anhydride, maleic anhydride, glutaric anhydride and adipic anhydride, before the conjugation of the GLP-1 analogue, or dicarboxylic acid such as maleic acid, glutaric acid and adipic acid is reacted in the presence of a condensing agent, and thereby it is preferable for the elongation of residence time in blood that a terminal functional group is returned to a carboxyl group and total charge is made anionic. In particular, succinic anhydride is preferable. The condensing agent used is not specifically limited, and for example, there can be used benzotriazol-1-yloxy-tris(dimethylamino)phosphonium hexafluorophosphate, benzotriazol-1-yloxy-tris(pyrrolidino)phosphonium hexafluorophosphate, N,N'-carbonyldiimidazole, N.N'-dicyclohexylcarbodiimide, 1-ethyl-3-(3-dimethylaminopropyl)carbodiimides hydrochloride, EDC/3,4-dihydro-3-hydroxy-4-oxo-1,2,3-benzotriazine, N-ethoxycarbonyl-2-ethoxy-1,2-dihydroquinoline, 4-(4,6-dimethoxy-1,3,5-triazin-2-yl)-4-methylmorpholium chloride n-hydrate, 2-(1H-benzotriazol-1-yl)-1,1,3,3-tetramethyluronium tetrafluoroborate and a mixture thereof.

Further, from the viewpoint as the hyaluronic acid modification product for obtaining practically sufficient residence time in blood at preparing a conjugate with the GLP-1 analogue, the molecular weight of the water-soluble HA modification product used for the present invention also affects its pharmacokinetic disposition. The molecular weight of the raw material HA used for the present invention is not specifically limited but when molecular weight is too low, the residence time of the obtained HA modification product in blood is short. Inversely, when molecular weight is too high, the viscosity of the water-soluble HA modification product obtained is very high and administration at high concentration is difficult. Further, when the molecular weight is larger than a certainvalue, the residence time in blood of the water-soluble HA modification products used for the present invention are settled; therefore the molecular weight of the raw material HA is preferably a viscosity average molecular weight of 5000 dalton to one million dalton, more preferably 10000 dalton to 300000 dalton and further preferably 80000 dalton to 300000 dalton.

Various known methods such as a light scattering method, an osmotic pressure method and a viscosity method which are described in, for example, "Essential Polymer Science" edited by Seiichi Nakahama et. al. (published by Kodansha Ltd, ISBN4-06-153310-X) can be utilized for the measurement method of the weight average molecular weight of HA. The viscosity average molecular weight indicated in the present specification can be measured by methods which are usually used in a technical field to which the present invention belongs, using an Ubbelohde viscometer and the like.

The residence time of the water-soluble HA modification product used for the present invention in blood is preferably those in which the mean residence time in the blood for a rat is 18 hours or more and more preferably 25 hours or more.

Further, according to another aspect of the present invention, the water-soluble HA modification product used for the present invention is resistant against degradation by hyaluronidase in comparison with the raw material HA. The phrase "is resistant against degradation by hyaluronidase" indicates that when each of the raw material HA and the water-soluble HA modification product of the present invention is enzyme-degraded by hyaluronidase, it has property that degradation speed is lower than the raw material HA or degradation does not proceed. For example, when 0.4 unit of hyaluronidase is added to 1 mg of the HA modification product and hyaluronidase treatment is carried out at 37°C for 24 hours, it can be judged as having property of "degradation does not proceed" (namely, being resistant against degradation by hyaluronidase) when disaccharide degraded product is less than that observed in the raw material HA, or unless the disaccharide degraded product is not observed. Specifically, the water-soluble hyaluronic acid modification product is digested by hyaluronidase which can degradade hyaluronic acid to disaccharide being its compositional unit and produce unsaturated disaccharide degraded product (for example, including the compound of the formula (IV)):

[Formula 22]

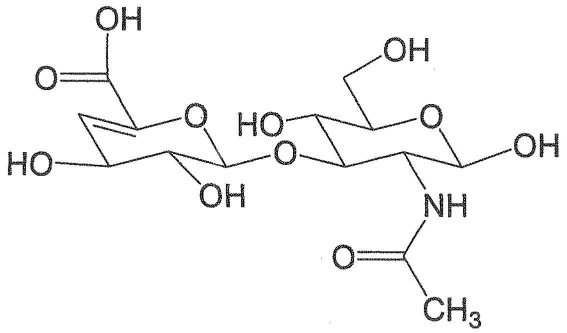

(IV)

which has Δ-4,5-glucuronic acid at the non-reducing end of the product, and the absorption peak at 232 nm of the product obtained is measured to be able to be judged. The measurement can be carried out by methods which usually used in the art, using conventional high performance liquid chromatography. For example, a gel permeation chromatography (GPC) column (for example, those in which Superdex 200 10/300 GL, Superdex 75HR 10/30 and Superdex Peptide HR 10/30) (either is manufactured by Amasham Bioscience Co.) are connected, etc.) can be used. Eluent used is not specifically limited, but for example, PBS (for example, 2 tablets of Phosphate Buffered Saline Tablets manufactured by Sigma-Aldrich Chemicals are taken out and dissolved in 400 mL of purified water to prepare eluent) can be used.

As the water-soluble hyaluronic acid modification product used in the present invention, the upper limit of fraction of a peak area derived from disaccharide to all peak area derived from the degraded product is preferably 30% or less in case of measurement by the above-mentioned method. 20% or less is more preferable and 13% or less is further preferable. Further, the lower limit may be 0% or more.

The fraction of a area derived from disaccharide to all peak area derived from the degraded product can be determined by the equation below:

[Formula 23]

$$\text{Fraction of disaccharide (\%)} = \frac{\text{Peak area of disaccharide}}{\text{All peak area of degraded solution} - \text{All peak area at no addition of enzyme}} \times 100$$

Further, as hyaluronidase, for example, Hyaluronidase SD (manufactured by SEIKAGAKU CORPORATION) and the like can be used.

The water-soluble HA modification product used in the present invention is not specifically limited from the viewpoint of a hyaluronic acid modification product for obtaining practically sufficient residence time in blood at preparing a conjugate with the GLP-1 analogue, but has a solubility of, for example, 10 mg/mL to 100 mg/mL for saline at room temperature. The concentration of the HA modification product at clinical administration for treatment is preferably 50 mg/mL or lower.

In the present specification, a"$C_{1-6}$ alkyl group" means a linear chain and branched chain alkyl group having 1 to 6 carbons, and examples include a "$C_{1-4}$ alkyl group" such as methyl, ethyl, n-propyl, isopropyl, n-butyl, sec-butyl, isobutyl and tert-butyl, and further, n-pentyl, 3-methylbutyl, 2-methylbutyl, 1-methylbutyl, 1-ethylpropyl, n-hexyl, 4-methylpentyl, 3-methylpentyl, 2-methylpentyl, 1-methylpentyl, 3-ethylbutyl and 2-ethylbutyl, etc.

In the present specification, a"$C_{1-6}$ alkylcarbonyl group" means an alkylcarbonyl group having a linear chain

and branched chain alkyl group having 1 to 6 carbons, and examples include acetyl, propionyl, butyryl, isobutyryl, pivaloyl, valeryl, isovaleryl, hexanoyl and the like.

In the present specification, a "$C_{1-400}$ alkylene group" means a linear chain alkylene group having 2 to 400 carbons, and examples include a $C_{1-200}$ alkylene group, a $C_{1-100}$ alkylene group, a $C_{1-50}$ alkylene group, a $C_{l}$-$_{20}$ alkylene group, a $C_{1-10}$ alkylene group and the like. When an oxygen atom may be inserted between two carbons contained in the alkylene group at one or more of the alkylene groups, the definition includes an ethylene oxide represented by, for example, $-CH_2CH_2-(OCH_2CH_2)_n-$ (wherein n is an integer selected from 0 to 199) and the like.

In the present specification, natural amino acid includes α-amino acid such as glycine, alanine, serine, proline, valine, threonine, cysteine, leucine, isoleucine, asparagine, aspartic acid, lysine, glutamine, glutamic acid, methionine, histidine, phenylalanine, arginine, tyrosine and tryptophan; additionally, β-amino acid such as β-alanine, γ-amino acid such as γ-aminobutyric acid and aminosulfonic acid such as taurine etc. Further, non-natural α-amino acid includes α-amino acid having an alkyl side chain (for example, norvaline, norleucine, t-leucine and the like), alanine and glycine substituted with an cycloalkyl group (for example, cyclopentylalanine, cyclohexylalanine, cyclohexylglycine and the like), or alanine and glycine substituted with an aryl group (for example, pyridylalanine, thienylalanine, naphthylalanine, substituted phenylalanine, phenylglycine and the like).

The introduction amount of a substituent introduced in the present invention can be adjusted by the addition amount of a condensing agent to HA.

Further, when the charge of the water-soluble HA modification product used in the present invention is cationic after formation of a conjugate with the GLP-1 analogue, residence time in blood is shortened by non-specific interaction with biological membrane and the like; therefore it is preferable to be converted to nonionic or anionic by being reacted with an acid anhydride, a lactone, alactide or a compound containing active ester or the like.

In the preparation method of the water-soluble HA modification product used in the present invention, for example, HA which was converted to tetrabutylammonium salt (TBA) is dissolved in DMSO, a diamine compound having amino groups at the both termini of a molecule is added to be condensed with the carboxyl group of HA by a BOP-type condensing agent and thus, HA introducing an amino group (hereinafter, also referred to as "HA-AM") can be synthesized.

The synthesis of a conjugate with the GLP-1 analogue is preferably site specific conjugation from the viewpoint of avoiding the lowering of activity.

Examples of the GLP-1 analogue of the present invention include a peptide with an amino acid sequence of GLP-1 (1-36) or GLP-1 (7-36) added with an amino acid sequence represented by -Xa-Cys at the C-terminal thereof, or a peptide with the amino acid sequence of the peptide in which one or more amino acids (for example 1 to 10, preferably 1 to 5 amino acids) are deleted, substituted and/or added in the amino acid sequence wherein the amino acid sequence may be optionally substituted and/or added with a natural amino acid and/or a non-natural amino acid, in which Xa is a direct bond or a sequence comprising one or more amino acids (for example 1 to 9, preferably 1 to 4 amino acids) selected from natural amino acids and non-natural amino acids acid, wherein the carboxyl group of cysteine of the C-terminal of the peptide may be optionally converted to an amide group.

Further, the GLP-1 analogue also includes a peptide with an amino acid sequence of GLP-1 (1-36), GLP-1 (1-37), GLP-1 (7-36) or GLP-1 (7-37)added with a mercapto compound represented by -Qa-SH at the C-terminal thereof, or a peptide with the amino acid sequence in which one or more amino acids (for example, 1 to 10 and preferably 1 to 5 amino acids) are deleted, substituted and/or added in the amino acid sequence. The substitution of the amino acid sequence may be optionally substituted with a natural amino acid and/or a non-natural amino acid,

wherein Qa is selected from $-NH-X^5-$, $-CO-X^5-$or $-CONH-X^5-$ and is linked with a carboxyl group, an amino group or a hydroxyl group which is contained in the C-terminal amino acid of the peptide, to form an amide bond, an urea bond or an ester bond, and

$X^5$ is a $C_{1-50}$ alkylene group in which an oxygen atom may be inserted between two carbon atoms contained in the alkylene group at one or more of sites of the alkylene group and the carbon atom of the alkylene group may be optionally substituted with one or more of substituents selected from a hydroxy group and a $C_{1-6}$ alkyl group independently.

In one aspect of the present invention, GLP-1 analogue indicates analogue which is native GLP-1 such as GLP-1 (1-37), GLP-1 (7-37) and GLP-1 (7-36) with deleted, substituted and/or added 1 to 5 amino acids, and analogue in which the amino acid of the C-terminal of the analogue is substituted with the sequence of 1 to 10 amino acids, or GLP-1 analogue in which a mercapto compound represented by $-X^5-SH$ is added to an amino group excluding α-amino group, a carboxyl group or a hydroxyl group through an amide bond, an urea bond or an ester bond. The amino acid added may be natural type or non-natural type.

The GLP-1 analogue is preferably analogue imparting substitution which is expected to exhibit DPPIV resistance and a mercapto group as a site-specific conjugation site. Specifically, among analogues in which alanine (at 8 position) of native GLP-1 is substituted with other natural amino acid or non-natural amino acid, there are preferably the GLP-1 analogues (cysteine of the C-terminal may be amidated) in which the amino acid of the C-terminal of the analogue is substituted with an amino acid sequence represented by $-X^1-Cys$ ($X^1$ indicates a direct bond or a sequence comprising 1 to 9 of amino acids independently selected from proline, glycine, serine and glutamic acid), or the GLP-1 analogue in

which a mercapto compound represented by $-X^5$-SH was added to an amino group excluding the α-amino group of the C-terminal amino acid, a carboxyl group or a hydroxyl group through an amide bond, an urea bond or an ester bond.

Preferable analogues are native GLP-1 (Human 7-37; described in Japanese Patent Application National Publication (Laid-Open) No.7-504679) analogues and examples include GLP-1 (7-36) $-X^2$-Cys, GLP-1 (7-36) $-X^2$-$CysNH_2$, [$Gly^8$]-GLP-1 (7-36) - $X^2$-Cys, [$Gly^8$]-GLP-1 (7-36) $-X^2$-$CysNH_2$, [$Ser^8$]-GLP-1 (7-36) $-X^2$-Cys, [$Ser^8$]-GLP-1 (7-36) $-X^2$-$CysNH_2$, [$Val^8$]-GLP-1 (7-36) $-X^2$-Cys, [$Val^8$]-GLP-1 (7-36) $-X^2$-$CysNH_2$, [$Leu^8$]-GLP-1 (7-36) $-X^2$-Cys, [$Leu^8$]-GLP-1 (7-36) $-X^2$-$CysNH_2$, [$Ile^8$]-GLP-1 (7-36) $-X^2$-Cys, [$Ile^8$]-GLP-1 (7-36) $-X^2$-$CysNH_2$, [$Thr^8$]-GLP-1 (7-36) $-X^2$-Cys, [$Thr^8$]-GLP-1 (7-36) $-X^2$-$CysNH_2$ (wherein $X^2$ represents a direct bond or -Gly-Pro-Pro-Pro-), GLP-1 (7-37) NH-$X^5$-SH, GLP-1 (7-36) -Lys-ε-NHCO-$X^5$-SH, GLP-1 (7-36) -$LysNH_2$-ε-NHCO-$X^5$-SH, [$Gly^8$]- GLP-1 (7-37) NH-$X^5$-SH, [$Gly^8$] -GLP-1 (7-36) -$LysNH_2$-ε-NHCO-$X^5$-SH (wherein $X^5$ is $-(CH_2)_n-$, a direct bond or $-(CH_2CH_2O)_m-$, and n and m are an integer selected from 2 to 20), and the like. Further, the above-mentioned $CysNH_2$ represents cysteine in which a carboxyl group is amidated and $LysNH_2$ represents lysine in which a carboxyl group is amidated.

In particular, there are preferably an analogue in which alanine (at 8 position) of native GLP-1 is converted to glycine and glycine (at 37 position) is substituted to cysteine and an analogue in which alanine (at 8 position) of native GLP-1 is converted to glycine and glycine (at 37 position) is changed to glycine-proline-proline-proline-cysteine. Further, the C-terminal of the GLP-1 analogue may be optionally amidated by known methods.

GLP-1 analogues of the present invention may be prepared by standard liquid-phase or solid-phase chemical synthesis, recombinant DNA techniques, cell-free protein synthesis or any other methods of preparing amino acid sequences.

The conjugate of the present invention can be administrated in any appropriate form as a pharmaceutical composition which contains one or more of pharmacologically acceptable diluent, wetting agent, emulsion, dispersant, auxiliary agent, antiseptic agent, buffer, binder, stabilizer and the like, in accordance with an intended administration route.

The pharmaceutical composition containing the conjugate of the present invention can be parenterally administrated systematically or locally. For example, intravenous administration such as drip, intramuscular administration, intraperitoneal administration, subcutaneous administration, intranasal administration, pulumonary administration and the like can be selected and administration method can be suitably selected depending on the age of a subject and symptom. Effective dose differs depending on administration route and administration frequency. Since 2 pM to 20000 pM is estimated as plasma concentration for obtaining medical benefits while avoiding adverse reactions (nausea, vomiting and the like), a dose is preferably adjusted so as to be the plasma concentration.

The diabetic complication which can be applied in the present invention is not specifically limited, and for example, diabetic retinopathy, diabetic nephropathy, diabetic neuropathy, arteriosclerosis, cardiac infarction, brain infarction, diabetic foot and the like.

The present invention makes it possible to provide a long-acting diabetic pharmaceutical which is incapable of producing by conventional methods.

The present invention also makes it possible to provide the conjugate of GLP-1 analogue, which has prolonged residence time in blood and is suitable for practical use and safe.

[Examples]

Suitable examples of the present invention will be described below in detail, but the present invention is not limited to these examples.

NMR measurements were carried out using a nuclear magnetic resonance spectrometer system, JNM-ECA500 (JEOL Co. Ltd.). The condition for the measurement is shown below:

Data point: 16384
Spectral width (X sweep): 15 ppm
Acquisition time (X acq time): 1.749 s
Pulse delay (Relaxation delay): 30 s
Transients (Scans): 64
Temperature: room temperature
Solvent for measurement: $D_2O$
HA units, in the following description, means a repeating unit (1 unit) of N-acetyl glucosamine-glucuronic acid in hyaluronic acid.

[Example 1]

Synthesis of hyaluronic acid modification products in an aprotic polar solvent (condensing agent, digestion by enzyme)

Various synthetic conditions were investigated in an aprotic solvent, and hyaluronic acid modification products obtained in each condition were evaluated for enzyme resistance.

(Example 1-1)

Sodium hyaluronate of molecular weight 200 kDa (HA: DENKA Co. Ltd.) was converted to tetra-butyl ammonium (TBA) salt using DOWEX 50WX8-400 (Sigma-Aldrich Co. Ltd.), which was converted to TBA salt form by tetra-butyl ammonium hydroxide (Sigma-Aldrich Co. Ltd.). 29.1 mg of Tetra-butyl ammonium salt of hyaluronate (hereinafter also called "HA-TBA") thus obtained was dissolved in DMSO (Wako Pure Chemical Industries, Co. Ltd.) at a concentration of 2.0 mg/mL. Ethylenediamine (hereinafter also called "EDA"; Sigma-Aldrich Co. Ltd.) and BOP (Wako Pure Chemical Industries, Co. Ltd.) were added in this order at the equivalence ratio of HA unit/BOP/EDA = 1/2.5/100 (mol/mol/mol), and the mixture was reacted at room temperature overnight. After adding 10 mL of 1 M Nacl aqueous solution, 5 N HCl was added to bring down pH to 3 and then the mixture was neutralized by adding 2 N NaOH. The mixture was dialyzed/purified (Spectra/por4, cut off molecular weight (hereinafter also called "MWCO"): 12 k - 14 kDa) against a large excess amount of distilled water (Milli Q water), subjected to ultrafiltration (YM-10, Millipore Co. Ltd.) and freeze dried to obtain 25.8 mg of hyaluronic acid (hereinafter also called "HA-AM") to which the designated amino group was incorporated.

(Example 1-2)

27.2 mg of hyaluronic acid (HA-AM) to which amino group was incorporated was obtained using the similar method to that in Example 1-1 except that 2,2'-(ethylenedioxy) bis(ethylamine) (hereinafter also called "EDOBEA": Sigma-Aldrich Co. Ltd.) was used in place of ethylenediamine, 35.1mg of HA-TBA was used, and the reaction was carried out at the equivalence ratio of HA unit/BOP/EDOBEA = 1/2.5/50 (mol/mol/mol).

(Example 1-3)

41.1 mg of hyaluronic acid (HA-AM) to which amino group was incorporated was obtained using the similar method to that in Example 1-2 except that hexamethylenediamine (HMDA: Sigma-Aldrich Co. Ltd.) was used in place of EDOBEA and 62.0 mg of HA-TBA was used.

(Example 1-4)

39.0 mg of hyaluronic acid (HA-AM) to which amino group was introduced was obtained using the similar method to that in Example 1-2 except that PyBOP (Kokusan Chemical Co. Ltd.) was used in place of BOP and 51.8 mg of HA-TBA was used.

(Test example 1)

Evaluation for enzyme degradation

HA-AM obtained in Examples 1-1 to 1-4 was dissolved in distilled water (Milli Q water) at a concentration of 2 mg/mL. To 55 μL of this solution, 132 μL of 0.2 M phosphate buffer (pH 6.2) and 77 μL of water were added and then 44 μL of 1 U/mL solution of hyaluronidase SD (SEIKAGAKU CORPORATION Co. Ltd.) (0.05M phosphate buffer (pH 6.2) containing 0.01% BSA) was added and the mixture was incubated at 37°C for 24 hours. 100 μL of each sample was withdrawn and the reaction was terminated by adding 720 μL of 50 mM acetic acid solution. As the control, a sample group without the enzyme addition was treated in the same way (data omitted). Each sample was subjected to gel permeation chromatography (hereinafter also called "GPC") to observe the change in the molecular weight of HA-AM and the generation pattern of the degradation products (absorption at 232 nm). The condition for GPC is shown below.

GPC Column: Superdex 200 10/300 GL, Superdex 75HR 10/30, Superdex PeptideHR 10/30 (Amersham Bioscience Co. Ltd.) (3 columns connected)

Eluent: PBS (pH 7.4)
Flow Rate: 0.4 mL/min
Injection Volume: 50 μL

Detection: UV (232 nm)
The disaccharide degradation product peak (Retention Time: 130 minutes) was not observed. GPC chromatograms are shown in Figure 1 (Examples 1-1 to 1-4).

Further, the rates of amino group incorporation in the Examples 1-1 to 1-4 were measured by the proton NMR method (HA: methyl proton of N-acetyl group, 1.8-1.9 ppm, AM: methylene proton juxtaposing free amino group, 2.9-3.1 ppm). Proton NMR spectra are shown in Figure 2. The rates of amino group incorporation were 97.5, 75.5, 88.3 and 84.5%, respectively.

[Example 2]

Evaluation for hyaluronidase resistance of calboxylated HA modification product

To evaluate the enzyme resistance in more detail, HA modification products were synthesized by converting the incorporated primary amine to succinamide (HA-AM-SUC). Thus obtained HA modification products were evaluated for enzyme resistance.

(Example 2-1)

Synthesis of HA modification product to which EDOBEA is incorporated

Six DMSO solutions of HA (200 kDa)-TBA (4.0 mg/mL) were prepared and to each solution, 2,2'-(ethylenedioxy) bis(ethylamine) (EDOBEA) was added at the equivalence ratio of HA unit/EDOBEA = 1/50 (mol/mol), and BOP was added at the equivalence ratio to HA unit, 0.4, 0.6, 0.8, 1.0, 1.5, 1.85 or 2.5, and the mixtures were reacted overnight. After adding 1 M NaCl aqueous solution in a half the volume of the reaction mixture, 5 N HCl was added to bring down pH to 3 and then the mixture was neutralized by adding 2 N NaOH. The mixture was dialyzed against 0.3 M NaCl solution and then dialyzed/purified (Spectra/por 4, cut off molecular weight (MWCO): 12 k - 14 kDa) against a large excess amount of distilled water (Milli Q water) and freeze dried to obtain HA modification product to which EDOBEA is incorporated.

(Example 2-2)

Conversion of primary amine to succinamide

Samples obtained as above (Example 2-1) were dissolved in distilled water (Milli Q water) to prepare 20.0 mg/mL solution, and 0.2 M carbonate buffer (pH 9.0) was added thereto to bring the concentration to 10.0 mg/mL. To this solution was added, DMSO solution of 1/10 volume of HA-AM solution containing succinic anhydride (Wako Pure Chemical Industries, Co. Ltd.) at 20 times molar amount of the HA units in the solution was added. The mixture was stirred at room temperature for 30 minutes. The mixture was dialyzed against 0.3 M NaCl aqueous solution, and then dialyzed/purified (Spectra/por4, cut off molecular weight (MWCO): 12 k - 14 kDa) against a large excess amount of distilled water (Milli Q water) and freeze dried to obtain HA-AM-SUC. NMR spectra are shown in Figure 3. The methylene peak (2.9-3.1) next to the amino group was completely disappeared and a peak (2.4-2.6 ppm) derived from succinic acid was newly observed concomitantly indicating that amino group was converted to succinamide and that carboxy group was newly incorporated.

(Example 2-3)

Evaluation for enzyme degradation

HA-AM-SUC obtained in Example 2-2 was dissolved in distilled water (Milli Q water) at a concentration of 4 mg/mL. To 25 μL of this solution, 200 μL of 0.1 M phosphate buffer (pH 6.2) and 25 μL of water were added, and then 50 μL of 1 U/mL solution of hyaluronidase SD (SEIKAGAKU CORPORATION Co. Ltd.) (in 0.2M phosphate buffer, pH 6.2) was added and the solution was incubated at 37°C for 24 hours. 100 μL of each sample was withdrawn and the reaction was terminated by adding 700 μL of 50 mM acetic acid solution. As the control, a sample group without the enzyme addition was treated in the same way (data omitted). Each sample was subjected to gel permeation chromatography (hereinafter also called "GPC") to observe the change in the molecular weight of HA-AM-SUC and the generation pattern of the degradation products (absorption at 232 nm). The results are shown in Figure 4 and Table 1. The condition for GPC is shown below.

GPC Column: Superdex 200 10/300 GL, Superdex PeptideHR 10/30 (Amersham Bioscience Co. Ltd.) (2 columns connected)

Eluent: PBS (pH 7.4)
Flow Rate: 0.4 mL/min
Injection Volume: 50 μL
Detection: UV (232 nm)

[Table 1]

| Table 1. Relationship between the incorporation rate of amide group and hyaluronidase resistance | |
|---|---|
| Incorporation rate (%) | Ratio of disaccharide peak area to total peak area (%) |
| 18.0 | 88.9 |
| 30.0 | 52.5 |
| 45.5 | 22.5 |
| 56.5 | 11.9 |
| 66.5 | 12.9 |
| 94.5 | 0.0 |
| 96.0 | 0.0 |

Here, among the degradation products, the % of was calculated as

$$100 \times (\text{Peak area of disaccharide})/(\text{Total peak area} - \text{Total peak area when enzyme is not added})\ (\%)$$

in the range where the peak derived from the solvent (Retention Time : 90 minutes) was excluded. The results indicated that the enzyme resistance was increased corresponding to the incorporation rate of amide group, that is, the carboxylation rate of HA.

[Example 3]

Relationship between molecular weight of hyaluronic acid modification product (HA-AM-SUC) and blood residence

(Molecular weight dependency)

To analyze the blood residence of hyaluronic acid modification product (HA-AM-SUC) from the aspect of molecular weight, HA modification products were synthesized from 23 k, 100k and 200 kDa HA, and samples were prepared by incorporating a fluorescent dye, FITC, and the blood retention of each sample was observed.

(Example 3-1)

Synthesis of HA-AM

DMSO solution(2 mg/mL) of HA (23 kDa)-TBA and respective DMSO solutions (4 mg/mL) of HA (100 kDa)-TBA and HA (200 kDa)-TBA were prepared. At the equivalence ratio of HA unit/BOP/2, 2'-(ethylenedioxy) bis (ethylamine) (EDOBEA) = 1/2.5/50 (mol/mol/mol), EDOBEA and BOP was added to each solution in this order, and the mixtures were reacted at room temperature overnight. After adding 1 M NaCl aqueous solution in a half the volume of the reaction mixture, 5 N HCl was added to bring down pH to 3 and then the mixture was neutralized by adding 2 N NaOH. The mixture was dialyzed/purified (Spectra/por 4, cut off molecular weight (MWCO): 12 k - 14 kDa) against a large excess amount of distilled water (Milli Q water) and then ultrafiltered (YM-10, Millipore Co. Ltd.) and freeze dried to obtain N-substituted amidated hyaluronic acid (HA-AM) in which the designated amino group was incorporated. The incorporation

rate of amide group was measured by the proton NMR method. NMR spectra are shown in Figure 5 (HA: methyl proton of N-acetyl group, 1.8-1.9 ppm, AM: methylene proton of EDOBEA part, 2.9-3.1 ppm). The incorporation rate for 23 kDa, 100 kDa and 200 kDa was 87 mol %, 92.5 mol % and 93.5 mol %, respectively.

(Example 3-2)

Synthesis of fluorescently labeled HA modification product

HA-AM obtained as described above (Example 3-1) was dissolved in distilled water (Milli Q water) to prepare 20.0 mg/mL solution, and 0.2 M carbonate buffer (pH 9.0) was added thereto to bring the concentration to 10.0 mg/mL. To this solution was added fluorescein isothiocyanate (hereinafter also called "FITC"; Pierce Co. Ltd.) in an amount of 0.07 mol per HA unit dissolved in DMSO solution of 1/10 volume of HA-AM solution, and the mixture was stirred at room temperature for 1 hour. Then 40 mol per HA unit of succinic anhydride (Wako Pure Chemical Industries, Co. Ltd.) dissolved in DMSO solution of 1/10 volume of the HA-AM solution was added and the mixture was stirred at room temperature for 30 minutes. After crude purification with gel-filtration using PD-10 column (Amersham Bioscience Co. Ltd.), the sample was dialyzed/purified (Spectra/por4, cut off molecular weight (MWCO): 12 k - 14 kDa) against a large excess amount of 0.5 M Nacl aqueous solution. The dialysate was replaced with distilled water (Milli Q water) and the dialysis/purification was continued. Thus obtained dialyzed aqueous solution was freeze dried to obtain fluorescently labeled HA-AM-SUC.

Each of fluorescently labeled HA-AM-SUC obtained here was dissolved in 50 mM carbonate buffer (pH 9.0) at a concentration of 0.25 mg/mL, and the molar concentration of N-fluoresceinyl thiocarbamoyl (FTC) group derived from FITC was measured from the absorbance at 494 nm of the solution, and the concentration of each unit was calculated according to the formulas below. Further, the conversion to mole fraction and the weight fraction of HA in the HA modification product were calculated.
Un-modified HA unit: x nmol/mL
HA-AM-SUC unit: y nmol/mL (a unit in which is incorporated amino group and reacted with succinic anhydride)
[Formula 24]

$$\text{Formula 1: } (401.3 \times x) + (631.58 \times y) + (544.97 \times (\text{Residual AM conc.})) + (898.9 \times (\text{FTC conc.})) = 250\ \mu g$$

$$\text{Formula 2: } x/(y + (\text{Residual AM conc.}) + (\text{FTC conc.})) = ((100\text{-AM}(\%)))/\text{AM}(\%)$$

Wherein residual AM conc. in the formula means the molar concentration of a unit containing un-reacted amino group, and FTC conc. means the molar concentration of a unit containing FTC group.

The results obtained are shown in Table 2.

[Table 2]

Table 2. FITC labeled HA-AM-SUC for Pharmacokinetics Tests

| Molecular weight (kDa) | AM (%) NMR | AM (%) TNBS | Residual Unit (Mol%) | HA-AM-FTC Unit (Mol%) | HA-AM-SUC Unit (Mol%) | Unmodified HA (Wt.%) |
|---|---|---|---|---|---|---|
| 23 | 87.0 | - | 1.0 | 86.0 | 13.0 | 63.7 |
| 100 | 92.5 | - | 0.9 | 91.6 | 7.5 | 62.3 |

(continued)

| Table 2. FITC labeled HA-AM-SUC for Pharmacokinetics Tests | | | | | | |
|---|---|---|---|---|---|---|
| | | | Residual HA-AM-FTC HA-AM-SUC Unmodified HA | | | |
| Molecular weight (kDa) | AM (%) NMR | AM (%) TNBS | Unit (Mol%) | Unit (Mol%) | Unit (Mol%) | HA (Wt.%) |
| 200 | 93.5 | 1.5 | 0.7 | 91.3 | 6.5 | 62.9 |
| -: Below limit of determination | | | | | | |

(Comparative Example 3-1)

Synthesis of fluorescently labeled HA-HZ-SUC

Sodium hyaluronate of 580 kDa (DENKA Co. Ltd.) was dissolved in distilled water at a concentration of 0.25% (W/V), and pH adjusted to 4.7-4.8 with 5 N HC1. 1-Ethyl-3-(3-dimethylaminopropyl) carbodiimide (EDC) and adipinic acid dihydrazide (hereinafter also called "ADH") was added at a molar ratio of HA unit: EDC: ADH = 1:5:40, the mixture was reacted at room temperature for 2 hours while keeping the pH at 4.7-4.8 with 5 N HC1. The reaction mixture was dialyzed (Spectra/por 7, cut off molecular weight (MWCO): 12 k - 14 kDa) against a large amount of 100 mM NaCl solution, 25% aqueous ethanol solution distilled water (Milli Q water) successively and dried to obtain hyaluronic acid incorporated with hydrazide group (HA-HZ). The incorporation rate of HZ in HA-HZ was 73% of carboxyl group of HA, measured as incorporation rate of ADH by the proton NMR method.

After dissolving this HA-HZ in distilled water (Milli Q water), an equal amount of 100 mM carbonate buffer (pH 9.0) was added to adjust the final concentration to 1 mg/mL. To this solution, FITC dissolved in DMSO of 1/10 volume of HA-HZ solution was added at the ratio of FITC/HA unit = 3.0 mol/mol, and reacted at room temperature in the dark for 1 hour. The reaction solution was applied to PD-10 columns (10 columns) which have been equilibrated with 50 mM carbonate buffer (pH9.0) to remove unreacted FITC. Succinic anhydride dissolved in DMSO (3.5 mL) was added to this crude product solution at the input ratio of succinic anhydride/HZ = 250 mol/mol and the reaction was carried out similarly. The reaction product was dialyzed against a large excess amount of distilled water (Milli Q water) and freeze dried to obtain fluorescently labeled HA-HZ-SUC having HA-HZ labeled with FITC.

Each of fluorescently labeled HA-HZ obtained here was dissolved in 50 mM carbonate buffer (pH 9.0) at a concentration of 0.25 mg/mL, and FITC concentration was measured from the absorbance at 494 nm of the solution, and the concentration of each unit was calculated according to the formulas below. Further, the conversion to mole fraction and the weight fraction of HA in the HA modification product were calculated.

Un-modified HA unit: x μmol/mL

HA-HZ-SUC unit: y μmol/mL (a unit which is incorporated hydrazide group and reacted with succinic anhydride)

Those units were defined as above and calculated by the following formula.

[Formula 25]

$$\text{Formula 1: } (379.3 \times x) + (635.57 \times y) + (924.88 \times (\text{FITC conc.})) = 250 \text{ mg}$$

$$\text{Formula 2: } x/(y + (\text{FITC conc.})) = (100\text{-HZ }(\%))/\text{HZ }(\%)$$

In the formulas, FITC conc. means the molar concentration of a unit containing FITC group. The results obtained are shown in Table 3.

[Table 3]

| Table 3. FITC labeled HA-HZ-SUC for Pharmacokinetics Tests | | | | | |
|---|---|---|---|---|---|
| | | Residual HA-FITC HA-SUC Unmodified HA | | | |
| HZ (%) NMR | HZ (%) TNBS | Unit (Mol %) | Unit (Mol %) | Unit (Mol %) | HA (Wt. %) |
| 73 | - | 1.1 | 71.9 | 27.0 | 67.8 |
| -: Below limit of detaermination | | | | | |

(Example 3-3)

Evaluation for Blood residence

Plasma samples of HA administered rats

Fluorescently labeled HA modification product in Example 3-2 Comparative Example 3-1 were administered once to rats at a dose of 10 mg/kg intravenously (iv) and subcutaneously (sc), and blood samples were collected (heparin treatment) before the administration and at 0.833, 0.5, 2, 4, 8, 24, 48, 72, 96 and 168 hours after the administration. Plasma samples were obtained by centrifugation and kept frozen at -20°C or lower until measurement.

Measuring Method

The standard curve and the test samples were analyzed by GPC. Following is the condition.
GPC Column: TSKgel G6000$PW_{xL}$ (TOSOH Co. Ltd.)

Mobile phase: PBS (pH 7.4)
Elution mode: Isocratic
Flow rate: 0.5 mL/min
Injection volume: 40 μL
Detection: Fluorescence(EX: 494 nm, EM: 518 nm)

Preparation of Samples for Measurement

Samples for the standard curve;

Each fluorescently labeled HA modification product was diluted with PBS (pH 7.4) and the standard solutions of 1, 5, 10, 50, 100, 500 μg/mL and 0 μg/mL were prepared (control, PBS (pH 7.4)). Samples for the standard curve were prepared by adding an equal volume of normal rat plasma to these standard solutions.

· Preparation of samples for measurement;

Samples for measurement were prepared by adding an equal volume of (pH 7.4) to the plasma samples of HA modification product administered rats.

· Calculation for the concentration of HA modification product in the plasma;

Peak area was calculated using software for analyses Millenium Ver 3.21 (Waters Co. Ltd.). Concentration of HA modification product in the plasma was calculated using the standard curve obtained from the of the peak of each standard solution.

Pharmacokinetic Data

From the data of the change in the plasma concentrations of fluorescently labeled HA modification products in Example 3-2 and Comparative Example 3-1, pharmacokinetic parameters were calculated using WinNonlin Ver 4.0.1 (Pharsight Co. Ltd.). Using the 3 points data of the final measurement of individual animal, model independent analysis was carried out to calculate a half life (t1/2) and mean residence time in blood (MRT). Change in the plasma concentration

of fluorescently labeled HA modification product is shown in Figure 6, and calculated pharmacokinetic parameters are shown in Table 4.

[Table 4]

| Table 4. Pharmacokinetic Parameters of FITC Labeled HA Derivatives | | | | |
|---|---|---|---|---|
| Molecular Weight (kDa) | Rate of Incorporation (%: NMR) | Cl (mL/hr/kg) | MRT(h) | t1/2 |
| 23 (iv) | 87.0 | 2.44 | 86.0 | 47.3 |
| 100 (iv) | 92.5 | 0.76 | 91.6 | 49.3 |
| 200 (iv) | 93.5 | 0.87 | 91.3 | 44.7 |
| 23 (sc) | 87.0 | 5.40 | 83.9 | 50.8 |
| 100 (sc) | 92.5 | 2.18 | 101.4 | 56.5 |
| 200 (sc) | 93.5 | 3.76 | 98.7 | 57.6 |
| 580 (iv) | 73.0 (HZ) | 2.52 | 16.3 | 11.2 |

By the pharmacokinetic test using fluorescently labeled HA modification product, it was confirmed that the blood retention of the HA modification product of the present invention (Example 3-2), synthesized in an aprotic polar organic solvent, is greatly improved after a single intravenous administration to rats, when compared to those of HA and HA derivatives hitherto reported (HA-HZ-SUC; Patent reference 12, 13 and 14). The mean residence time (MRT) of a HA modification product of molecular weight 600 kDa (70% mol modification) after a single dose intravenous administration to rats has been reported to be about 16 hours, and it is clear that the blood residence is extended about 5.5 times. In 23 kDa HA modification product, it was observed that the plasma concentration had a tendency to decrease at the early stage, suggesting the possibility of some low molecular weight HA modification product being excreted from the kidney. As to 100 kDa and 200 kDa HA modification product, characteristics concerning blood residence (CL, MRT, t1/2) were almost the same value.

[Example 4]

Relationship between incorporation rate of amide group and blood residence in hyaluronic acid modification product (HA-AM-SUC)

To analyze the blood residence of hyaluronic acid modification product (HA-AM-SUC) from the of modification rate, HA modification products with various modification rate were synthesized from 200 kDa HA, and samples were prepared by incorporating a fluorescent dye, FITC, and the blood residence of each sample was observed.

(Example 4-1)

Preparation of HA-AM

Five DMSO solutions of HA (200 kDa)-TBA (4.0 mg/mL) were prepared. To each solution, EDOBEA was added at the equivalence ratio of HA unit/EDOBEA = 1/50 (mol/mol) and BOP reagent was added at the equivalence ratio of 0.25, 0.5, 0.75, 1.0 or 1.5 to HA unit, and the mixtures were reacted overnight. After adding 1 M Nacl solution in a half the volume of the reaction mixture, 5 N HCl was added to bring down pH to 3 and then the mixture was neutralized by adding 2 N NaOH. The mixture was dialyzed/purified (Spectra/por 4, cut off molecular weight (MWCO): 12 k - 14 kDa) against a large excess amount of distilled water (Milli Q water) and then ultrafiltered (YM-10, Millipore Co. Ltd.) and freeze dried. The incorporation rate of N-substituted amide group having an amino group at the terminus of the substituent was measured by the proton NMR method. NMR spectra are shown in Figure 7. (HA: methyl proton of N-acetyl group, 1.8-1.9 ppm, AM: methylene proton of EDOBEA part, 2.9-3.1 ppm) Each incorporation rate was BOP reagent rate 0.25: 20.0 mol%, 0.5: 36.5 mol%, 0.75: 54.5 mol%, 1.0: 69.0 mol%, 1.5: 90.5 mol%.

(Example 4-2)

Synthesis of fluorescently labeled HA modification product

HA-AMs obtained in Example 4-1 dissolved in distilled water (Milli Q water) to prepare 20.0 mg/mL solutions,

and 0.2 M carbonate buffer (pH 9.0) was added thereto to bring the concentration to 10.0 mg/mL. To this solutions was added fluorescein isothiocyanate in amounts of 0.07 mol per HA unit (to HA solutions with amide incorporation rate of 69% and 90.5%), 0.175 mol per HA unit (to HA A solutions with amide incorporation rate of 54.5%) and 0.28 mole per HA unit (to HA solutions with amide incorporation rate of 36.5%) and 0.63 mol per HA unit (to HA solutions with amide incorporation rate of 20.0%) dissolved in DMSO solution of 1/10 volume of HA-AM solution, and the mixture was stirred at room temperature for 1 hour. Then 40 mol per HA unit of succinic anhydride dissolved in DMSO solution of 1/10 volume of the HA-AM solution was added and the mixture was stirred further at room temperature for 30 minutes. After the 20% amine modified sample was dialyzed against a large excess amount of DMSO, and other samples were directly dialyzed/purified against (Spectra/por 4, cut off molecular weight (MWCO): 12 k - 14 kDa) a large excess amount of 25% aqueous EtOH solution, and then dialyzed/purified against 0.5 M Nacl aqueous solution. The dialysis was continued by changing the dialysate to distilled water (Milli Q water), and the solution thus obtained was freeze dried to obtain fluorescently labeled HA-AM-SUC.

Each of fluorescently labeled HA-AM-SUC obtained here dissolved in 50 mM carbonate buffer (pH 9.0) at a concentration of 0.25 mg/mL, and the concentration of FITC was measured from the absorbance at 494 nm of the solution, and the concentration of each unit was calculated according to the formulas below. Further, the conversion to mole fraction and the weight fraction of HA in the HA modification product were calculated.

Un-modified HA unit: x nmol/mL

HA-AM-SUC unit: y nmol/mL (a unit which is incorporated amino group and reacted with succinic anhydride)

[Formula 26]

Formula 1: $(401.3 \times x) + (631.58 \times y) + (544.97 \times (\text{Residual AM conc.})) + (898.9 \times (\text{FITC conc.}) = 250\ \mu g$

Formula 2: $x/(y + (\text{Residual AM conc.}) + (\text{FITC conc.})) = ((100\text{-AM}(\%)))/\text{AM}(\%)$

The results obtained are shown in Table 5.

[Table 5]

Table 5. FITC Labeled HA-AM-SUC for Pharmacokinetic Test

| BOP Ratio (BOP Mol/ HA Unit Mol) | AM (%) NMR | AM (%) TNBS | Unmodified Residual Unit (Mol%) | HA-AM-FITC Unit (Mol%) | HA-AM-SUC Unit (Mol%) | HA HA (Wt.%) |
|---|---|---|---|---|---|---|
| 0.25 | 20.0 | 1.6 | 0.3 | 18.1 | 80.0 | 87.2 |
| 0.50 | 36.5 | 2.4 | 0.9 | 33.2 | 63.5 | 81.7 |
| 0.75 | 54.5 | 2.4 | 1.6 | 50.5 | 45.5 | 75.6 |
| 1.00 | 69.0 | - | 0.6 | 68.4 | 31.0 | 68.2 |
| 1.50 | 90.5 | - | 0.9 | 89.6 | 9.5 | 62.8 |

-: Below limit of determination

(Example 4-3)

Evaluation for blood residence

<u>Plasma sample of HA administered rats</u>

Fluorescently labeled HA modification product in Example 4-2 was administered once to rats at a dose of 10 mg/kg intravenously (iv) an blood samples were collected (heparin treatment) before the administration and at 0.5, 2,

4, 8, 24, 48, 72, 96 and 168 hours after the administration. Plasma samples were obtained by centrifugation and kept frozen at -20°C or lower until measurement.

Measuring method

Samples for the standard curve and for measurement were distributed in a 96 well plate and analyzed using a microplate reader. Conditions are as follows.
Microplate reader: SPECTRA MAX GEMINI (Molecular Devices Co. Ltd.)

Sample volume: 100 μL/well
Detection: Fluorescence(EX: 485 nm, EM: 538 nm)

Preparation of samples for measurement

· Samples for the standard curve;

Each fluorescently labeled HA modification product was diluted with PBS (pH 7.4) and the standard solutions of 1, 5, 10, 50, 100, 500 μg/mL and 0 μg/mL (control, PBS (pH 7.4)) were prepared. Samples for the standard curve were prepared by adding an equal volume of normal rat plasma to these standard solutions.

· Preparation of samples for measurement;

Samples for measurement were prepared by adding an equal volume of PBS (pH 7.4) to the plasma samples of HA modification product administered rats.

· Calculation for the concentration of HA modification product in plasma;

Concentration of HA modification product in the plasma was calculated from the standard curve obtained from the fluorescent intensity of the each standard solution using an analytical software, SOFTmax PRO (Molecular Devices Co. Ltd.).

Pharmacokinetic data

From the data of the change in the plasma concentrations of fluorescently labeled HA modification products in Example 4-2, pharmacokinetic parameters were calculated using WinNonlin Ver 4.0.1 (Pharsight Co. Ltd.). Model independent analysis was carried out on the change in the plasma concentration of each rat to obtain mean residence time in blood (MRT). Half life (tl/2) was calculated using 3 points data of the final measurement of individual animal. Change in the plasma concentration of fluorescently labeled HA modification product is shown in Figure 8, and relationship between the incorporation rate of AM and MRT is shown in Figure 9. Also, calculated pharmacokinetic parameters are shown in Table 6.
[Table 6]

Table 6. Pharmacokinetic Parameters of N-substituted amidated HA derivatives

| Incorporation Rate (%: NMR) | Cl (mL/hr/kg) | MRT (h) | t1/2 |
|---|---|---|---|
| 20.0 | 16.37 | 2.81 | 1.95 |
| 36.5 | 12.96 | 2.90 | 1.62 |
| 54.5 | 10.47 | 4.26 | 2.76 |
| 69.0 | 1.20 | 49.6 | 36.7 |
| 90.5 | 0.97 | 60.6 | 44.5 |

The relationship between the incorporation rate of amide group and MRT shown in Figure 9 suggests that when the modification rate was about 55% or more, a drastic increase of MRT, that is, increase of blood residence occurred In particular, when the incorporation rate is 55 mol % or above, preferably 65 mol % or above, more preferably 69 mol % or above, there is a good possibility that a HA modification product having preferable characteristic in blood residence

time, that is MRT of 18 hours or more may be obtained.

Since HA derivatives that acquired enzyme resistance have much reduced binding ability to the HA receptors such as CD44, the long term blood residence seems to be acquired.

[Example 5]

Preparation and evaluation of water soluble modified HA-GLP-1 analogue 1 conjugate

(Example 5-1)

Preparation of HA-EDOBEA-$(CH_2)_{10}$-MI/SUC

To aqueous solution (10 mg/mL, 17.66 mL) of HA-EDOBEA (200 kDa HA-TBA, Incorporation rate of EDOBEA: 95.5%), which was obtained by the similar method as in Example 3-1 except that reagent was 2.5 equivalent per HA unit, 0.2 M phosphate buffer (pH 7.0, 22.075 mL) was added. DMSO solution (0.573 mg/mL, 4.415 mL) of N-[κ-maleimidoundecanoyloxy] sulfosuccinimide ester (hereinafter also called "Sulfo-KMUS") (Piece Co. Ltd.) was added and the mixture was shaken at room temperature for 30 minutes, DMSO solution of succinic anhydride (283.95 mg/mL, 4.415 mL) was added and the mixture was further shaken at room temperature for 1.5 hours. The mixture was dialyzed/purified (Spectra/por 4, MWCO: 12 k - 14 kDa) against a large excess amount of distilled water (Milli Q water) at 4°C and freeze dried to obtain hyaluronic acid modification product to which maleimide group and succinic acid was incorporated (hereinafter also called "HA-EDOBEA-MI/SUC", 177.80 mg).

(Example 5-2)

Preparation of hyaluronic acid-GLP-1 analogue 1 conjugate solution

An analogue of native GLP-1 (Human, 7-37; JP Patent Publication (Kohyo) No. 7-504679) in which the second (position 8) alanine was converted to glycine and the 31st (position 37) glycine to cysteine (hereinafter also called "GLP-1 analogue 1") was obtained by a solid phase peptide synthesis method (Peptide Institute Inc.). To 0.2 M phosphate buffer (pH 7.0) solution of GLP-1 analogue 1 (2.0 mg/mL), 1/20 volume of aqueous solution (20 mM) of tris (2-carboxyethyl) phosphine hydrochloride (hereinafter also called "TCEP") (Piece Co. Ltd.) was added. This GLP-1 analogue 1 solution (1.3263 mL) containing TCEP was added to HA-EDOBEA-MI/SUC aqueous solution (20 mg/mL, 1.2 mL) obtained in Example 5-1 and the mixture was left standing at 37°C for 2 hours. The same GLP-1 analogue 1 solution (1.3263 mL) containing TCEP was added again and the mixture was similarly left standing. 0.1 M phosphate buffer (pH 7.0) solution of cysteine hydrochloridemonohydrate (3.6 mg/ml, 0.3853 mL) was added and the mixture was left standing at 37°C for 1 hour. The reaction mixture was divided into 3 parts and subjected to GPC at the following condition to collect the conjugate fraction. The collected fractions were concentrated by a centrifugal ultrafiltration (Vivaspin 20, MWCO: 50000, Funakoshi Co. Ltd.) to about 4.85 mL to obtain the HA-GLP-1 analogue 1 conjugate solution of the title. A sample, which was obtained by the similar operation to the present Example except that GLP-1 analogue 1 was not used, was used in calibration as a control, and the concentrations of GLP-1 analogue 1 and HA-EDOBEA-MI/SUC, and the incorporation rate of GLP-1 analogue 1 were calculated by the 280 nm absorbance of thus obtained HA-GLP-1 analogue 1 solution and the carbazole sulfuric acid method. The concentrations of GLP-1 analogue 1, HA-EDOBEA-MI/SUC and the incorporation rate of GLP-1 analogue 1 were 42.6 nmol/mL, 3.54 and 3.7/HA (mol/mol), respectively.

<u>GPC condition</u>

System: FPLC (Amersham Bioscience Co. Ltd.)
GPC Column: HiLoad 16/60 Superdex 200prep grede (Amersham Bioscience Co. Ltd.)
Mobile phase: PBS (pH 7.4)
Flow rate: 1.0 mL/min
Detection: UV (280 nm)

Assay Method for HA modification product by the carbazole sulfuric acid method

[Reagents]

Sulfuric acid solution: sulfuric acid solution of $Na_2B_4O_7 \cdot 10H_2O$ (25 mM)
Carbazole solution: ethanol solution of carbazole (1.25 mg/mL: 0.125%)

[Standard Substance]

Hyaluronic acid modification product (HA-EDOBEA-SUC), in which succinic anhydride was incorporated only to HA-EDOBEA, was prepared by the similar method as in Example 5-1 except that DMSO solution of N-[κ-maleimidoundecanoyloxy] sulfosuccinimide ester was not added and dissolved in PBS at 0.5 mg/mL. A series of two fold dilution of this 0.5 mg/mL HA-EDOBEA-SUC solution was prepared (5 concentration points: 0.03125-0.5 mg/mL).

[Quantitative Determination]

To ice cold sulfuric acid solution (1 mL), PBS (Control), the standard substance (each 0.2 mL) and a solution of HA-GLP-1 analogue 1 conjugate which was obtained in Example 5-2 and diluted 19.8 fold with PBS (0.2 mL) were added and mixed. The mixtures were heated in a hot water bath for about 25 minutes and then cooled in running water to room temperature. Carbazole solution (40 μL) was added to each mixture and mixed. The mixtures were heated again in a hot water bath for 30 minutes and cooled to room temperature in running water. Absorbance at 530 nm was measured and the concentrations of HA Modification Product in the sample solutions were assayed from the standard curve prepared from the absorbance of the control and the standard substance.

(Example 5-3)

Evaluation for blood residence of HA-GLP-1 analogue 1 conjugate

Plasma sample of HA-GLP-1 analogue 1 conjugate administered rats

The A-GLP-1 analogue 1 conjugate solution obtained in Example5-2 was diluted with PBS containing 0.05% Tween 80 (pH 7.4, hereinafter called "solvent Z") and administered once to rats at a dose of 50 μg/kg intravenously (iv) and blood samples were collected (heparin treatment) at 1, 4, 8, 24, 48, 72, 96 and 168 hours after the administration. Plasma samples were obtained by centrifugation and kept frozen at -20°C or lower until measurement.

Measuring Method

Samples for the standard curve and for measurement were distributed in a 96 well plate of GLP-1 (Active) ELISA KIT (Linco Research, Inc.; hereinafter also called "Kit W") and analyzed using a microplate reader. Conditions are as follows.

Microplate reader: SPECTRA MAX GEMINI (Molecular Devices Co. Ltd.)

Detection: Fluorescence (EX: 355 nm, EM: 460 nm)

Preparation of Samples for Measurement

· Samples for the standard curve;

HA-GLP-1 analogue 1 conjugate obtained in Example 5-2 was diluted with solvent Z and Assay Buffer included in Kit W and the standard solutions of 12.8, 6.4, 3.2, 1.6, 0.8, 0.4, 0.2 0.1 μg/mL and 0 μg/mL were prepared. Samples for the standard curve were prepared by adding 5 μL of normal rat plasma to standard solutions.

· Preparation of samples for measurement;

Samples for measurement were prepared by adding Assay Buffer included in Kit W to the plasma samples of HA-GLP-1 analogue 1 conjugate administered rats.

· Calculation for the concentration of HA modification product in the plasma;

Concentration of HA-GLP-1 analogue 1 conjugate in the plasma was calculated from the standard curve obtained from the fluorescent intensity of the each standard solution using an analytical software, SOFTmax Pro (Molecular Devices Co. Ltd.).

Pharmacokinetic Data

From the data of the change in the plasma concentrations of administered HA-GLP-1 analogue 1 conjugate, pharmacokinetic parameters were calculated using WinNonlin Ver 4.0.1 (Pharsight Co. Ltd.). Model independent analysis was carried out on the change in the plasma concentration of each rat to obtain mean residence time in blood (MRT). Half life (tl/2) was calculated using 3 points data of the final measurement of individual animal. Change in the plasma concentration of HA-GLP-1 analogue 1 conjugate is shown in Figure 10. Also, calculated pharmacokinetic parameters are shown in Table 7.

[Table 7]

Table 7. Pharmacokinetic parameters after HA-GLP-1 analogue 1 conjugate was intravenously administered to rats

| | Mean Value | Standard Deviation | |
|---|---|---|---|
| Half Life | 23.6 | 3.1 | (hr) |
| Total Clearance | 1.8 | 0.1 | (mL/hr/kg) |
| Mean Residence Time | 30.2 | 4.3 | (hr) |
| Distribution Volume | 53.4 | 4.9 | (mL/kg) |

It has been reported that the half life of native GLP-1 and a variant in which the second alanine of the native GLP-1 was substituted to glycine is 1-5 minutes after administered intravenously to human and pig (Current Medicinal Chemistry Vol. 10; 2471-2483, 2003 and Diabetologia, Vol. 41: 271-278, 1998). From Figure 10 and Table 7, the half life of HA-GLP-1 analogue 1 conjugate after administered intravenously was 23.6 hours and MRT was 30.2 hours, suggesting a great increase in blood residence compared to native GLP-1 and the like.

(Example 5-4)

Oral glucose tolerance test

After 8 weeks old male BKS. Cg -+ $Lepr^{db}$/+ $Lepr^{db}$/Jcl mice (Japan CLEA Co. Ltd.) were fasted overnight, the HA-GLP-1 analogue 1 conjugate solution, which was obtained in Example 5-2 and diluted with solvent Z (at doses as peptide, 1.5 μg/kg, 15 μg/kg or 150 μg/kg), or solvent Z was intravenously administered to the mice. After 1 minute of the intravenous administration, 50% glucose solution (Otsuka Pharamaceutical Co. Ltd.) was orally administered at a dose of 3 g glucose/kg. Similarly, GLP-1 (Human, 7-37; Peptide Institute, Inc.) diluted with solvent Z (150 μg/kg or 1500 μg/kg) or solvent Z was intravenously administered. After 1 minute of the intravenous administration, 50% glucose solution was orally administered at a dose of 3 g glucose/kg. Tests were carried out on groups consisting of 6 mice group.

Before the glucose administration (0 hour), 0.5, 1, 2 and 4 hours after the administration, blood was collected from the tail, and the blood glucose level was measured by an enzymatic assay (hexokinase method; Reagent, Autosera S GLU (Daiichi Pure Chemicals Co. Ltd.); Instrument, BioMajesty JCA-BM1250 (JEOL Co. Ltd.)).
The blood glucose lowering rate was calculated according to the following formula.

[Formula 27]

$$\text{Blood glucose lowering rate (\%)} = \frac{\text{Mean AUC for blood glucose increase level in solvent administered group} - \text{AUC for blood glucose increase of individual animal}}{\text{Mean AUC for blood glucose increase level in solvent administered group}} \times 100$$

"AUC for blood glucose increase level" represents the area of the increase portion in a graph of the blood glucose level changes after glucose administration plotted with respect to time, up to 4 hours after glucose administration, with the glucose level prior to glucose administration as the baseline. Specifically, the AUC for blood glucose increase level can be obtained by the following formula, where $A^1$ = blood glucose level before the glucose administration, $B^1$ = blood glucose level 30 minutes after the glucose administration, $C^1$ = blood glucose level 1 hour after the glucose administration, $D^1$ = blood glucose level 2 hours after the glucose administration and $E^1$ = blood glucose level 4 hours after the glucose administration:

[Formula 28]

$$AUC = 0.5 \times \frac{A^1 + B^1}{2} + 0.5 \times \frac{B^1 + C^1}{2} + 1 \times \frac{C^1 + D^1}{2} + 2 \times \frac{D^1 + E^1}{2} - 4 \times A^1$$

From the calculated blood glucose lowering rate, means and standard errors were calculated in each group and shown in Table 8 and Table 9. In HA-GLP-1 analogue 1 conjugate, the blood glucose lowering effect was greatly increased compared with GLP-1 (Human, 7-37), and the utility as a therapeutic drug for diabetes was indicated.

[Table 8]

Table 8. Blood glucose lowering c rate (%) of GLP-1 (Human, 7-37) administration

| Amount Administered | Mean | Standard Erroe |
|---|---|---|
| 150 μg/kg | 15 | 15 |
| 1500 μg/kg | 60 | 17 |

[Table 9]

Table 9. Blood glucose lowering rate (%) of HA-GLP-1 analogue 1 conjugate administration

| Amount Administered | Mean | Standard Error |
|---|---|---|
| 1.5 μg/kg | 3 | 11 |
| 15 μg/kg | 60 | 21 |
| 150 μg/kg | 120 | 15 |

[Example 6]

Preparation and evaluation of water soluble HA modification product-GLP-1 analogue 2

(Example 6-1)

Preparation of HA-EDOBEA-$(EO)_4$-MI/SUC

To each of three preparations of aqueous solution (10 mg/mL, 4.0 mL) of HA-EDOBEA (100 kDa HA-TBA, Incorporation rate of EDOBEA: 97.0%), which was obtained as described in Example 3-1 except that 2.5 equivalent BOP reagent per HA unit was used, 0.2 M phosphate buffer (pH 7.0, 5.0 mL) and 0.1 M phosphate (pH 7.0, 30 mL) was added. To these mixtures, DMSO solution (2.283 mg/mL, 1.0 mL) of N-hydroxysuccinimidyl 15-(3-maleimidepropionyl)-amide-4, 7, 10, 13-tetraoxapentadecanoate (hereinafter also called "NHS-$(EO)_4$-MI") (Quanta BioDesign Co. Ltd.) was added and the mixture was shaken at room temperature for 30 minutes. DMSO solution of succinic anhydride (287 mg/mL, 1.0 mL) was added to the mixture and shaken at room temperature for another 1.5 hours. The mixture was dialyzed/purified (Spectra/por 4, MWCO: 12 k - 14 kDa) against a large excess amount of distilled water (Milli Q water) at 4°C and freeze dried to obtain hyaluronic acid modification product to which maleimide group and succinic acid was incorporated (hereinafter also called HA-EDOBEA-$(EO)_4$-MI/SUC", 46.93, 48.01, 22.57 mg).

Evaluation of the amount of maleimide incorporation (hereinafter also called "MI/HA") per HA 1 molecule indicated 7.0, 7.8 and 7.4 (mol/mol).

<u>MI/HA quantitative determination method in HA-EDOBEA-$(EO)_4$-MI/SUC</u>

[Molecular Weight Calculation]

Succinic acid incorporation rate of HA-EDOBEA-$(EO)_4$-MI/SUC was assayed by the proton method (HA: methyl proton of N-acetyl group, 1.8-1.9 ppm, succinic acid: ethylene proton, 2.4-2.55 ppm). Since the incorporation rate of $(EO)_4$-MI was low, it was not taken into account, and the mean molecular weight of HA-EDOBEA-$(EO)_4$-MI/SUC and the mean molecular weight per unit were calculated.

[Quantitative Determination of MI]

To aqueous solution of HA-EDOBEA-$(EO)_4$-MI/SUC (20.0 mg/mL, 55 μL), 0.2 M phosphate buffer containing 20 mM ethylenediamine tetraacetic acid disodium salt dihydrate (pH 7.0, 0.55 μL) containing 2 M cysteine or the same buffer without cysteine was added. After left standing the mixtures at 37°C for 30 minutes, residual cysteine was assayed by Ellman's reagent. As controls, similar treatments were carried out for distilled water (55 μL) mixed with 0.2 M phosphate buffer containing 20 mM ethylenediamine tetraacetic acid disodium salt dihydrate (pH 7.0, 0.55 μL) containing 2M cysteine, and for hyaluroic acid modification product (HA-EDOBEA-SUC) in which only succinic acid was incorporated to HA-EDOBEA that was obtained as in Example 6-1 except DMSO was added in place of DMSO solution of NHS-$(EO)_4$-MI. Non-specific consumption was calibrated by the control, and the quantity of cysteine consumption was calculated as maleimide quantity from the ratio to HA concentration, MI/HA(mol/mol).

[Quantitative Determination of Cysteine Using Ellman's Reagent]

As a reaction solvent, 0.1 M phosphate buffer (pH 8.0) containing 1 mM ethylenediamine tetraacetic acid disodium salt dihydrate was prepared. Cysteine was dissolved in the reaction solvent to prepare the standard sample solution of 1.2, 0.6, 0.3, 0.15 and 0.075 mM. Ellman's reagent was dissolved in DMSO (40 mg/mL) and diluted 10 fold with the reaction solvent to prepare Ellman's reagent solution. The reaction solvent (1.0 mL) was mixed with Ellman's reagent solution (20 μL) and to this mixture, the reaction buffer as a blank, the standard sample solution or test sample solution (each 100 μL) were added and mixed, and then left standing in the dark at room temperature for 15 minutes. Absorbance at 412 nm was measured, the standard curve was prepared from the absorbance of the blank and the standard samples, and the quantity of residual cysteine in the test samples was assayed. The absorbance of the hyaluronic acid derivative at 412 nm was calibrated by the measurement of the test sample without adding cysteine.

(Example 6-2)

Preparation of HA-GLP-1 analogue 2 conjugate solution

A variant of native GLP-1 (Human, 7-37; JP Patent Publication (Kohyo) No. 7-504679) in which the second (position 8) alanine was converted to glycine and a proline-proline-proline-cysteine was added to the C-terminal side of the 31st (position 37) glycine and the C-terminal was amidated (hereinafter also called "GLP-1 analogue 2") was obtained by a solid phase peptide synthesis method (Peptide Institute Inc.). HA-EDOBEA-$(EO)_4$-MI/SUC obtained in Example 6-1 (MI/HA = 7.0, 7.8, 7.4 (mol/mol)) aqueous solutions (20 mg/mL, 2.0, 2.0, 0.83 mL) were mixed together to prepare HA-EDOBEA-$(EO)_4$-MI/SUC (mean MI/HA = 7.4 (mol/mol)) aqueous solution (20 mg/mL). This HA-EDOBEA-$(EO)_4$-MI/SUC aqueous solution (20 mg/mL, 4.02 mL) was added to GLP-1 analogue 2 solution (2.0 mg/mL, 7.052 mL) in 0.2 M phosphate buffer (pH 7.0) the mixture was left standing at 37°C for 1 hour. Cysteine hydrochloride monohydrate solution (11.98 mg/mL, 1.107 mL) in 0.1 M phosphate buffer (pH 7.0) was added and left standing at 37°C for another 30 minutes. The reaction mixture was divided into three parts and subjected to GPC in the following condition to obtain the conjugate fraction. Thus obtained conjugate fraction was filtered (MILLEX-GV, $\phi$ = 0.22 $\mu$m, Millipore Co. Ltd.) and then concentrated by centrifugal ultrafiltration (Centricon Plus-20, nominal molecular weight limit: 30,000, Millipore Co. Ltd) until the volume became about 1/20. The concentrated solution was diluted about 20 fold with PBS, and again was concentrated and diluted by a similar procedure. The solution was again concentrated and filtered (MILLEX-GV, $\phi$ = 0.22 $\mu$m, Millipore Co. Ltd.) after adjusting the volume to 16 mL to obtain the HA-GLP-1 analogue 2 conjugate solution described in the title of this Example. Figure 11 shows the GPC chromatogram. In the sample containing GLP-1 analogue 2, a strong absorbance at 280 nm, which is believed to be derived from peptide, observed in the high molecular weight fraction, suggesting that GLP-1 analogue 2 was bound to hyaluronic acid modification product. A sample obtained by the similar procedure to the present without using GLP-1 analogue was for calibration as a control, and the concentrations of GLP-1 analogue 2 and HA-EDOBEA-$(EO)_4$-MI/SUC, and the incorporation rate of GLP-1 analogue 2 calculated by the 280 nm absorbance of thus obtained HA-GLP-1 analogue 2 solution and the sulfuric acid method. The concentrations of GLP-1 analogue 2, HA-EDOBEA-$(EO)_4$-M1/SUC and the incorporation rate of GLP-1 analogue 2 were 161.8 nmol/mL, 3.22 mg/mL and 7.9/HA (mol/mol), respectively.

GPC Condition

System: FPLC or AKTAexplorer (Amersham Bioscience Co. Ltd.)
GPC Column: HiLoad 26/60 Superdex 200prep grede (Amersham Bioscience Co. Ltd.)
Mobile phase: 30% acetonitrile, 0.1% trifluoroacetic acid solution
Flow rate: 2.5 mL/min
Detection: UV (280 nm)

Method Quantitative Determination for HA modification product by the carbazole sulfuric acid method

[Reagents]

Sulfuric acid solution: sulfuric acid solution of $Na_2B_4O_7 \cdot 10\ H_2O$ (25 mM)
Carbazole solution: ethanol solution of carbazole (1.25 mg/mL: 0.125%)

[Standard Substance]

Hyaluronic acid modification product (HA-EDOBEA-SUC), in which only succinic acid was incorporated to HA-EDOBEA, was prepared by the similar method as in Example 6-1 except that DMSO was added in place of NHS-$(EO)_4$-MI solution and dissolved in PBS at 0.5 mg/mL. A series of two fold dilution of this 0.5 mg/mL HA-EDOBEA-SUC solution was prepared (5 concentration points: 0.03125-0.5 mg/mL).

[Quantitative Determination]

To ice cold sulfuric acid solution (1 mL), PBS (Control), the standard substance (each 0.2 mL) and a solution of HA-GLP-1 analogue 2 conjugate which was obtained in Example 6-2 (15 $\mu$L) and diluted 20 fold with PBS (0.2 mL) were added and mixed. The mixtures were heated in a hot water bath for about 25 minutes and then cooled in running water to room temperature. Carbazole solution (40 $\mu$L) was added and the mixture and mixed. The mixtures were heated

again in a hot water bath for 30 minutes and cooled to room temperature in running water. Absorbance at 530 nm was measured and the concentrations of HA modification product in the sample solutions were assayed from the standard curve prepared from the absorbance of the control and the standard substance.

Methods for determining the concentration of GLP-1 analogue 2 in HA-GLP-1 analogue 2 conjugate and the incorporation rate of GLP-1 analogue 2

A sample obtained according to the procedure of the present Example without using GLP-1 analogue and obtained HA-GLP-1 analogue 2 solution (50 μL) were serially diluted 2 fold with PBS and absorbance at 280 nm was measured. The proportional constant was calculated from concentration of HA modification product in the sample, which was obtained by the similar procedure to the present example without using GLP-1 analogue assayed by the carbazole-sulfuric acid method, and absorbance at 280 nm, and contribution of HA in the 280 nm absorbance of the conjugate solution was calculated from the concentration of HA modification product in HA-GLP-1 analogue 2 conjugate solution. The concentration of GLP-1 analogue 2 was calculated from the molar absorbance coefficient assuming the differential the absorbance of GLP-1 analogue 2. The incorporation rate of GLP-1 analogue 2 (mol/mol) was calculated from the ratio of the concentrations of GLP-1 analogue 2 and HA modification product.

(Example 6-3)

Evaluation for CAMP production capability of HA-GLP-1 analogue 2 conjugate

Based on the disclosed DNA sequence information of human GLP-1 receptor (Graziano et al., Biochem. Biophys. Res. Commun. 1993. 196: 141-146 (1993)), an expression vector was constructed. Recombinant HEK293 cells which express human GLP-1 receptor were obtained by transforming human fetal embryonic kidney derived cells with this expression vector.

The human GLP-1 receptor-expressing cells were cultured in a 96 well plate for 3 days and used for the assay. Cell culture media was replaced with reaction fluid (DMEM containing 0.5 mM 3-isobutyl-1-methylxanthine and 3.7 g/L $NaHCO_3$). Subsequently and GLP-1 or HA-GLP-1 analogue 2 conjugate, which were obtained in Example 6-2 and were diluted with DMEM containing 0.05% or Tween 80, were added. Cells were incubated for 30 minutes at 37°C in a CO2 incubator. The reaction was terminated by addition of cell lysis buffer.

Cyclic AMP generated in the cells by the reaction between HA-GLP-1 analogue 2 conjugate and GLP-1 receptor was measured by the chemiluminescence ELISA (Enzyme-linked immunosorbent assay) method using cAMP-Screen™ (Applied Biosystems Co. Ltd.) and ARVO HTS 1420 Multilabel Counter (Perkin Elmer (Wallac) Co. Ltd.). Cyclic AMP in the cell lysate was calculated from the standard curve obtained from the luminescence intensity of the standard solution of cyclic AMP using an analytical software, Graphpad Prism Version 4.02 (GraphPad Software Inc.), and converted to cyclic AMP amount in cell. EC 50 value of HA-GLP-1 analogue 2 conjugate in the cyclic AMP production was calculated from the concentration-response curve using Graphpad Prism Version 4.02. In particular, the EC50 value of the conjugate was calculated based on the concentration of GLP-1 analogue 2 in HA-GLP-1 analogue 2 conjugate. In Table 10, the cyclic AMP production capability of the HA-GLP-1 analogue 2 conjugate is expressed as a relative value, when the EC50 value of native GLP-1 (Human, 7-37) is assumed to be 1.

[Table 10]

Table 10. CAMP production capability of HA-GLP-1 analogue 2 conjugate

| Test Sample | EC50Value (Relative Value) |
|---|---|
| GLP-1 | 1 |
| HA-GLP-1 Analogue 2 Conjugate | 112 |

It was confirmed that HA-GLP-1 analogue 2 conjugate still kept the cAMP production capability as GLP-1, although its EC50 value was higher than that of native GLP-1 probably of the steric hindrance by the macromolecule, HA.

(Example 6-4)

Evaluation for blood residence of HA-GLP-1 analogue 2 conjugate

Plasma sample of HA-GLP-1 analogue 2 conjugate administered rats

The HA-GLP-1 analogue 2 conjugate solution obtained in Example 6-2 was diluted with PBS containing 0.05% Tween 80 (pH 7.4, hereinafter called "solvent Z") and administered once to rats at a dose of 50 μg/kg intravenously and blood samples were collected (heparin treatment) at 1, 4, 8, 24, 48, 72, 96, 120 and 168 hours after the administration. Plasma samples were obtained by centrifugation and kept frozen at -20°C or lower until measurement.

Measuring Method

Samples for the standard curve and for measurement were distributed in a 96 well plate of GLP-1 (Active) ELISA KIT (Linco Research, Inc.; hereinafter also called "Kit W") and analyzed using a microplate reader. Conditions are as follows.

Microplate reader: SPECTRA MAX GEMINI (Molecular Devices Ltd.)

Detection: Fluorescence (EX: 355 nm, EM: 460 nm)

Preparation of Samples for Measurement

· for the standard curve;

HA-GLP-1 analogue 2 conjugate solution obtained in Example 6-2 was diluted with solvent Z and Assay Buffer included in Kit W and the standard solutions of 3.2, 1.6, 0.8, 0.4, 0.2, 0.1, 0.05 μg/mL and 0 μg/mL were prepared. Samples for the standard curve were prepared by adding 5 μL of normal rat plasma to these standard solutions.

· Preparation of samples for measurement;

Samples for measurement were prepared by adding Assay Buffer included in Kit W to the plasma samples of HA-GLP-1 analogue 2 conjugate administered rats.

Calculation for the concentration of HA modification product in the plasma;

Concentration of HA-GLP-1 analogue 2 conjugate in the plasma was calculated from the standard curve obtained from the fluorescent intensity of the each standard solution using an analytical software, SOFTmax Pro (Molecular Devices Co. Ltd.).

Pharmacokinetic Data

From the data of the change in the plasma concentrations of administered HA-GLP-1 analogue 2 conjugate, pharmacokinetic parameters were calculated using WinNonlin Ver 4.0.1 (Pharsight Co. Ltd.). Model independent analysis was carried out on the change in the plasma concentration of each animal to obtain mean residence time in blood (MRT). Half life (t1/2) was calculated using 3 points data of the final measurement of individual animal. Change in the plasma concentrations of HA-GLP-1 analogue 2 conjugate is shown in Figure 12. Also, calculated pharmacokinetic parameters are shown in Table 11.

[Table 11]

Table 11. Pharmacokinetic parameters after HA-GLP-1 analogue 2 conjugate was intravenously administered to rats

| | Mean Value | Standard Deviation | |
|---|---|---|---|
| Half Life | 21.3 | 7.1 | (hr) |
| Total Clearance | 1.4 | 0.1 | (mL/hr/kg) |
| Mean Residence Time | 32.6 | 3.3 | (hr) |

(continued)

Table 11. Pharmacokinetic parameters after HA-GLP-1 analogue 2 conjugate was intravenously administered to rats

| | Mean Value | Standard Deviation | |
|---|---|---|---|
| Distribution Volume | 44.4 | 4.4 | (mL/kg) |

It has been reported that the half life of native GLP-1 and a variant in which alanine (at 8 position) of the native GLP-1 was substituted to glycine is 1-5 minutes after administered intravenously to human and pig (Current Medicinal Chemistry Vol. 10; 2471-2483, 2003 and Diabetologia, Vol. 41: 271-278, 1998). From Figure 12 and Table 11, the half life of HA-GLP-1 analogue 2 conjugate after administered intravenously to rats is 21.3 hours and MRT is 32.6 hours, suggesting a great increase in blood residence compared to native GLP-1 and the like.

(Example 6-5)

Oral glucose tolerance test

After 8 weeks old male BKS. Cg -+ $Lepr^{db}$/+ $Lepr^{db}$/Jcl mice (Japan CLEA Co. Ltd.) were fasted overnight to 24 hours, the HA-GLP-1 analogue 2 conjugate solution, which was obtained in Example 6-2 and diluted with solvent Z (at a dose as peptide, 300 μg/kg), or solvent Z was subcutaneously administered to the mice. After 6, 24 or 48 hours of the subcutaneous administration, 50% glucose solution (Otsuka Pharamaceutical Co. Ltd.) was orally administered at a dose of 3 g glucose/kg. Similarly, a variant of native GLP-1 (human, 7-37; JP Patent Publication (Kohyo) No. 7-504679) in which the second (position 8) alanine was converted to glycine (hereinafter also called "GLP-1 analogue 0") (Peptide Institute Inc.) diluted with solvent Z (3000 μg/kg) or solvent Z was subcutaneously administered. After 2 minutes or 4 hours and 2 minutes of the subcutaneous administration (in the case of solvent Z, after 1 or 5 hours) 50% glucose solution was orally administered at a dose of 3 g glucose/kg. Tests were carried out on groups consisting of 6 mice per group.

Before the glucose administration (0 hour), 0.5, 1 and 2 hours after the administration, blood was collected from the tail, and the blood glucose level was measured by an enzymatic assay (hexokinase method; Reagent, Autosera S GLU (Daiichi Pure Chemicals Co. Ltd.); Instrument, BioMajesty JCA-BM1250 (JEOL Co. Ltd.)).

The blood glucose lowering rate was calculated according to the formula described in Example 5-4.

Here, "AUC for blood glucose increase level" represents the area of increase portion in a graph of the blood glucose level changes after glucose administration plotted with respect to time, up to 2 hours after glucose administration, with the glucose level prior to glucose administration as the baseline. Specifically, the AUC for blood glucose increase level can be obtained by the following formula where $A^1$ = blood glucose level before the glucose administration, $B^1$ = blood glucose level 30 minutes after the glucose administration, $C^1$ = blood glucose level 1 hour after the glucose administration and $D^1$ = blood glucose level 2 hours after the glucose administration:

[Formula 29]

$$AUC = 0.5 \times \frac{A^1 + B^1}{2} + 0.5 \times \frac{B^1 + C^1}{2} + 1 \times \frac{C^1 + D^1}{2} - 2 \times A^1$$

From the calculated blood glucose lowering rate, means and standard errors are calculated in each group and shown in Table 12 and Table 13. In HA-GLP-1 analogue 2 conjugate, the blood glucose lowering effect lasted significantly longer comparing with GLP-1 analogue 0, and the utility as a therapeutic drug for diabetes was indicated.

[Table 12]

Table 12. Blood glucose lowering rate (%) of GLP-1 analogue 0 administration

| Time After Administration | Mean Value | Standard Error |
|---|---|---|
| 2 Minutes | 102 | 3 |
| 4 Hour 2 Minutes | 18 | 10 |

[Table 13]

Table 13. Blood glucose lowering rate (%) of HA-GLP-1 analogue 2 administration

| Time After Administration | Mean Value | Standard Error |
|---|---|---|
| 6 Hours | 53 | 6 |
| 24 Hours | 23 | 8 |
| 48 Hours | 18 | 5 |

[Example 7]

Preparation and evaluation of water soluble HA modification product-GLP-1 analogue 1 and GLP-1 analogue 2 conjugates

Water soluble HA modification product-GLP-1 analogues having different HA molecular weight, different linker structure between water soluble HA modification product and maleimide and different GLP-1 analogue were prepared.

(Example 7-1)

Preparation of HA-EDOBEA-$(CH_2)_{10}$-MI/SUC

To aqueous solution (10 mg/mL, 9.0 mL) of HA-EDOBEA (200 kDa HA-TBA, Incorporation rate of EDOBEA: 98.5%), which was obtained as described in Example 3-1 except that 2.5 equivalent BOP reagent per HA unit used, 0.2 M phosphate buffer (pH 7.0, 11.25 mL) was added. To these mixtures, DMSO solution (0.567 mg/mL, 2.25 mL) of N-[κ-maleimidoundecanoyloxy] sulfosuccinimide ester (hereinafter also called "Sulfo-KMUS") (Piece Co. Ltd.) was added and the mixture was shaken at room temperature for 30 minutes. DMSO solution of succinic anhydride (290 mg/mL, 2.25 mL) was added and the mixture was further shaken at room temperature for 1.5 hours. The mixture was dialyzed/purified (Spectra/per 4, MWCO: 12 k - 14 kDa) against a large excess amount of distilled water (Milli Q water) at 4°C and freeze dried to obtain HA-EDOBEA-$(CH_2)_{10}$-MI/SUC (94.81 mg).

MI/HA, evaluated by the method described in Example 6-1, was 5.0 (mol/mol).

(Example 7-2)

Preparation of HA-EDOBEA-$(CH_2)_{10}$-MI/SUC and HA-EDOBEA-$(EO)_4$-MI/SUC

To aqueous solutions (10 mg/mL, 3.5 and 4.0 mL) of HA-EDOBEA (100 kDa HA-TBA, Incorporation rate of EDOBEA: 95.5%), which was obtained as described in Example 3-1 except that 2.5 equivalent BOP reagent per HA unit was used, 0.2 M phosphate buffer (pH 7.0, 4.375, 5.0 mL) and 0.1 M phosphate buffer (pH 7.0, 8.75, 10.0 mL) were added. DMSO solution (0.858 mg/mL, 0.875 mL) of Sulfo-KMUS (Piece Co. Ltd.) or DMSO solution (1.528 mg/mL, 1.0 mL) of NHS-$(EO)_4$-MI (Quanta BioDesign Co. Ltd.) was added to each solution and shaken at room temperature for 30 minutes. DMSO solutions of succinic anhydride (283 mg/mL, 0.875, 1.0 mL) were added to each mixture and the mixture was shaken at room temperature for another 1.5 hours, The mixtures were dialyzed/purified (Spectra/por 4, MWCO: 12 k - 14 kDa) against a large excess amount of distilled water (Milli Q water) at 4°C and freeze dried to obtain HA-EDOBEA-$(CH_2)_{10}$-MI/SUC and HA-EDOBEA-$(EO)_4$-MI/SUC (37.87, 42.42 mg).

MI/HA of each, evaluated by the method described in Example 6-1, was 4.7, 4.7 (mol/mol).

(Example 7-3)

Preparation of solutions of HA-GLP-1 analogue 2 and GLP-1 analogue 1 conjugates

GLP-1 analogue 2 solution in 0.2 M phosphate buffer (pH 7.0) (2.0 mg/mL, 0.895 mL) was added to HA-EDOBEA-$(CH_2)_{10}$-MI/SUC (MI/HA = 5.0 (mol/mol)) aqueous solution (20 mg/mL, 0.15 mL) obtained in Example 7-1. GLP-1 analogue 2 or GLP analogue 1 solutions in 0.2 M phosphate buffer (pH 7.0) (2.0 mg/mL, 0.1527, 0.1684 mL) was added to HA-EDOBEA-$(CH_2)_{10}$-MI/SUC (MI/HA = 4.7 (mol/mol)) aqueous solution (20 mg/mL, 0.15, 0.15 mL) obtained in Example 7-2. Also, GLP-1 analogue 1 solution in 0.2 M phosphate buffer (pH 7.0) (2.0 mg/mL, 0.1539 mL) was added to HA-EDOBEA-$(EO)_4$-MI/SUC (MI/HA = 4.7 (mol/mol)) aqueous solution (20.0 mg/mL, 0.15 mL) obtained in Example 7-2. Each mixture was left standing at 37°C for 1 hour. Cysteine hydrochloride monohydrate solutions in 0.1

M phosphate buffer (pH 7.0) (11.22 (used in HA modification product described in Example 7-1), 21.28 (used in HA modification product described in Example 7-2) mg/mL, each 0.15 mL) was added, and the mixtures were left standing at 37°C for 30 minutes. The reaction mixtures subjected to GPC under the following condition to obtain the conjugate fraction. The obtained fractions were concentrated by centrifugal ultrafiltration (Centricon Plus-20, nominal molecular weight limit: 30,000, Millipore Co. Ltd) until the volume became about 1/20. The concentrated solution was diluted about 20 fold with PBS, and again was concentrated and diluted by a similar procedure. The solution was again concentrated and the volumes of the obtained solution was adjusted with PBS to about 1.2 mL (used in HA modification product described in Example 7-1), 0.6 mL(used in HA modification product described in Example 7-1) to obtain the HA-GLP-1 analogue 2 conjugate and HA-GLP-1 analogue 1 conjugate solutions (sample No. 7-3-1-7-3-4). The results of assay, carried out according to the method of Example 6-2 except the dilution rate of sample solution was changed appropriately, are shown in Table 14.

GPC Condition

System: FPLC or AKTAexplorer (Amersham Bioscience Co. Ltd.)
GPC Column: Superdex 200 10/300 GL (Amersham Bioscience Co. Ltd.)
Mobile phase: 30% acetonitrile, 0.1% trifluoroacetic acid aqueous solution
Flow rate: 0.6 mL/min
Detection: UV (280 nm)

[Table 14]

Table 14. Preparation of HA-GLP-1 analogue 2 and GLP-1 analogue 1 conjugate solution

| Sample | HA Molecular Weight (kDa) | $(CH_2)_{10}$ or $(EO)_4$ | MI/HA (mol/mol) | GLP-1 Analogue 2 or 1 | GLP-1 Analogue (nmol/mL) | HA-EDOBEA-$(CH_2)_{10}$ or $(EO)_4$-MI/SUC (mg/mL) | GLP-1 Analogue /HA (mol/mol) |
|---|---|---|---|---|---|---|---|
| 7-3-1 | 200 | $(CH_2)_{10}$ | 5.0 | 2 | 27.1 | 1.87 | 4.5 |
| 7-3-2 | 100 | $(CH_2)_{10}$ | 4.7 | 1 | 118.7 | 4.02 | 4.6 |
| 7-3-3 | 100 | $(CH_2)_{10}$ | 4.7 | 2 | 112.2 | 3.88 | 4.5 |
| 7-3-4 | 100 | $(EO)_4$ | 4.7 | 1 | 128.2 | 3.80 | 5.2 |

(Example 7-4)

Evaluation for CAMP production capability of A-GLP-1 analogue conjugates

cAMP production capability of HA-GLP-1 analogue conjugates obtained in Example 7-3 was measured by the similar method to that in Example 6-3.

In Table 15, the cyclic AMP production capability of various HA-GLP-1 analogue conjugates is expressed as a relative value when the EC50 value of native GLP-1 (Human, 7-37) is assumed to be 1.

[Table 15]

Table 15. CAMP production capability of HA-GLP-1 analogue 1 and GLP-1 analogue 2 conjugates

| Test sample | EC50 Value (Relative Value) |
|---|---|
| GLP-1 | 1 |
| 7-3-1 | 77 |
| 7-3-2 | 206 |
| 7-3-3 | 74 |
| 7-3-4 | 80 |

It was confirmed that HA-GLP-1 analogue 2 conjugates kept CAMP production capability as GLP-1 in both cases, where HA with 200 kDa molecular weight was used, and hydrophobic $(CH_2)_{10}$ group was used as a linker between water soluble HA modification product and maleimide group.

(Example 7-5)

Evaluation for blood residence of A-GLP-1 analogue conjugates

The HA-GLP-1 analogue conjugate solutions obtained in Example 7-3 were diluted with solvent Z and administered once to rats at a dose of 50 μg/kg intravenously and blood samples were collected (heparin treatment) at 1, 4, 8, 24, 48, 72, 120 and 168 hours after the administration. Plasma samples were obtained by centrifugation and kept frozen at -20°C or lower until measurement.

Measuring Method

Samples for the standard curve and for measurement were distributed in a 96 well plate of GLP-1 (Active) ELISA KIT (Linco Research, Inc.; hereinafter also called "Kit W") and analyzed using a microplate reader. Conditions are as follows.

Microplate reader: SPECTRA MAX GEMINI (Molecular Devices Co. Ltd.)

Detection: Fluorescence(EX: 355 nm, EM: 460 nm)

Preparation of Samples for Measurement

Samples for the standard curve;

HA-GLP-1 analogue conjugate solutions obtained in Example 7-3 were diluted with solvent Z and Assay Buffer included in Kit W and the standard solutions of 3.2, 1.6, 0.8, 0.4, 0.2, 0.1, 0.05 μg/mL and 0 μg/mL were prepared. Samples for the standard curve were prepared by adding 5 μL of normal rat plasma to these standard solutions.

· Preparation of samples for measurement;

Samples for measurement were prepared by adding Assay Buffer included in Kit W to the plasma samples of HA-GLP-1 analogue conjugate administered rats.

· Calculation for the concentration of HA modification product in the plasma;

Concentration of HA-GLP-1 analogue conjugates in the plasma was calculated from the standard curve obtained from the fluorescent intensity of each standard solution using an analytical software, SOFTmax Pro (Molecular Devices Co. Ltd.).

Pharmacokinetic Data

From the data of the change in the plasma concentrations of administered HA-GLP-1 analogue conjugates, pharmacokinetic parameters were calculated using WinNonlin Ver 4.0.1 (Pharsight Co. Ltd.). Model independent analysis was carried out on the change in the plasma concentration of each animal to obtain mean residence time in blood (MRT). Half life (tl/2) was calculated using 3 points data of the final measurement of individual animal. Change in the plasma concentration of HA-GLP-1 analogue conjugates is shown in Figure 13. Also, calculated pharmacokinetic parameters are shown in Table 16.
[Table 16-1]

Table 16. Pharmacokinetic parameters after HA-GLP-1 analogue conjugates were intravenously administered to rats

| Test Sample 7-3-1 | Mean Value | Standard Deviation | |
|---|---|---|---|
| Half Life | 18.2 | 2.3 | (hr) |
| Clearance | 1.43 | 0.15 | (mL/hr/kg) |
| Mean Residence Time | 25.3 | 1.9 | (hr) |
| Distribution Volume | 36.1 | 3.1 | (mL/kg) |

(continued)

| Test Sample 7-3-2 | Mean Value | Standard Deviation | |
|---|---|---|---|
| Half Life | 23.6 | 8.3 | (hr) |
| Clearance | 0.86 | 0.09 | (mL/hr/kg) |
| Mean Residence Time | 37.3 | 4.5 | (hr) |
| Distribution Volume | 31.9 | 2.6 | (mL/kg) |

[Table 16-2]

| Test Sample 7-3-3 | Mean Value | Standard Deviation | |
|---|---|---|---|
| Half Life | 18.8 | 1.4 | (hr) |
| Clearance | 1.50 | 0.10 | (mL/hr/kg) |
| Mean Residence Time | 29.1 | 0.6 | (hr) |
| Distribution Volume | 43.7 | 2.8 | (mL/kg) |

| Test Sample 7-3-4 | Mean Value | Standard Deviation | |
|---|---|---|---|
| Half Life | 26.3 | 0.8 | (hr) |
| Clearance | 1.23 | 0.06 | (mL/hr/kg) |
| Mean Residence Time | 41.2 | 1.1 | (hr) |
| Distribution Volume | 50.6 | 3.4 | (mL/kg) |

It became apparent that the half life was 18-26 hours and the mean blood residence was 25.3-41.2 hours by forming HA-GLP-1 analogue conjugate using any peptide and linker, indicating the great improvement of the residence time.

It is indicated that the molecular weight and the linker structure of HA-GLP-1 analogue conjugates may be chosen according to the objective.

[Example 8]

Preparation and Evaluation of water soluble HA modification product-GLP-1 conjugates

Water soluble HA modification product-GLP-1 analogue conjugates having various incorporation rate of GLP-1 analogue were prepared.

(Example 8-1)

Preparation of HA-EDOBEA-$(EO)_4$-MI/SUC

To aqueous solutions (10 mg/mL, 5.0, 9.0 mL) of HA-EDOBEA (100 kDa HA-TBA, Incorporation rate of EDOBEA: 100%), which was obtained as described in Example 3-1 except that 2.5 equivalent BOP reagent per HA unit was used, 0.2 M phosphate buffer (pH 7.0, 6.25, 11.25 mL) was added. DMSO solutions (0.377, 1.510 mg/mL, 1.25, 2.25 mL) of NHS-$(EO)_4$-MI (Quanta BioDesign Co. Ltd.) were added to each mixture. Also to each of 3 aqueous solutions (10 mg/ml, 5.0 mL) of the same HA-EDOBEA (100 kDa HA-TBA, Incorporation rate of EDOBEA: 100%), 0.2 M phosphate buffer (pH7.0, 6.25 ml) and 0.1 M phosphate buffer (pH 7.0, 37.5 mL) were added, respectively. DMSO solutions (2.114, 2.717, 3.321 mg/mL, 1.25 mL) of NHS-$(EO)_4$-MI (Quanta BioDesign Co. Ltd.) were added to each mixture. The mixtures were shaken at room temperature for 30 minutes. DMSO solution of succinic anhydride (293 mg/mL, an equal volume to the DMSO solution of NHS-$(EO)_4$-MI) was added to each mixture and the mixtures were shaken at room temperature for another 1.5 hours. The mixture was dialyzed/purified (Spectra/por 4, MWCO: 12 k - 14 kDa) against a large excess amount of distilled water (Milli Q water) at 4°C and freeze dried to obtain HA-EDOBEA-$(EO)_4$-MI/SUC to which maleimide group and succinic acid was incorporated (57.78, 99.24, 61.33, 60.92, 59.02 mg).

Each MI/HA was evaluated by the method described in Example 6-1 to be 1.2, 4.7, 6.0, 7.3, 9.3 (mol/mol).

The amount of incorporated maleimide (maleimide/HA (mol/mol)) was possible to be controlled by added

amount of NHS-$(EO)_4$-MI.

(Example 8-2)

Preparation of HA-GLP-1 analogue 2 conjugate solution

To each of HA-EDOBEA-$(EO)_4$-MI/SUC aqueous solution obtained in Example 8-1 (20 mg/mL, 0.15 mL), GLP-1 analogue 2 solution in 0.2 M phosphate buffer (pH 7.0) was added so that MI and GLP-1 analogue 2 were equimolar (2.0 mg/mL, 0.432, 0.1677, 0.2128, 0.2588, 0.3319 mL) and the mixtures were left standing at 37°C for 1 hour. 0.1 M phosphate buffer (pH 7.0) solution of cysteine hydrochloride monohydrate (4.21, 9.93, 11.03, 11.90, 12.95 mg/mL, 0.0193, 0.0318, 0.0363, 0.0409, 0.0482 mL) was added to each mixture and the mixtures were left standing at 37°C for another 30 minutes. The reaction mixtures were subjected to GPC under the condition described in Example 7-3 to obtain the conjugate fractions. Thus obtained conjugate fraction was filtered (MILLEX-GV, $\phi$ = 0.22 $\mu$m, Millipore Co. Ltd.) and then concentrated by centrifugal ultrafiltration (Centricon Plus-20, nominal molecular weight limit: 30,000, Millipore Co. Ltd) until the volume became about 1/20. The concentrated solution was diluted about 20 fold with PBS, and again was concentrated and diluted by a similar procedure. The solution was again concentrated and the volume of the obtained solution of the concentrated solutions was adjusted with PBS to about 0.48, 0.45, 0.48, 0.60, 0.60 mL. The solutions were filtered (MILLEX-GV, $\phi$ = 0.22 $\mu$m, Millipore Co. Ltd.) to obtain the HA-GLP-1 analogue 2 conjugate solutions described in the title of this Example (Sample No. 8-2-1 to 8-2-5). The assay was carried out according to the method in Example 6-2 except dilution rate of the samples are appropriately changed. Table 17 shows the results.
[Table 17]
Table 17. Preparation of HA-GLP-1 analogue 2

| Sample | MI/HA (mol/mol) | GLP-1 Analogue (nmol/mL) (mg/mL) | HA-EDOBEA-$(EO)_4$-MI/SUC | GLP-1 Analogue/HA (mol/mol) |
|---|---|---|---|---|
| 8-2-1 | 1.2 | 27.1 | 1.87 | 1.2 |
| 8-2-2 | 4.7 | 118.7 | 4.02 | 5.9 |
| 8-2-3 | 6.0 | 112.2 | 3.88 | 7.0 |
| 8-2-4 | 7.3 | 112.2 | 3.88 | 7.5 |
| 8-2-5 | 9.3 | 112.2 | 3.8 | 10.7 |

It is believed that the GLP-1 analogue incorporated can be assayed as the amount close to the quantity of maleimide by adding an equal amount of GLP-1 analogue to the quantity of maleimide, and the GLP-1 analogue is incorporated to the conjugate almost qualitatively.
The fact that the amount of incorporation of maleimide and the GLP-1 analogue can be controlled suggests that the conjugates may be produced with good reproducibility.

(Example 8-3)

Evaluation for cAMP production capability of HA-GLP-1 analogue 2 conjugates

cAMP production capability of various HA-GLP-1 analogue conjugates obtained in Example 8-2 was measured by the similar method to that in Example 6-3.
In Table 18, the cyclic AMP production capability of various HA-GLP-1 analogue conjugates is expressed as a relative value when the EC50 value of native GLP-1 is 1.
[Table 18]
Table 18 cAMP production capability of HA-GLP-1 analogue 1 and GLP-1 analogue 2 conjugates

| Test sample | EC50 Value (Relative Value) |
|---|---|
| GLP-1 | 1 |
| 8-2-1 | 99 |
| 8-2-2 | 79 |

(continued)

Table 18 cAMP production capability of HA-GLP-1 analogue 1 and GLP-1 analogue 2 conjugates

| Test sample | EC50 Value (Relative Value) |
|---|---|
| 8-2-3 | 79 |
| 8-2-4 | 95 |
| 8-2-5 | 151 |

It was confirmed that HA-GLP-1 analogue 2 conjugate kept the CAMP production capability as GLP-1 after changing the incorporation rate of GLP-1 analogue.
When HA-GLP-1 analogue conjugates are used as a long-acting prophylactic or a long-acting therapeutic medicament for diabetes, diabetic complications and/or obesity the incorporation rate of GLP-1 analogue may be chosen from the range, in which the increase of viscosity due to HA is acceptable, in the administration dosage that is determined by the amount of administered GLP-1 and administration method.

[Example 9]

Preparation and evaluation of water soluble HA modification product-GLP-1 analogue conjugates

Water soluble HA modification product-GLP-1 analogue conjugates with various GLP-1 analogues having different incorporation rates of GLP-1 analogue were prepared.

(Example 9-1)

Preparation of HA-GLP-1 analogue conjugate solutions

To HA-EDOBEA-$(CH_2)_{10}$-MI/SUC aqueous solutions obtained in Example 7-1 (MI/HA = 5.0 (mol/mol)) (20 mg/mL, 0.15 mL), GLP-1 analogue 1 or 2 solutions in 0.2 M phosphate buffer (pH 7.0) was added so that GLP-1 analogue/HA was 1.0, 2.0, 4.0, 5.0, 6.0, 7.5 (mol/mol) (GLP-1 analogue 1: 2.0 mg/mL, 0.0162, 0.0325, 0.0649, 0.0812, 0.0974, 0.1217 mL, GLP-1 analogue 2: 2.0 mg/mL, 0.0179, 0.0358, 0.0716, 0.0895, 0.1074, 0.1343 mL) and the mixtures were left standing at 37°C for 1 hour. 0.1 M phosphate buffer (pH 7.0) solution of cysteine hydrochloride monohydrate (11.22 mg/mL, 0.0150 mL) was added to each mixture and the mixtures were left standing at 37°C for another 30 minutes. The reactions mixtures were subjected to GPC under the condition described in Example 7-3 to obtain the conjugate fractions. Thus obtained conjugate fraction was filtered (MILLEX-GV, $\phi$ = 0.22 $\mu$m, Millipore Co. Ltd.) and then concentrated by centrifugal ultrafiltration (Centricon Plus-20 nominal molecular weight limit: 30,000, Millipore Co. Ltd) until the volume became about 1/20. The concentrated solutions were diluted about 20 fold with PBS, and again were concentrated and diluted by a similar procedure. The solutions were again concentrated and the volume of the concentrated solutions was adjusted with PBS to about 0.48, 0.45, 0.48, 0.60, 0.60 mL. The solutions were filtered (MILLEX-GV, $\phi$ = 0.22 $\mu$m, Millipore Co. Ltd.) to obtain the HA-GLP-1 analogue 1 conjugate and HA-GLP-1 analogue 2 conjugate solutions described in the title of this Example. The assay was carried out according to the method in Example 6-2 except dilution rate of the samples are appropriately changed.
Table 19 shows the results.
[Table 19]

Table 19. Preparation of HA-GLP-1 analogue 1 and HA-GLP-1 analogue 2

| Sample | MI/HA (mol/mol) | Input GLP-1 Analogue/ MI (mol/mol) | GLP-1 Analogue 1 or 2 | GLP-1 Analogue (nmol/mL) | HA-EDOBEA-$(CH_2)_{10}$-MI/SUC (mg/ML) | GLP-1 Analogue/ HA (mol/mol) |
|---|---|---|---|---|---|---|
| 9-1-1 | 5.0 | 1.0 | 5 | 13.6 | 4.39 | 1.0 |
| 9-1-2 | 5.0 | 2.0 | 5 | 28.6 | 4.76 | 1.9 |
| 9-1-3 | 5.0 | 4.0 | 5 | 57.2 | 3.65 | 4.9 |
| 9-1-4 | 5.0 | 5.0 | 5 | 65.7 | 4.32 | 4.8 |
| 9-1-5 | 5.0 | 6.0 | 5 | 67.3 | 3.65 | 4.9 |
| 9-1-6 | 5.0 | 7.5 | 5 | 78.2 | 4.32 | 5.1 |

(continued)

Table 19. Preparation of HA-GLP-1 analogue 1 and HA-GLP-1 analogue 2

| Sample | MI/HA (mol/mol) | Input GLP-1 Analogue/ MI (mol/mol) | GLP-1 Analogue 1 or 2 | GLP-1 Analogue (nmol/mL) | HA-EDOBEA-$(CH_2)_{10}$-MI/SUC (mg/ML) | GLP-1 Analogue/ HA (mol/mol) |
|---|---|---|---|---|---|---|
| 9-1-7 | 5.0 | 1.0 | 6 | 15.8 | 4.26 | 1.1 |
| 9-1-8 | 5.0 | 2.0 | 6 | 31.2 | 4.81 | 2.0 |
| 9-1-9 | 5.0 | 4.0 | 6 | 59.9 | 4.25 | 4.4 |
| 9-1-10 | 5.0 | 5.0 | 6 | 75.0 | 4.60 | 5.1 |
| 9-1-11 | 5.0 | 6.0 | 6 | 61.6 | 3.59 | 5.4 |
| 9-1-12 | 5.0 | 7.5 | 6 | 69.5 | 4.64 | 4.7 |

When maleimide incorporation (MI/HA (mol/mol)) was constant, it was shown that the incorporation rate of GLP-1 analogue could be controlled by adjusting the added amount of GLP-1 analogue. On the other hand, an increase of the added amount of GLP-1 analogue from maleimide equivalent 1 to 1.5 resulted in almost no change of the GLP-1 incorporation rate, suggesting that the conjugate was formed by the specific reaction between maleimide and thiol group of GLP-1 analogue.

(Example 9-2)

Evaluation of CAMP production capability of HA-GLP-1 analogue conjugate CAMP production capability of HA GLP-1 analogue conjugates obtained in Example 9-1 was measured by the similar method to that in Example 6-3.
In Table 20, the cyclic AMP production capability of various HA-GLP-1 analogue conjugates is expressed as a relative value when the EC50 value of native GLP-1 is 1.
[Table 20]

Table 20. CAMP production capability of HA-GLP-1 analogue 1 and GLP-1 analogue 2 conjugates

| Test sample | EC50 Value (Relative Value) |
|---|---|
| GLP-1 | 1 |
| 9-1-1 | 350 |
| 9-1-2 | 379 |
| 9-1-3 | 691 |
| 9-1-4 | 439 |
| 9-1-5 | 576 |
| 9-1-6 | 441 |
| 9-1-7 | 153 |
| 9-1-8 | 115 |
| 9-1-9 | 112 |
| 9-1-10 | 109 |
| 9-1-11 | 162 |
| 9-1-12 | 177 |

It was confirmed that HA-GLP-1 analogue conjugates kept the CAMP production capability as GLP-1 after changing the incorporation rate of GLP-1 analogue to maleimide.

[Example 10]

Preparation and evaluation of water soluble HA modification product-GLP-1 analogue 1 and GLP-1 analogue 3 conjugates

Water soluble HA modification product-GLP-1 analogue conjugates having different GLP-1 analogue C terminals were prepared.

(Example 10-1)

Preparation of HA-EDOBEA-$(EO)_4$-MI/SUC

To aqueous solution (10 mg/mL, 5.0 mL) of HA-EDOBEA (100 kDa HA-TBA, Incorporation rate of EDOBEA: 97.0 %), which was obtained as described in Example 3-1 except that 2.5 equivalent BOP reagent per HA unit was used, 0.2 M phosphate buffer (pH 7.0, 6.25 mL) and 0.1 M phosphate buffer (pH 7.0, 37.5 mL) were added, respectively. DMSO solution (1.826 mg/mL, 1.25 mL) of NHS-$(EO)_4$-MI (Quanta BioDesign Co. Ltd.) was added to the mixture and the mixture was shaken at room temperature for 30 minutes, DMSO solution of succinic anhydride (287 mg/mL, 1.25 mL) was added to the mixture and the mixture was shaken at room temperature for another 1.5 hours. The mixture was dialyzed/purified (Spectra/por 4, MWCO: 12 k - 14 kDa) against a large excess amount of distilled water (Milli Q water) at 4°C and freeze dried to obtain HA-EDOBEA-$(EO)_4$-MI/SUC (54.0 mg).

MI/HA was evaluated by the method described in Example 6-1 to be 7.7 (mol/mol).

(Example 10-2)

Preparation of HA-GLP-1 analogue 3 and GLP-1 analogue 1 conjugate solutions

A variant of native type GLP-1 (Human, 7-37; JP Patent Publication (Kohyo) No. 7-504679) in which the second (position 8) alanine was converted to glycine, the $31^{st}$ (position 37) glycine was converted to cysteine, and the C-terminal was amidated (hereinafter also called "GLP-1 analogue 3") was obtained by a solid phase peptide synthesis method (Peptide Institute Inc.). To aqueous solution (20 mg/mL, 0.15 mL) of HA-EDOBEA-$(EO)_4$-MI/SUC obtained in Example10-1 (MI/HA = 7.7 (mol/mol)), GLP-1 analogue 3 or solution of GLP-1 analogue 3 in 0.2 M phosphate buffer (pH 7.0) (2.0 mg/mL, 0.250 mL) was added. Each mixture was left standing at 37°C for 1 hour. Cysteine hydrochloride hydrate solution (12.96 mg/mL, 0.040 mL each) in 0.1 M phosphate buffer (pH 7.0) was added and the mixture was left standing at 37°C for another 30 minutes. The reaction mixtures were subjected to GPC under the following condition to obtain the conjugate fraction. Thus obtained conjugate fraction was filtered (MILLEX-GV, $\phi$ = 0.22 $\mu$m, Millipore Co. Ltd.) and then concentrated by centrifugal ultrafiltration (Centricon Plus-20, nominal molecular weight limit: 30,000, Millipore Co. Ltd) until the volume became about 1/20. The concentrated solution was diluted about 20 fold with PBS, and again was concentrated and diluted by a similar procedure. The solution was again concentrated and the volume of the obtained solution was adjusted with PBS to about 0.6 ml and filtered (MILLEX-GV, $\phi$ = 0.22 $\mu$m, Millipore Co. Ltd.) to obtain the HA-GLP-1 analogue 1 and analog 3 conjugate solutions described in title of this Example (Sample No. 10-2-1 to 10-2-2). The results of assay, carried out according to the method of Example 6-2 except that the dilution rate of sample solution was changed appropriately, are shown in Table 21.

GPC Condition

System: FPLC (Amersham Bioscience Co. Ltd.)
GPC Column: Superdex 200 10/300 GL (Amersham Bioscience Co. Ltd.)
Mobile phase: 30% acetonitrile, 0.1% trifluoroacetic acid solution
Flow rate: 0.6 mL/min
Detection: UV (280 nm)

[Table 21]

Table 21. Preparation of HA-GLP-1 analogue 3 and GLP-1 analogue 1 conjugate solutions

| Sample | HA Molecular Weight (kDa) | MI/HA (mol/mol) | GLP-1 Analogue 3 or 1 | GLP-1 Analogue (nmol/mL) | HA-EDOBEA-$(EO)_4$-MI/SUC (mg/mL) | GLP-1 Analogue/ HA (mol/mol) |
|---|---|---|---|---|---|---|
| 10-2-1 | 100 | 7.7 | 3 | 166.3 | 2.94 | 8.9 |
| 10-2-2 | 100 | 7.7 | 1 | 191.1 | 3.04 | 9.9 |

(Example 10-3)

Evaluation for CAMP production capability of HA-GLP-1 analogue conjugates

CAMP production capability of HA-GLP-1 analogue 3 and GLP-1 analogue 1 conjugates obtained in Example 10-2 was measured by the similar method to that in Example 6-3.

In Table 22, the cyclic AMP production capability of HA-GLP-1 analogue 3 and GLP-1 analogue 1 conjugates is expressed as a relative value when the EC50 value of native GLP-1 is 1.

[Table 22]

Table 22. cAMP production capability of HA-GLP-1 analogue 3 and GLP-1 analogue 1 conjugates

| Test sample | EC50 Value (Relative Value) |
|---|---|
| GLP-1 | 1 |
| 10-2-1 | 146 |
| 10-2-2 | 575 |

It was confirmed that HA-GLP-1 analogue conjugate kept the production capability as GLP-1 after changing the C terminal of GLP-1 analogue from carboxyl group to amide group.

## Claims

1. A hyaluronic acid-peptide conjugate or a salt thereof wherein one or more glucagon-like peptide-1 (GLP-1) analogues are bound with a water-soluble hyaluronic acid modification product.

2. The hyaluronic acid-peptide conjugate or a salt thereof according to Claim 1, wherein the GLP-1 analogues are bound with the hyaluronic acid modification product through a divalentlinker.

3. The hyaluronic acid-peptide conjugate or a salt thereof according to Claim 1 or 2, wherein 70% or more of carboxyl groups contained in the glucuronic acid portion of hyaluronic acid is converted to an N-substituted amide group in the hyaluronic acid modification product, in which the substituents of respective N-substituted amide groups in the hyaluronic acid modification product may be the same or different and at least one of the substituents is a divalent linker which is linked with the GLP-1 analogues.

4. The hyaluronic acid-peptide conjugate or a salt thereof according to any one of Claims 1 to 3, used for prevention or treatment of a disease selected from diabetes, hyperglycemia, diabetic complication and obesity.

5. The hyaluronic acid-peptide conjugate or a salt thereof according to any one of Claims 1 to 4, wherein the GLP-1 analogue is a peptide with an amino acid sequence of GLP-1 (1-36) or GLP-1 (7-36) with an amino acid sequence represented by -Xa-Cys at the C-terminal thereof, or a peptide with an amino acid sequence of the peptide in which 1 to 5 amino acids are deleted, substituted and/or added wherein the amino acid sequence may be substituted and/or added with a natural amino acid and/or a non-natural amino acid, in which Xa is a direct bond or a sequence comprising 1 to 9 amino acids independently selected from proline, glycine, serine and glutamic acid, and wherein the carboxyl group of cysteine of the C-terminal of the peptide may be optionally converted to an amide group.

6. The hyaluronic acid-peptide conjugate or a salt thereof according to Claim 5, wherein the GLP-1 analogue is a peptide with an amino acid sequence of GLP-1 (1-36) or GLP-1 (7-36) added with an amino acid sequence represented by -Xa-Cys at the C-terminal thereof in which the 8-position of alanine ($Ala^8$) of the sequence is substituted with a natural amino acid or a non-natural amino acid, and wherein the carboxyl group of cysteine of the C-terminal of the peptide may be optionally converted to an amide group.

7. The hyaluronic acid-peptide conjugate or a salt thereof according to Claim 5, wherein the GLP-1 analogue is a peptide with an amino acid sequence of GLP-1 (1-36) or GLP-1 (7-36) added with an amino acid sequence represented by -Xa-Cys at the C-terminal thereof in which the 8-position of alanine ($Ala^8$) of the sequence is substituted with an amino acid selected from glycine, serine, valine, leucine, isoleucine and threonine, and wherein the carboxyl group of cysteine of the C-terminal of the peptide may be optionally converted to an amide group.

8. The hyaluronic acid-peptide conjugate or a salt thereof according to Claim 5, wherein the GLP-1 analogue is a peptide represented by His-Gly-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-Xa-Cys (Sequence Number 1) wherein the carboxyl group of cysteine of the C-terminal of the peptide may be optionally converted to an amide group.

9. The hyaluronic acid-peptide conjugate or a salt thereof according to any one of Claims 5 to 8, wherein Xa is a direct bond or -Gly-Pro-Pro-Pro-.

10. The hyaluronic acid-peptide conjugate or a salt thereof according to any one of Claims 1 to 4, wherein the GLP-1 analogue is a peptide with an amino acid sequence of GLP-1 (1-36), GLP-1 (1-37), GLP-1 (7-36) or GLP-1 (7-37) added with a thiol compound represented by -Qa-SH at the C-terminal thereof, or a peptide with the amino acid sequence of the peptide in which 1 to 5 amino acids are deleted, substituted and/or added in the amino acid sequence, wherein the amino acid sequence may be optionally substituted and/or added with a natural amino acid and/or a non-natural amino acid, in which Qa is selected from -NH-$X^5$-, -CO-$X^5$- and -CONH-$X^5$- and is linked with a carboxyl group, an amine group or a hydroxyl group which is contained in the C-terminal amino acid of the peptide, to form an amide bond, a urea bond or an ester bond, and
$X^5$ is a $C_{1-50}$ alkylene group in which an oxygen atom may be inserted between two carbon atoms contained in the alkylene group at one or more sites of the alkylene group and a carbon atom of the alkylene group may be independently substituted with one or more substituents selected from a hydroxyl group and a $C_{1-6}$ alkyl group.

11. The hyaluronic acid-peptide conjugate or a salt thereof according to any one of Claims 5 to 10, wherein one end of

a divalent linker is bound with a GLP-1 analogue via a mercapto group introduced into a GLP-1 analogue.

**12.** The hyaluronic acid-peptide conjugate or a salt thereof according to Claim 11, wherein the divalent linker is represented by the formula (I):

[Formula 1]

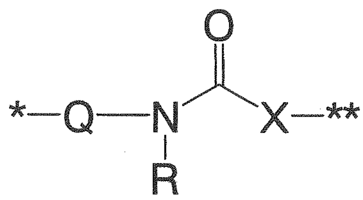

(I)

wherein Q is a $C_{1-400}$ alkylene group, in which an oxygen atom may be inserted between two carbon atoms contained in the alkylene group at one or more sites of the alkylene group, further, -CO-, -NHCO- or -CONH- may be optionally inserted between carbon atoms or at the end of the alkylene group at one or more sites of the alkylene group and a carbon atom of the alkylene group may be optionally substituted with one or more substituents selected from a hydroxyl group and a $C_{1-6}$ alkyl group independently;
X is $-CH_2-CH_2-$**, $-CH_2-CH_2-CH_2-$** or $-CH(-CH_3)-CH_2-$** and R is a hydrogen atom or a $C_{1-6}$ alkyl group; or
X and R together with a carbon atom and a nitrogen atom to which they are attached may form a group represented by formula (II);

[Formula 2]

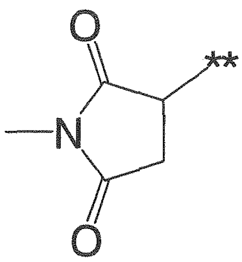

(II)

wherein

* represents the position linked to a nitrogen atom of an amide group in a hyaluronic acid modification product and ** represents the position linked to a sulfur atom of a mercapto group of the GLP-1 analogue.

**13.** The hyaluronic acid-peptide conjugate or a salt thereof according to Claim 9 or 10, wherein the divalent linker is a group represented by the formula (Ia):

[Formula 3]

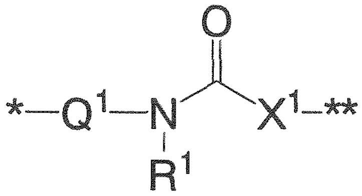

(Ia)

wherein $Q^1$ is a $C_{1-10}$ alkylene group, in which an oxygen atom may be inserted between two carbon atoms contained in the alkylene group at one or more sites of the alkylene group, and a carbon atom of the alkylene group may be optionally substituted with one or more substituents selected from a hydroxyl group and a $C_{1-6}$ alkyl group independently;

$X^1$ is $-CH_2-CH_2-**$, $-CH_2-CH_2-CH_2-**$ or $-CH(-CH_3)-CH_2-**$ and $R^1$ is a hydrogen atom or a $C_{1-6}$ alkyl group; or $X^1$ and $R^1$ together with a carbon atom and a nitrogen atom to which they are attached may form a group represented by the formula (IIa);

[Formula 4]

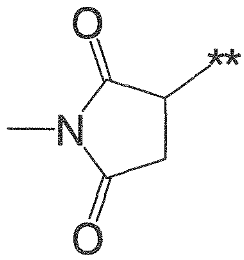

(IIa);

or

$X^1$ is a group represented by the formula (Ib):

[Formula 5]

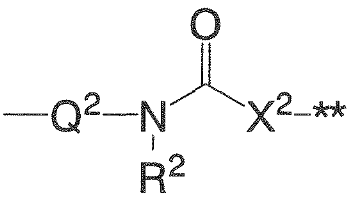

(Ib)

wherein $Q^2$ is a $C_{1-10}$ alkylene group, in which an oxygen atom may be inserted between two carbon atoms contained in the alkylene group at one or more sites of the alkylene group, and a carbon atom of the alkylene group may be optionally substituted with one or more substituents selected from a hydroxyl group and a $C_{1-6}$ alkyl group independently; ;

$X^2$ is $-CH_2-CH_2-**$, $-CH_2-CH_2-CH_2-**$ or $-CH(-CH_3)-CH_2-**$ and $R^2$ is a hydrogen atom or a $C_{1-6}$ alkyl group; or $X^2$ and $R^2$ together with a carbon atom and a nitrogen atom to which they are attached may form a group represented by the formula (IIb);

[Formula 6]

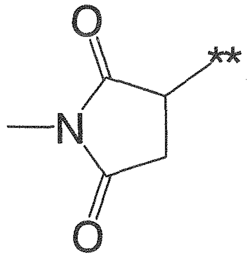

(IIb);

or

$X^2$ is a group represented by the formula (Ic):

[Formula 7]

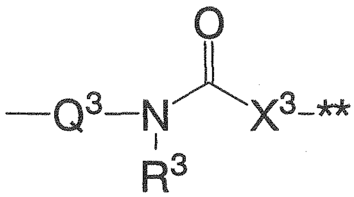

(Ic)

wherein $Q^3$ is a $C_{1-10}$ alkylene group, in which an oxygen atom may be inserted between two carbon atoms contained in the alkylene group at one or more sites of the alkylene group, and a carbon atom of the alkylene group may be optionally substituted with one or more substituents selected from a hydroxyl group and a $C_{1-6}$ alkyl group independently;

$X^3$ is $-CH_2-CH_2-$**, $-CH_2-CH_2-CH_2-$** or $-CH(-CH_3)-CH_2-$** and $R^3$ is a hydrogen atom or a $C_{1-6}$ alkyl group; or $X^3$ and $R^3$ together with a carbon atom and a nitrogen atom they are attached may form a group represented by the formula (IIc);

[Formula 8]

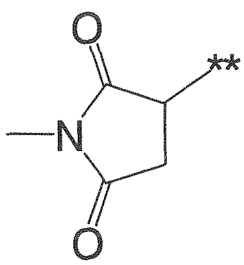

(IIc);

* represents the position linked to a nitrogen atom of an amide group in the hyaluronic acid modification product and ** represents the position linked to a sulfur atom of a mercapto group of the GLP-1 analogue.

**14.** The hyaluronic acid-peptide conjugate or a salt thereof according to any one of Claims 1 to 13, wherein one of the substituents introduced on the nitrogen atom of the amide group is represented by the formula (IV):

[Formula 9]

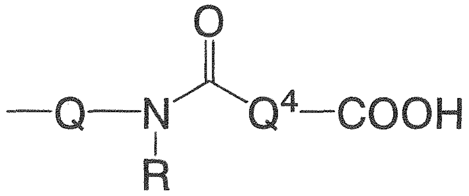

(IV)

wherein Q and R are as defined in Claim 12 and $Q^4$ is a $C_{1-6}$ alkylene group.

**15.** The hyaluronic acid-peptide conjugate or a salt thereof according to Claim 13 or 14, wherein $Q^1$ is represented by the formula: $-(CH_2)_m-$ or $-(CH_2)_m-(O-CH_2-CH_2)_n-$wherein m is an integer respectively selected from 1 to 10 independently and n is an integer selected from 1 to 200.

**16.** The hyaluronic acid-peptide conjugate or a salt thereof according to any one of Claims 13 to 15, wherein $X^1$ is a group represented by the formula: $-(CH_2)_m-(O-CH_2-CH_2)_p-NHCO-(CH_2)_q-Y^1-$

** or $-(CH_2)_r-Y^1-$**
Wherein m is an integer selected from 1 to 10, p is an integer selected from 1 to 200 and q and r are integers independently selected from 1 to 10, $Y^1$ is a group represented by the formula (IIc):

[Formula 10]

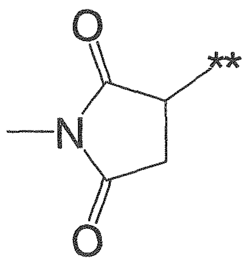

(IIc)

** represents the position linked to a sulfur atom of a mercapto group of the GLP-1 analogue.

**17.** The hyaluronic acid-peptide conjugate or a salt thereof according to any one of Claims 1 to 16, wherein the modification rate of a carboxyl group contained in hyaluronic acid to an N-substituted amide group is 85% by mole or more.

**18.** The hyaluronic acid-peptide conjugate or a salt thereof according to any one of Claims 1 to 17, wherein the introduction rate of an amide group substituted with a linker bound with a GLP-1 analogue is 0.1% by mole to 15% by mole in average, to a carboxyl group contained in hyaluronic acid.

**19.** The hyaluronic acid-peptide conjugate or a salt thereof according to any one of Claims 1 to 18, wherein viscosity average molecular weight is 5000 daltons to one million daltons.

**20.** A pharmaceutical composition comprising the hyaluronic acid-peptide conjugate or a salt thereof according to any one of Claims 1 to 19.

**21.** A pharmaceutical used for prevention or treatment of a disease selected from diabetes, hyperglycemia, diabetic complication and obesity, comprising the hyaluronic acid-peptide conjugate or a salt thereof according to any one of Claims 1 to 19.

**22.** The pharmaceutical composition according to Claim 20, administrated by means selected from intravenous administration, intramuscular administration, subcutaneous administration, intraperitoneal administration, intranasal administration and pulmonary administration.

**23.** A method for prevention or treatment of a disease selected from diabetes, hyperglycemia, diabetic complication and obesity, comprising an administration of a clinically effective amount of the hyaluronic acid-peptide conjugate or a salt thereof according to any one of Claims 1 to 19.

**24.** A process for producing the hyaluronic acid-peptide conjugate or a salt thereof according to any one of Claims 1 to 19, comprising a step of obtaining a water-soluble hyaluronic acid modification product by converting a carboxyl group contained in the glucuronic acid portion of hyaluronic acid to an N-substituted amide group in an aprotic polar solvent, using a condensing agent represented by the formula (V):

[Formula 11]

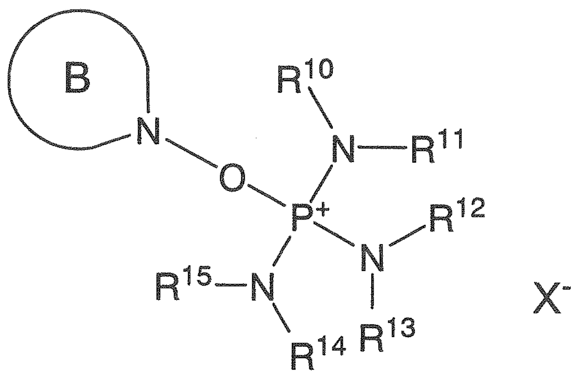

(V)

wherein each of $R^{10}$, $R^{11}$, $R^{12}$, $R^{13}$, $R^{14}$ and $R^{15}$ is independently selected from a $C_{1-6}$ alkyl group, or each of $R^{10}$ and $R^{11}$, $R^{12}$ and $R^{13}$ and $R^{14}$ and $R^{15}$ together with the nitrogen atom to which they are attached may independently form a nitrogen-containing heterocyclic ring, a ring B is a monocyclic or condensed nitrogen-containing heterocyclic ring which may be optionally substituted, and $X^-$ represents an anion.

**25.** The process according to Claim 24 wherein the above-mentioned aprotic polar solvent is selected from dimethylformamide, dimethylacetamide, dimethylsulfoxide, 1,3-dimethyl-2-imidazolidinone, sulfolane, N-methylpyrrolidone or a mixed solvent of 2 or more of the solvents.

**26.** The process according to Claim 25, wherein the aprotic polar solvent is dimethylsulfoxide.

**27.** The process according to any one of Claims 24 to 26, wherein the ring B in the formula (V) is benzotriazole-1-yl.

**28.** The process according to Claim 27, wherein the condensing agent is a BOP-type condensing agent selected from benzotriazol-1-yloxy-tris(dimethylamino)phosphonium hexafluorophosphate, benzotriazol-1-yloxy-tris(pyrrolidino) phosphonium hexafluorophosphate and a mixture thereof.

**29.** The hyaluronic acid-peptide conjugate or a salt thereof according to any one of Claims 1 to 19, which can be produced by the process according to any one of Claims 24 to 28.

**30.** A GLP-1 analogue which is a peptide with an amino acid sequence of GLP-1 (1-36) or GLP-1 (7-36) added with an amino acid sequence represented by -Xa-Cys at the C-terminal thereof, or a peptide with the amino acid sequence of the peptide in which 1 to 5 of amino acids are deleted, substituted and/or added in the amino acid sequence wherein the amino acid sequence may be optionally substituted and/or added with a natural amino acid or a non-natural amino acid, in which Xa is a direct bond or a sequence comprising 1 to 9 amino acids independently selected from proline, glycine, serine and glutamic acid, wherein the carboxyl group of cysteine of the C-terminal of the peptide may be optionally converted to an amide group.

**31.** The GLP-1 analogue, which is a peptide with an amino acid sequence of GLP-1 (1-36) or GLP-1 (7-36) added with an amino acid sequence represented by -Xa-Cys at C-terminal thereof in which the alanine in the 8-position ($Ala^8$) of the amino acid sequence is substituted with a natural amino acid or a non-natural amino acid, wherein the carboxyl group of cysteine of the C-terminal of the peptide may be optionally converted to an amide group and Xa is as defined in Claim 30.

**32.** The GLP-1 analogue according to Claim 31, which is a peptide with an amino acid sequence of GLP-1 (1-36) or GLP-1 (7-36) added an amino acid sequence represented by -Xa-Cys at the C-terminal thereof in which the alanine in the 8-position ($Ala^8$) of the amino acid sequence is substituted with amino acid selected from glycine, serine, valine, leucine, isoleucine and threonine and the carboxyl group of cysteine of the C-terminal of the peptide may be optionally converted to an amide group.

**33.** The GLP-1 analogue according to Claim 30, which is a peptide represented by Sequence Number 1 or a peptide in which the carboxyl group of cysteine of the C-terminal of the peptide is converted to an amide group.

**34.** The GLP-1 analogue according to Claim 33, wherein Xa is a direct bond or -Gly-Pro-Pro-Pro-.

[Figure 1]

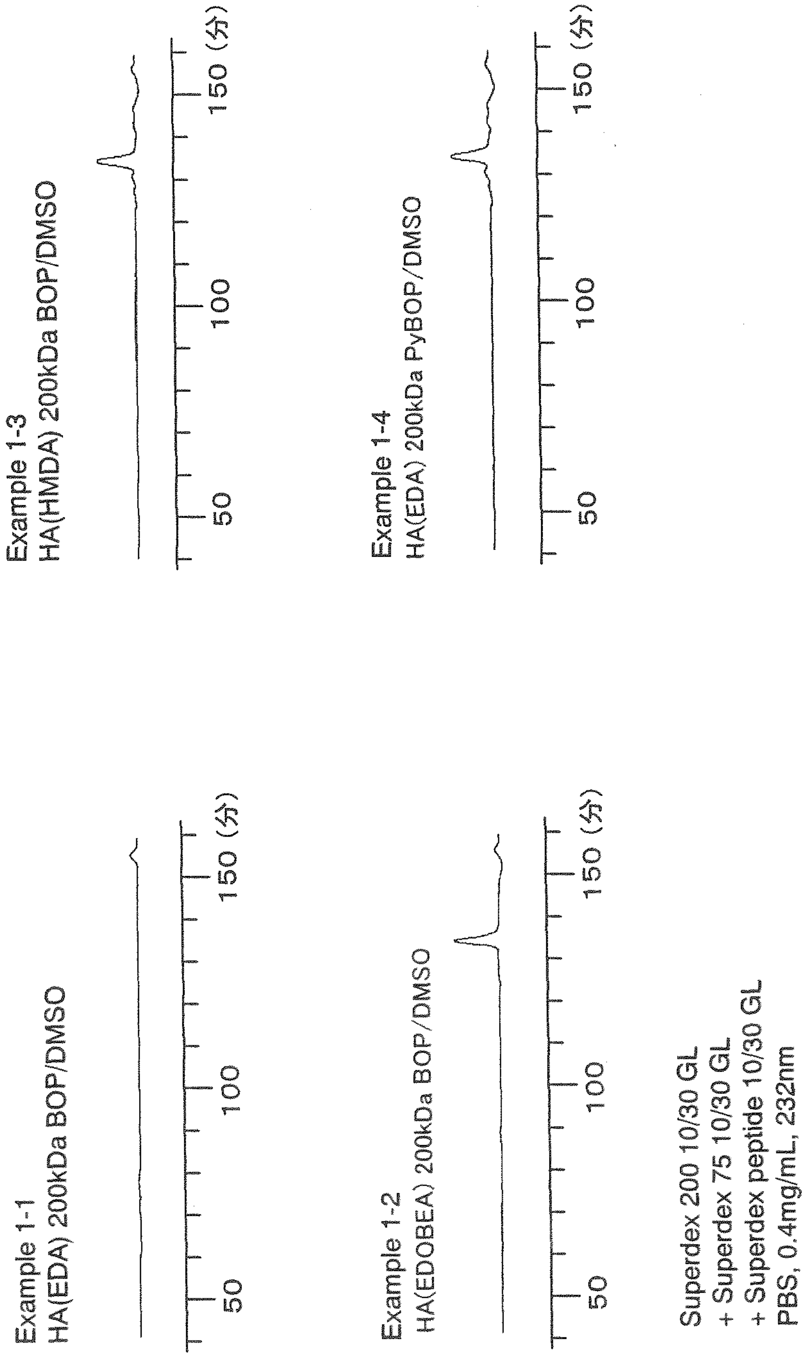
Example 1-1
HA(EDA) 200kDa BOP/DMSO
50
100
150 (分)
Example 1-2
HA(EDOBEA) 200kDa BOP/DMSO
50
100
150 (分)
Superdex 200 10/30 GL
+ Superdex 75 10/30 GL
+ Superdex peptide 10/30 GL
PBS, 0.4mg/mL, 232nm
Example 1-3
HA(HMDA) 200kDa BOP/DMSO
50
100
150 (分)
Example 1-4
HA(EDA) 200kDa PyBOP/DMSO
50
100
150 (分)

[Figure 2]

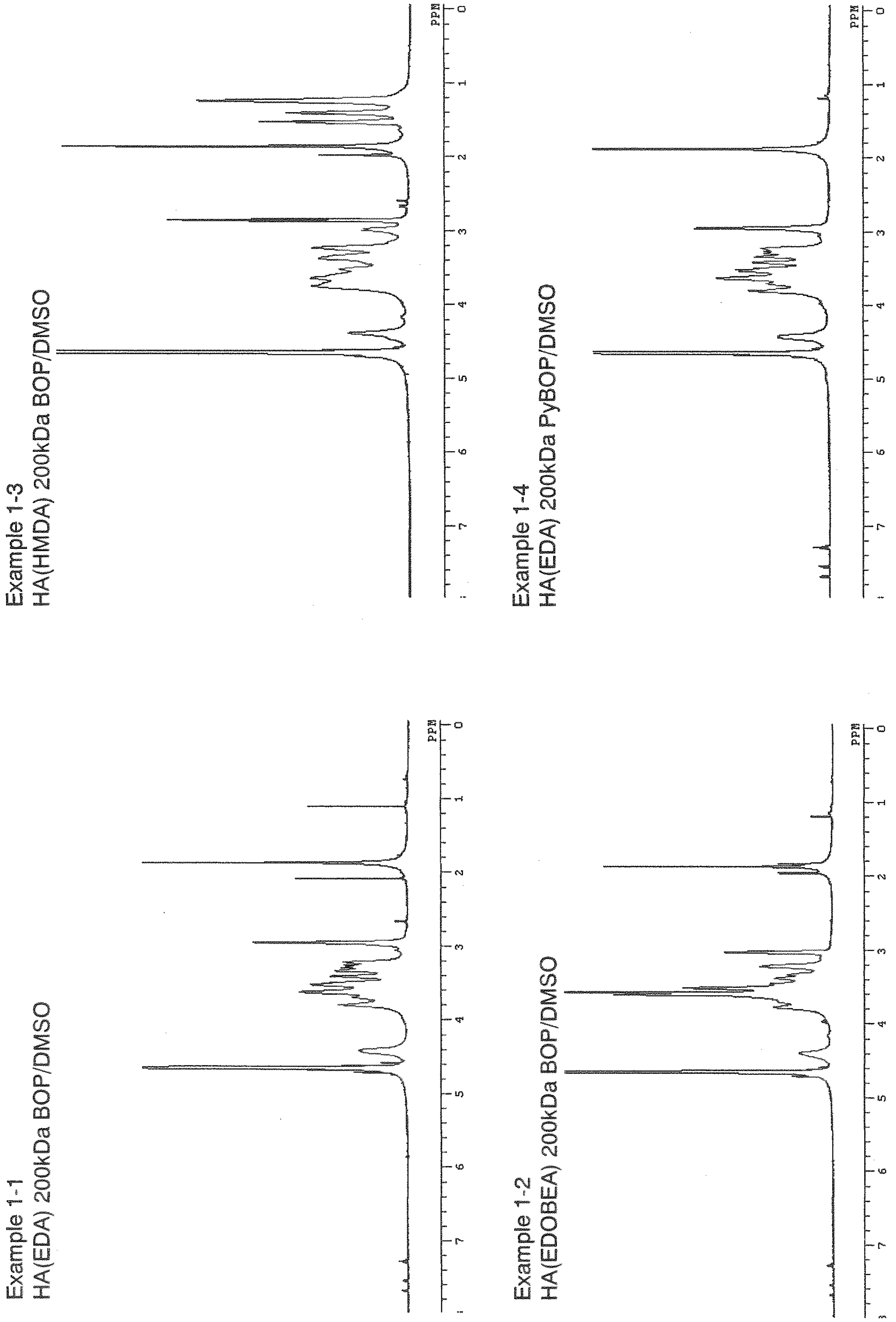
Example 1-3
HA(HMDA) 200kDa BOP/DMSO
Example 1-4
HA(EDA) 200kDa PyBOP/DMSO
Example 1-1
HA(EDA) 200kDa BOP/DMSO
Example 1-2
HA(EDOBEA) 200kDa BOP/DMSO
PPM

[Figure 3]

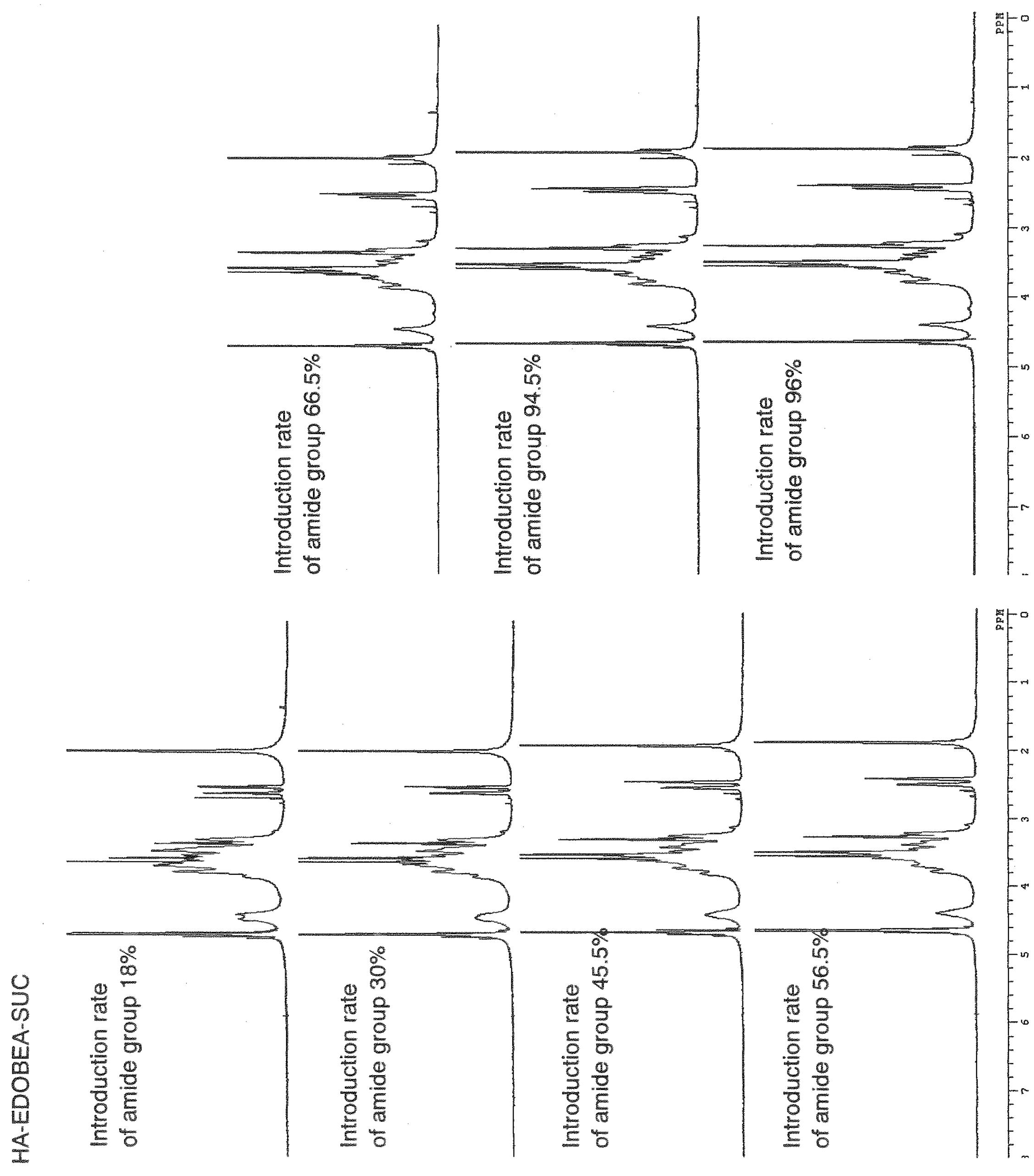
HA-EDOBEA-SUC
Introduction rate of amide group 18%
Introduction rate of amide group 30%
Introduction rate of amide group 45.5%
Introduction rate of amide group 56.5%
Introduction rate of amide group 66.5%
Introduction rate of amide group 94.5%
Introduction rate of amide group 96%
PPM

[Figure 4]

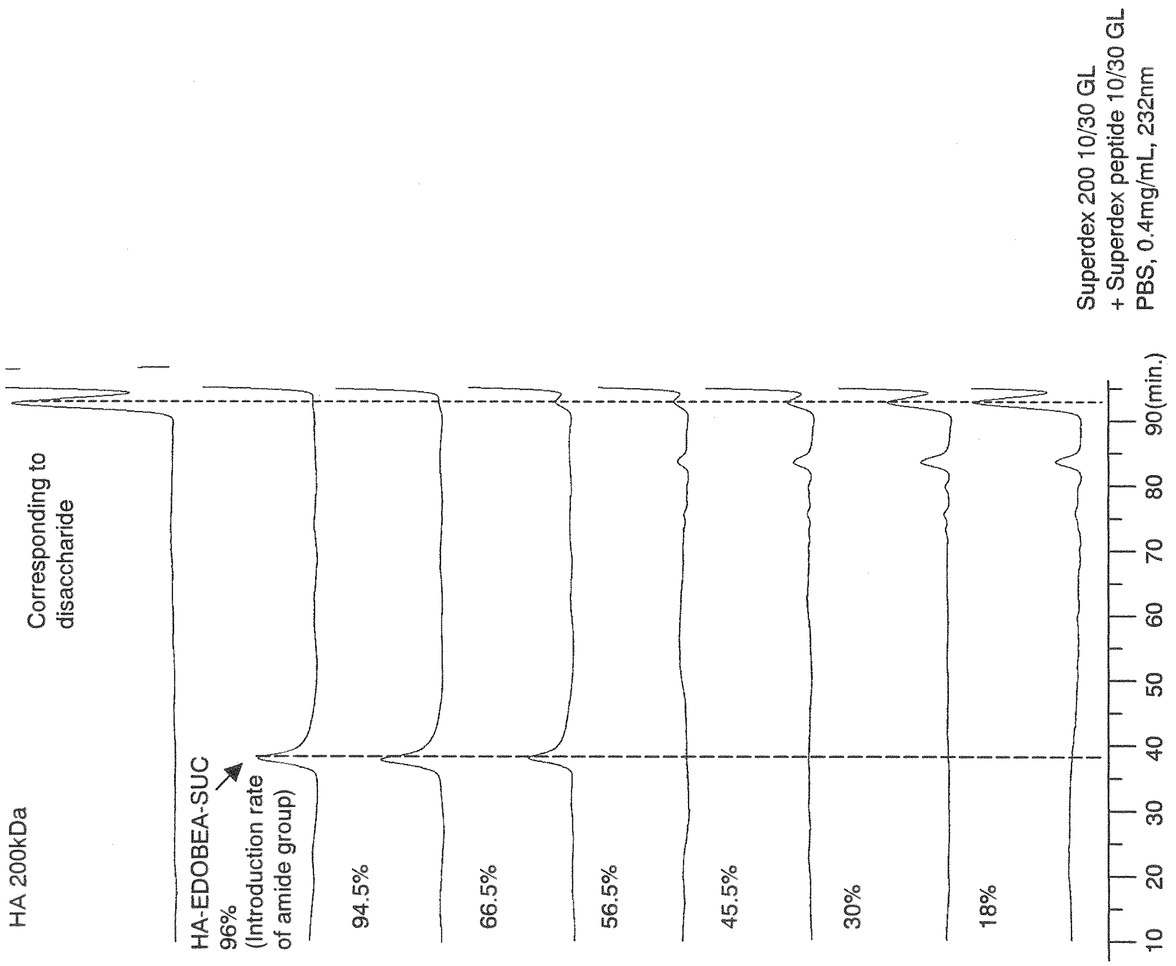
HA 200kDa
Corresponding to disaccharide
HA-EDOBEA-SUC
96%
(Introduction rate of amide group)
94.5%
66.5%
56.5%
45.5%
30%
18%
10
20
30
40
50
60
70
80
90(min.)
Superdex 200 10/30 GL
+ Superdex peptide 10/30 GL
PBS, 0.4mg/mL, 232nm

[Figure 5]

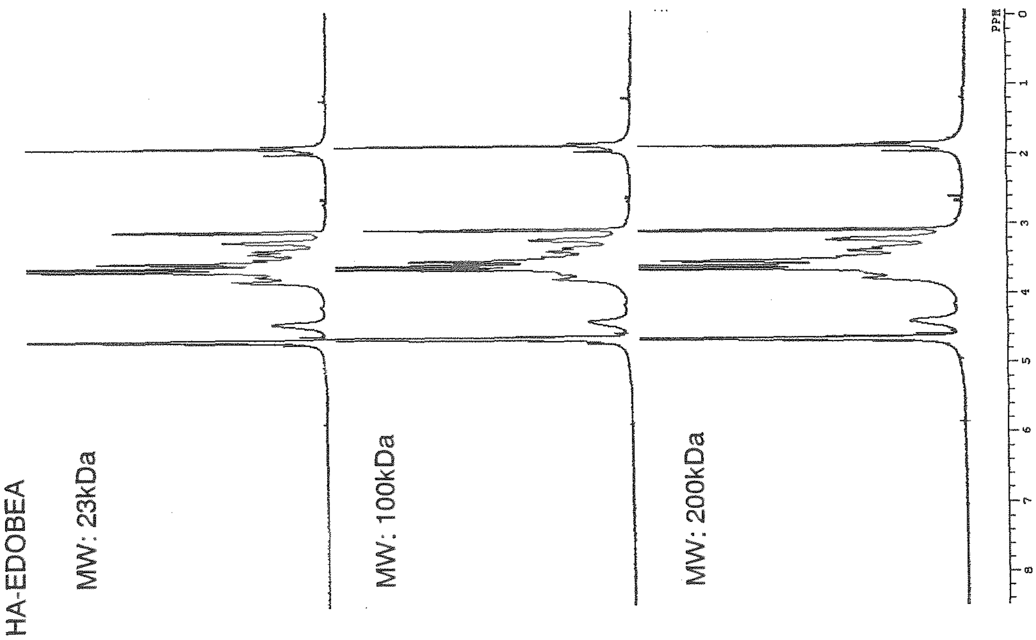

[Figure 6]

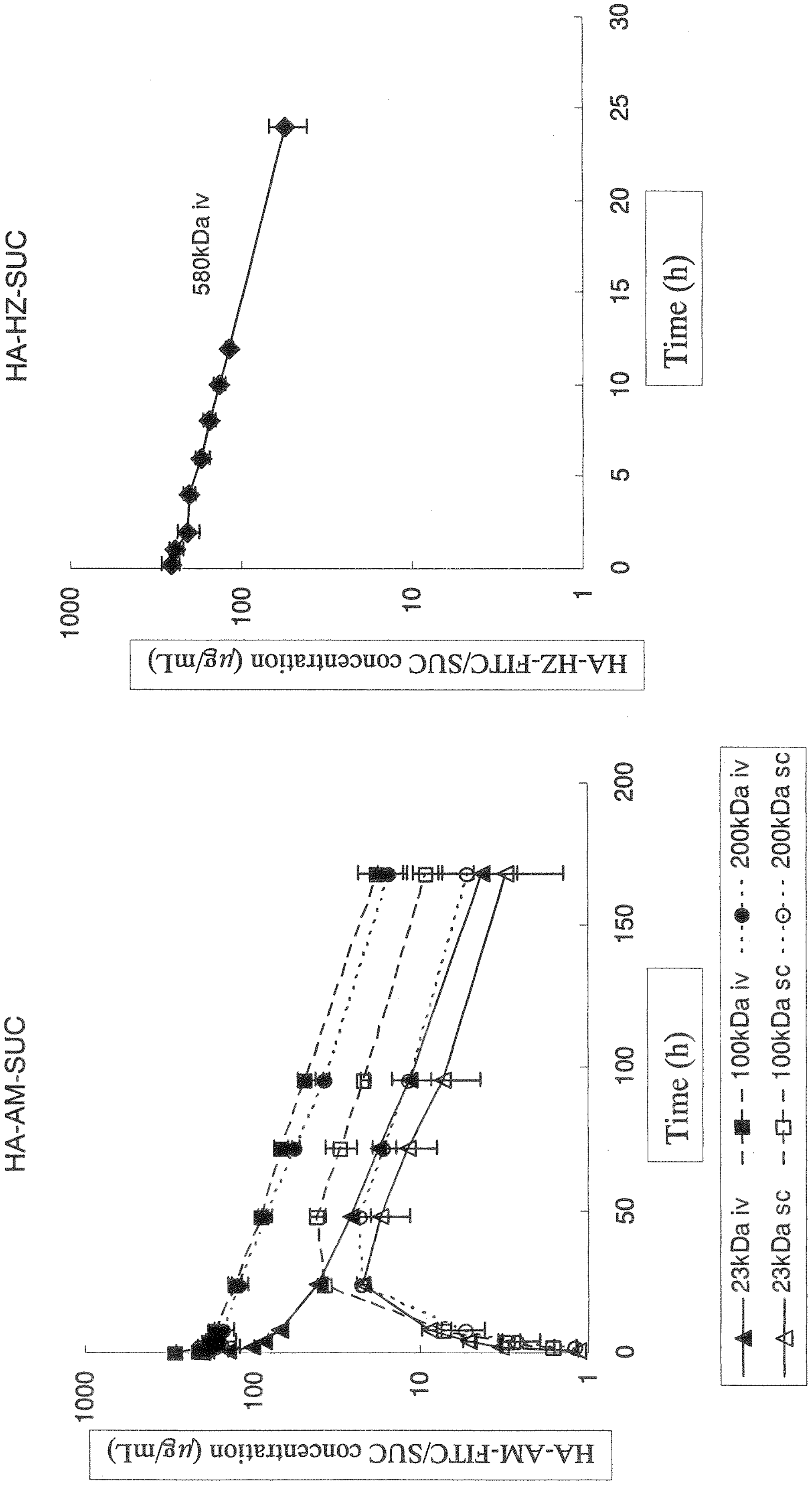
HA-HZ-SUC
580kDa iv
HA-HZ-FITC/SUC concentration (μg/mL)
Time (h)
HA-AM-SUC
HA-AM-FITC/SUC concentration (μg/mL)
Time (h)
23kDa iv
100kDa iv
200kDa iv
23kDa sc
100kDa sc
200kDa sc

[Figure 7]

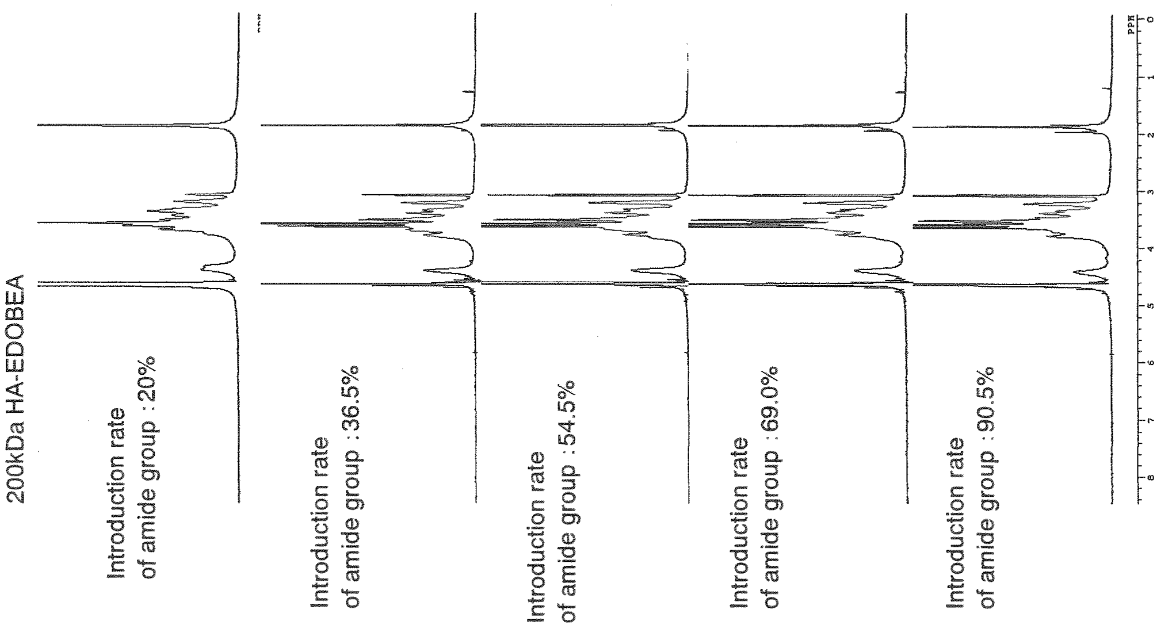
200kDa HA-EDOBEA
Introduction rate of amide group :20%
Introduction rate of amide group :36.5%
Introduction rate of amide group :54.5%
Introduction rate of amide group :69.0%
Introduction rate of amide group :90.5%

[Figure 8]

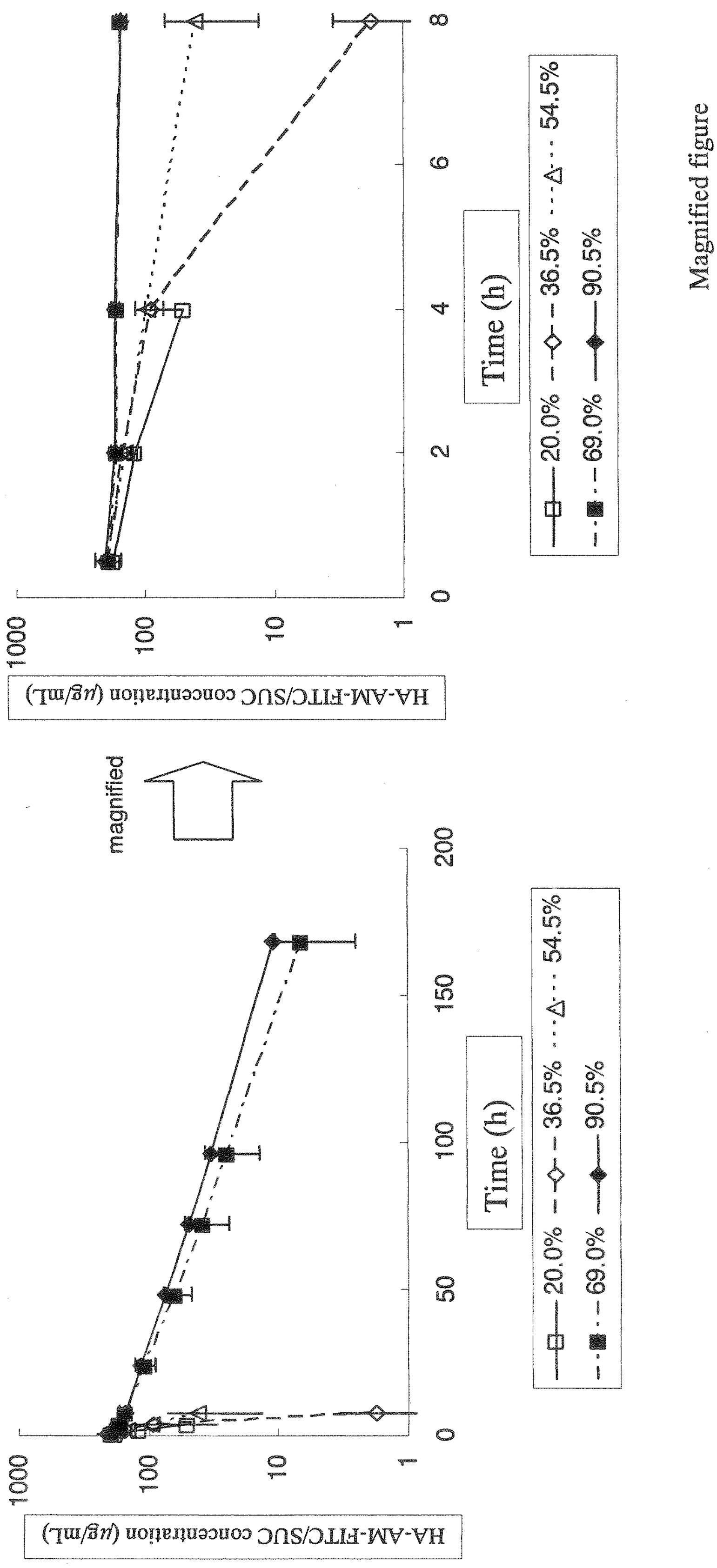
HA-AM-FITC/SUC concentration (μg/mL)
1000
100
10
1
0
2
4
6
8
Time (h)
20.0%
36.5%
54.5%
69.0%
90.5%
Magnified figure
magnified
HA-AM-FITC/SUC concentration (μg/mL)
1000
100
10
1
0
50
100
150
200
Time (h)
20.0%
36.5%
54.5%
69.0%
90.5%

[Figure 9]

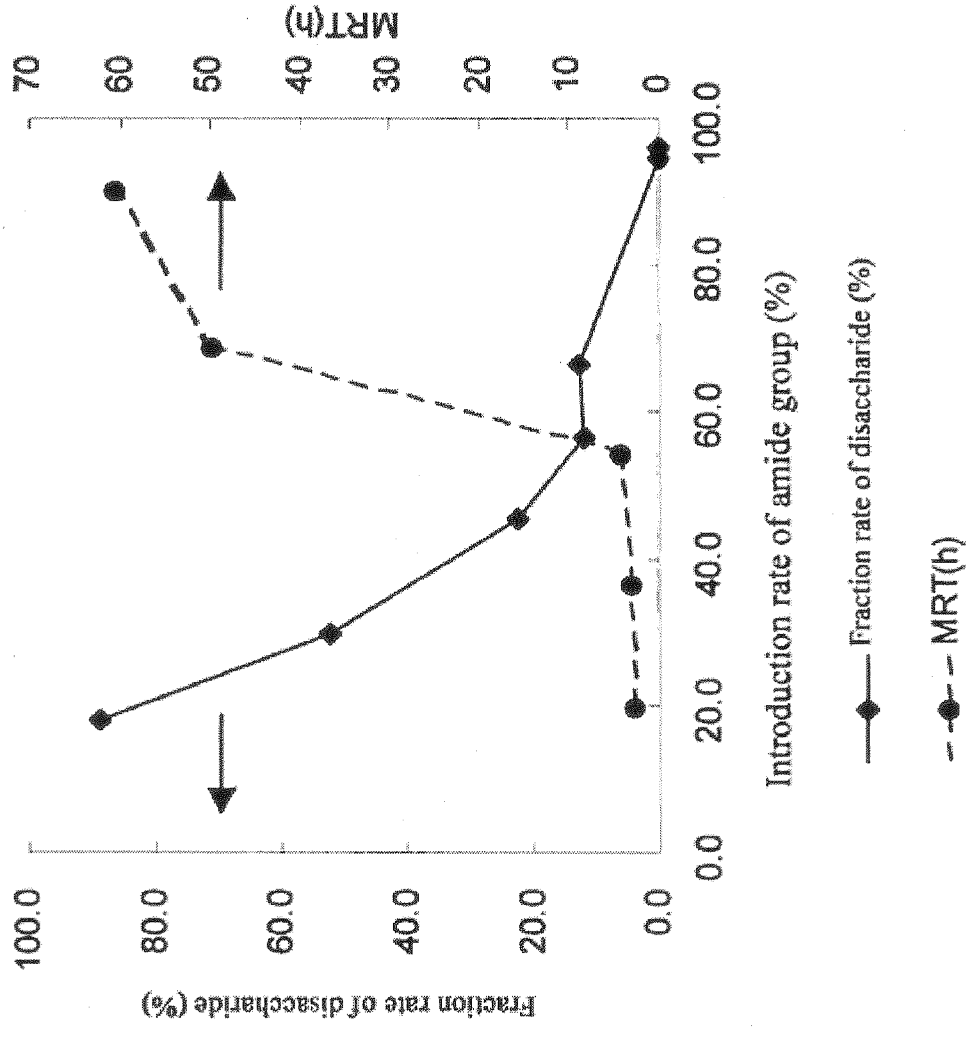
Fraction rate of disaccharide (%)
100.0
80.0
60.0
40.0
20.0
0.0
MRT(h)
70
60
50
40
30
20
10
0
0.0
20.0
40.0
60.0
80.0
100.0
Introduction rate of amide group (%)
Fraction rate of disaccharide (%)
MRT(h)

[Figure 10]

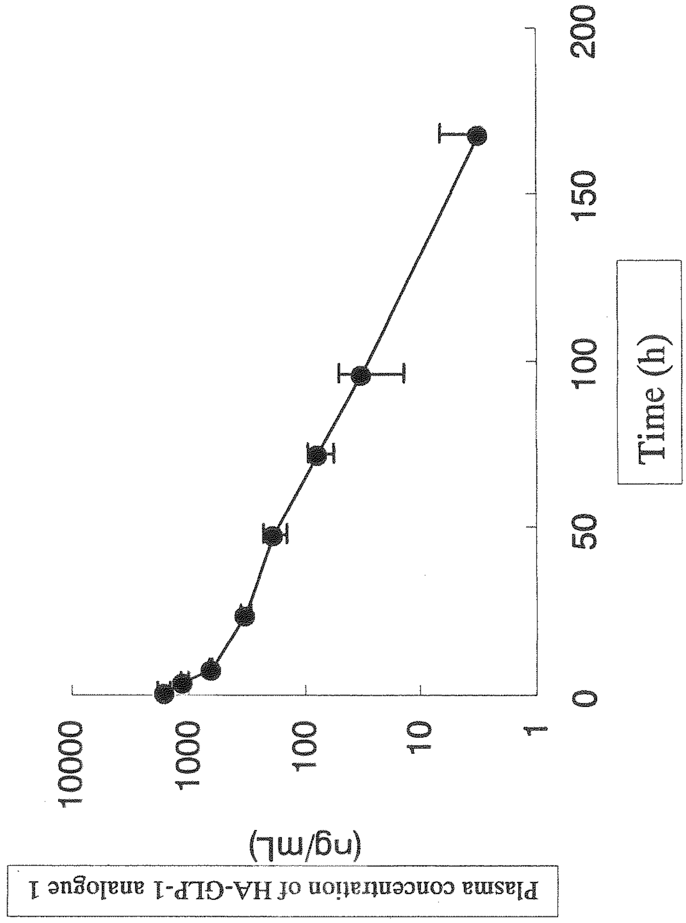
Plasma concentration of HA-GLP-1 analogue 1
(ng/mL)
10000
1000
100
10
1
0
50
100
150
200
Time (h)

[Figure 11]

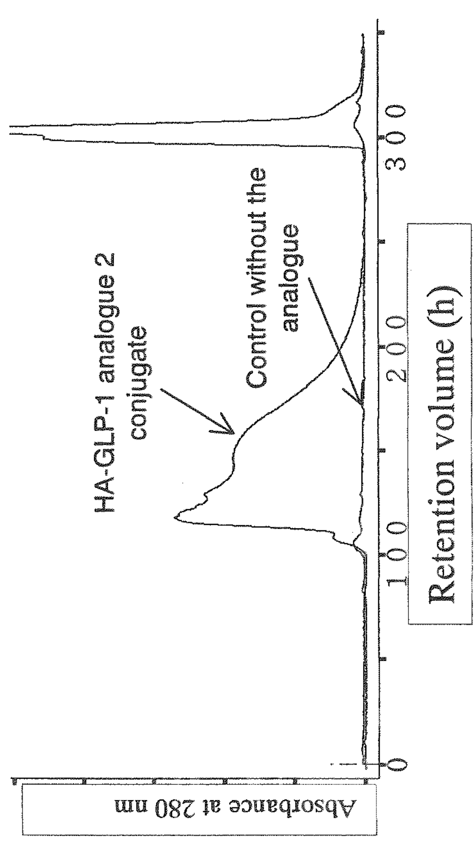
HA-GLP-1 analogue 2 conjugate
Control without the analogue
Retention volume (h)
Absorbance at 280 nm
0
100
200
300

[Figure 12]

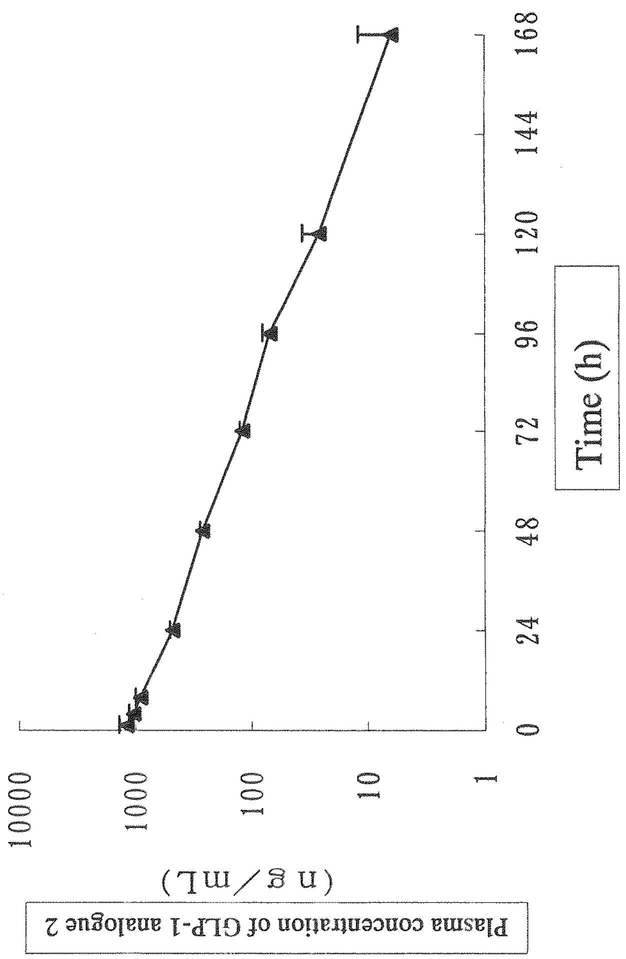
Plasma concentration of GLP-1 analogue 2
(ng/mL)
10000
1000
100
10
1
0
24
48
72
96
120
144
168
Time (h)

[Figure 13]

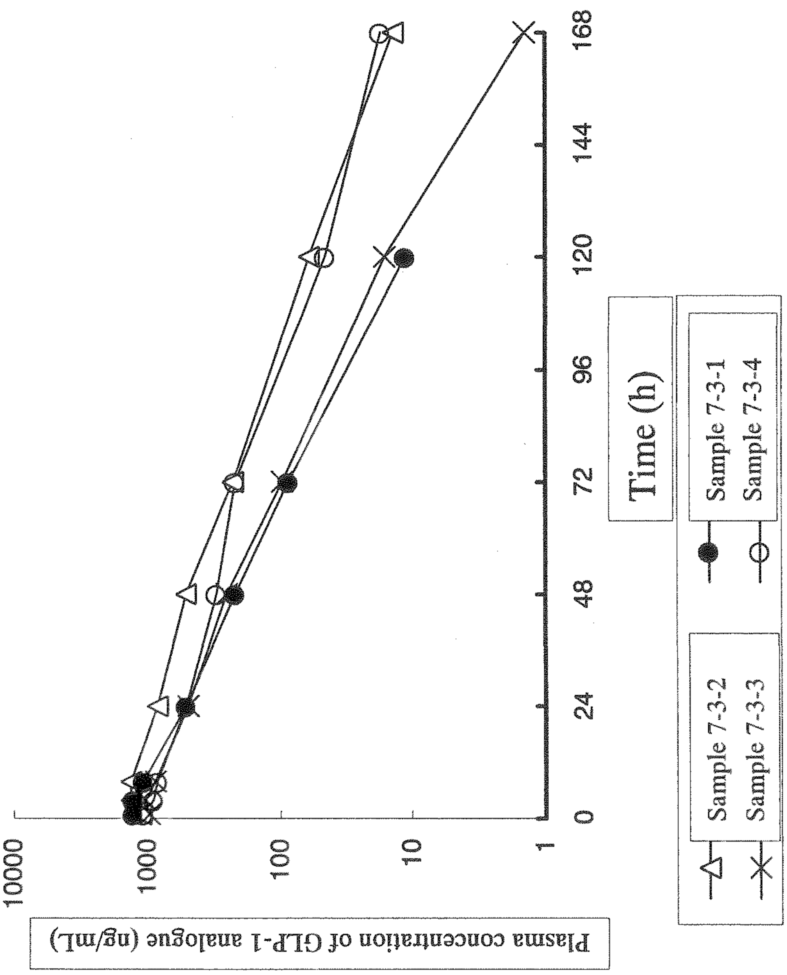
Plasma concentration of GLP-1 analogue (ng/mL)
10000
1000
100
10
1
0
24
48
72
96
120
144
168
Time (h)
Sample 7-3-1
Sample 7-3-2
Sample 7-3-3
Sample 7-3-4

**INTERNATIONAL SEARCH REPORT**

International application No.
PCT/JP2006/304480

A. CLASSIFICATION OF SUBJECT MATTER
*C08B37/08*(2006.01), *A61K47/48*(2006.01), *C07K14/605*(2006.01)

According to International Patent Classification (IPC) or to both national classification and IPC

B. FIELDS SEARCHED

Minimum documentation searched (classification system followed by classification symbols)
A61K47/48, C07K14/605, C08B37/08

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
CAplus(STN), MEDLINE(STN), REGISTRY(STN), SwissProt/PIR/Geneseq

C. DOCUMENTS CONSIDERED TO BE RELEVANT

| Category* | Citation of document, with indication, where appropriate, of the relevant passages | Relevant to claim No. |
|---|---|---|
| Y<br><br>A | WO 2003/074087 A1 (BIOTECHNOLOGIE GES MITTELHESSEN MBH),<br>12 September, 2003 (12.09.03),<br>Full text; particularly, Par. Nos. [0026] to [0035], [0052]; Claim 17; example 17<br>& JP 2005-528349 A | 1,2,4-16,<br>18-22,24-29<br>3,17 |
| Y<br><br>A | WO 2003/040309 A2 (BAYER PHARM CORP.),<br>15 May, 2003 (15.05.03),<br>Full text; particularly, Par. No. [0135]<br>& JP 2005-508639 A | 1,2,4-16,<br>18-22,24-29<br>3,17 |
| Y<br><br>A | WO 2003/103572 A2 (LILLY & CO ELI),<br>18 December, 2003 (18.12.03),<br>Full text; particularly, Claims; examples<br>& EP 1575490 A2 | 1,2,4-16,<br>18-22,24-29<br>3,17 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 June, 2006 (07.06.06) | 13 June, 2006 (13.06.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No.
PCT/JP2006/304480

C (Continuation). DOCUMENTS CONSIDERED TO BE RELEVANT

| Category* | Citation of document, with indication, where appropriate, of the relevant passages | Relevant to claim No. |
|---|---|---|
| Y<br><br>A | WO 2004/046200 A1 (CHUGAI SEIYAKU KABUSHIKI KAISHA),<br>03 June, 2004 (03.06.04),<br>Full text<br>& EP 1564220 A1 | 1,2,4-16,<br>18-22,24-29<br>3,17 |
| Y<br><br>A | JP 7-2695 A (PFIZER INC.),<br>06 January, 1995 (06.01.95),<br>Full text; particularly, Claims<br>& EP 619322 A2 | 1,2,4-16,<br>18-22,24-29<br>3,17 |
| Y<br><br>A | JP 6-508169 A (GENZYME CORP.),<br>14 September, 1994 (14.09.94),<br>Full text; particularly, Claims 1, 13, 41;<br>page 5, upper right column to lower left column;<br>examples 27, 28<br>& WO 92/20349 A1 | 1,2,4-16,<br>18-22,24-29<br>3,17 |
| X<br>Y<br><br><br>A | JP 10-510155 A (BIONEBRASKA INC.),<br>06 October, 1998 (06.10.98),<br>Seq. No. 28<br>& WO 96/17942 A1 | 30,34<br>1,2,4-16,<br>18-22,24-29,<br>31-33<br>3,17 |
| Y<br><br>A | WO 2003/087019 A1 (CHUGAI SEIYAKU KABUSHIKI KAISHA),<br>23 October, 2003 (23.10.03),<br>Full text; particularly, pages 14 to 18<br>& EP 1496037 A1 | 1,2,4-16,<br>18-22,24-29<br>3,17 |
| A | JP 2001-503963 A (LILLY & CO ELI),<br>27 March, 2001 (27.03.01),<br>& WO 97/29180 A1 | 1-22,24-29 |
| A | JP 2002-529550 A (AQUISITIO SPA),<br>10 September, 2002 (10.09.02),<br>& WO 2000/27887 A2 | 1-22,24-29 |
| Y | JP 2003-523366 A (LILLY & CO ELI),<br>05 August, 2003 (05.08.03),<br>Claim 4; Par. No. [0024]<br>& WO 2001/55213 A2 | 7,8,31-33 |
| A | JP 2005/097175 A2 (CENTOCOR INC.),<br>20 October, 2005 (20.10.05),<br>(Family: none) | 1-22,24-29 |

Form PCT/ISA/210 (continuation of second sheet) (April 2005)

**INTERNATIONAL SEARCH REPORT**

International application No.
PCT/JP2006/304480

**Box No. II Observations where certain claims were found unsearchable (Continuation of item 2 of first sheet)**

This international search report has not been established in respect of certain claims under Article 17(2)(a) for the following reasons:

1. [X] Claims Nos.: 23
because they relate to subject matter not required to be searched by this Authority, namely:
Claim 23 pertains to methods for treatment of the human body by surgery or therapy.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

Although the technical feature common to the invention of claims 1-29 and the invention of claims 30-34 is a glucagon-like peptide-1 (GLP-1) analogue, this substance cannot be considered as a special technical feature since it had been well-known before the filing of this application.

Since there is no technical relationship between the invention of claims 1-29 and the invention of claims 30-34 involving the same or corresponding special technical feature, these inventions are not considered so linked as to form a single general inventive concept.

1. [X] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the

[ ] The additional search fees were accompanied by the applicant's protest and, where applicable, payment of a protest fee..

[ ] The additional search fees were accompanied by the applicant's protest but the applicable protest fee was not paid within the time limit specified in the invitation.

[X] No protest accompanied the payment of additional search fees.

Form PCT/ISA/210 (continuation of first sheet (2)) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9111457 A
- JP 11310597 A
- WO 9943705 A
- WO 00069911 A
- WO 9531214 A
- WO 0007617 A
- WO 03103572 A
- WO 9729180 A
- WO 9206714 A
- JP 2001081103 A
- JP 2273176 A
- JP 5085942 A
- WO 0105434 A
- WO 0160412 A
- JP 2002519481 A
- WO 9419376 A
- WO 04022004 A
- WO 03040309 A
- JP 7504679 A


### Non-patent literature cited in the description


- *Trends Pharacol. Sci.,* 2003, vol. 24, 377-383
- *Int. J. Pharm.,* 2003, vol. 255, 167-174
- *J. Control. Rel.,* 2003, vol. 88, 35-42
- *J. Inter. Med.,* 1997, vol. 242, 27-33
- *Exp. Cell Res.,* 1996, vol. 228, 216-228
- Essential Polymer Science. Kodansha Ltd
- *Current Medicinal Chemistry,* 2003, vol. 10, 2471-2483
- *Diabetologia,* 1998, vol. 41, 271-278
- **GRAZIANO et al.** *Biochem. Biophys. Res. Commun.,* 1993, vol. 196, 141-146

SEQUENCE LISTING

```
<110>  Chugai Seiyaku Kabushiki Kaisha

<120>  CONJUGATE OF WATER-SOLUBLE HYALURONIC ACID MODIFICATION PRODUCT WITH
GLP-1 ANALOGUE

<130>  N2760 EP

<140>  PCT/JP2006/304480
<141>  2006-03-08

<150>  JP 2005-064122
<151>  2005-03-08

<150>  JP 2006-035645
<151>  2006-02-13

<160>  1

<210>  1
<211>  32
<212>  PRT
<213>  Artificial

<220>
<223>  Artificial

<400>  1

His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Xaa Cys
            20                  25                  30
```